# EUROPEAN PATENT SPECIFICATION

(11) **EP 3 212 663 B1**
(45) Date of publication and mention of the grant of the patent: **27.05.2020**
(21) Application number: 15795274.8
(22) Date of filing: 31.10.2015
(51) Int. Cl.: C07K 14/81

(54) **C1 ESTERASE INHIBITOR FUSION PROTEINS AND USES THEREOF**
C1-ESTERASEINHIBITORFUSIONSPROTEINE UND VERWENDUNGEN DAVON
PROTÉINES DE FUSION DE L'INHIBITEUR DE LA C1 ESTÉRASE ET LEURS UTILISATIONS

(30) Priority: 31.10.2014 US 201462073657 P
(43) Date of publication of application: 06.09.2017
(73) Proprietor: Shire Human Genetic Therapies, Inc., Lexington, MA 02421 (US)
(72) Inventor: NORTON, Angela, Lexington, Massachusetts 02421 (US); PAN, Clark, Lexington, Massachusetts 02421 (US); ISKENDERIAN, Andrea, Lexington, Massachusetts 02421 (US); STRACK-LOGUE, Bettina, Lexington, Massachusetts 02421 (US)
(74) Representative: Goodfellow, Hugh Robin
(86) International application number: PCT/US2015/058521
(87) International publication number: WO 2016/070156

(56) References cited:
- WO-A1-2011/116291
- WO-A1-2013/093027
- WO-A1-2014/152940
- WO-A2-2013/003641
- RATH TIMO ET AL: "Fc-fusion proteins and FcRn: structural insights for longer-lasting and more effective therapeutics", CRITICAL REVIEWS IN BIOTECHNOLOGY, vol. 35, no. 2, 22 October 2013 (2013-10-22), pages 235-254, XP002753804,

## Description

This application claims priority from U.S. provisional patent application serial number 62/073657, filed October 31, 2014.

### BACKGROUND

C1-inhibitor (C1-INH), also known as C1 esterase inhibitor, is the largest member of the serpin protein superfamily. It is a heavily glycosylated serine proteinase inhibitor having the main function of inhibiting the spontaneous activation of the complement system. C1-INH regulates the complement cascade system, plays a key role in the regulation of the contact (kallikrein-kinin) amplification cascade, and participates in the regulation of the coagulation and fibrinolytic systems. Karnaukhova, E., C1-Esterase Inhibitor: Biological Activities and Therapeutic Applications. J Hematol Thromb Dis, 1: 113 (2013).

Dysfunction and/or deficiency of C1-INH in subjects has been correlated with a variety of autoimmune disease due to the failure of C1-INH to inhibit the activation of the complement system. An example of such a disease is hereditary angioedema (HAE), a rare, but potentially life-threatening disorder characterized by unpredictable and recurrent attacks of inflammation. Symptoms of HAE attacks include swelling of the face, mouth and/or airway that occur spontaneously or are triggered by mild trauma. Such swelling can also occur in any part of the body. In some cases, HAE is associated with low plasma levels of C1-inhibitor, while in other cases the protein circulates in normal or elevated amounts but it is dysfunctional. In addition to the episodes of inflammation, it also can cause more serious or life threatening indications, such as autoimmune diseases or lupus erythematosus.

CINRYZE®, a human plasma derived C1 esterase inhibitor, has been approved for prophylactic use and treatment of acute attacks of HAE. Berinert® (also a plasma-derived human C1-INH, CSL Behring) is indicated for treatment of acute HAE attack. The supply of human plasma derived C1 esterase inhibitor is tied to the availability of blood and plasma donations. Ruconest® (conestat alfa, Pharming N.V.) a recombinant C1-INH expressed in engineered rabbits is indicated for IV administration for treatment of acute HAE attack. However, because Ruconest® is made in rabbits, its glycosylation profile is different from that of human plasma-derived C1-INH. The result is that Ruconest has an extremely short half-life of about 2.4-2.7 hours. See Ruconest® FDA Label and Prescribing Information.

Therefore, it remains a need in the art for improved C1 esterase inhibitor for the treatment of various C1 esterase mediated indication.

WO 2013/003641 discloses fusion proteins that comprise a serpin polypeptide operably linked to a second polypeptide which comprises at least one of an immunoglobulin Fc polypeptide, a cytokine targeting polypeptide, a WAP domain containing polypeptide and an albumin polypeptide.

WO 2014/152940 discloses an mRNA encoding a therapeutic protein fused to a polypeptide that is capable of binding to an Fc receptor for delivery to one or more target cells for production of therapeutic levels of the therapeutic protein.

WO 2013/093027 discloses C1-INH for use in a method of preventing the formation and/or reducing the size of a secondary edema of the central nervous system (CNS) in a subject having at least one disorder selected from the group consisting of stroke, ischemic stroke, hemorrhagic stroke, perinatal stroke, traumatic brain injury and spinal cord injury.

Rath Timo et. al., Crit. Bev. Biotechnol., 2015, 35(2): 235-254, reviews Fc-fusion therapeutics and FcRn-dependent delivery approaches in development, and the effect of engineering of the FcRn-Fc interaction on the development of therapeutics.

WO 2011/116291 discloses production of human C1-INH or portions thereof in human cells.

### SUMMARY

The present invention provides, among other things, improved long-acting recombinant C1 esterase inhibitors that can be used to effectively treat various complement-mediated disorders and that can be manufactured in a cost-effective matter.

In particular, the present invention provides C1 esterase inhibitor fusion proteins that exhibit longer half-life than Ruconest®. In some embodiments, the C1 inhibitor fusion proteins of the invention exhibit a half-life comparable to or longer than plasma-derived C1-INH. For example, the present inventors have demonstrated that certain exemplary C1 inhibitor fusion proteins according to the present invention have extended serum half-life of at least 4 days. It is contemplated that the long serum half-life of a recombinant C1 inhibitor leads to superior *in vivo* efficacy and permits a preferable dosing regimen and route of administration. In certain embodiments, the C1 inhibitor fusion proteins of the invention may be administered subcutaneously with less frequency than approved C1 inhibitors, while still achieving desired efficacy (e.g., prophylaxis). Moreover, the C1 inhibitor fusion proteins of the invention can be produced recombinantly in host cells such that the disclosed C1 inhibitor fusion proteins are not dependent on blood supply, do not pose a risk of transmission of infectious agents, and are less expensive to manufacture. Since they are recombinantly produced in host cells, they offer more consistency in production and final product than those products purified from human blood, human blood components (e.g. plasma), or animal milk. Moreover, the C1 inhibitor fusion proteins provided herein are not dependent on animal husbandry considerations, including animal age and/or maturity, milk production, animal illness, etc., all of which may affect both quantity and quality (e.g., glycosylation profile, heterogeneity of expressed protein, etc.) of the rabbit expressed C1-INH. Thus, the present invention provides for the cost effective and reliable manufacturing of recombinant C1 esterase inhibitors, and safer, more effective treatment of HAE and other complement-mediated disorders. Other features, objects, and advantages of the present invention are apparent in the detailed description that follows. It should be understood, however, that the detailed description, while indicating embodiments of the present invention, is given by way of illustration only, not limitation.

In one aspect, the present invention provides a fusion protein comprising a human C1-inhibitor polypeptide and an Fc domain wherein the fusion protein has an amino acid sequence selected from: SEQ ID NO:11, SEQ ID NO:13, SEQ ID NO:14, SEQ ID NO:15, or SEQ ID NO:16. In some embodiments of the present disclosure, the human C1-inhibitor polypeptide comprises an amino acid sequence at least at least 50% (e.g., at least 55%, 60%, 65%, 70%, 75%, 80%, 85%, 90%, 95%, 96%, 97%, 98%, 99%, or 100%) identical to the full length human C1-inhibitor having SEQ ID NO:1.

In some embodiments, the fusion protein comprising a human C1-inhibitor polypeptide and an Fc domain includes an amino acid sequence identical to the full length human C1-inhibitor protein SEQ ID NO:1.

In some embodiments of the present disclosure, the fusion protein comprising a human C1-inhibitor polypeptide and an Fc domain comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:2. In some embodiments, the fusion protein comprising a human C1-inhibitor polypeptide and an Fc domain includes an amino acid sequence identical to the full length human C1-inhibitor protein SEQ ID NO:2. In some embodiments of the fusion protein comprising a human C1-inhibitor polypeptide and an Fc domain, the Fc domain is attached to the N-terminus of the human C1-inhibitor polypeptide. In some embodiments the Fc domain is a human IgG1 Fc domain. In some embodiments of the fusion protein comprising a human C1-inhibitor polypeptide and an Fc domain, the Fc domain is attached to the N-terminus of the human C1-inhibitor polypeptide. In some embodiments the Fc domain is derived from human IgG1 Fc domain. In some embodiments, an Fc domain suitable for the present disclosure comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:3. In some embodiments, the fusion protein comprising a human C1-inhibitor polypeptide and an Fc domain includes an amino acid sequence identical to the human IgG1 Fc domain SEQ ID NO:3.

In some embodiments of the present disclosure, an Fc domain suitable for the present disclosure comprises a L234A mutation. In some embodiments of the present disclosure, an Fc domain suitable for the present disclosure comprises a L235A mutation. In some embodiments, an Fc domain suitable for the present invention comprises a L234A mutation and a L235A mutation. In some embodiments of the present disclosure, an Fc domain suitable for the present disclosure comprises a L234A mutation or a L235A mutation.

In some embodiments, an Fc domain suitable for the present invention comprises the amino acid sequence of SEQ ID NO:4. In some embodiments, an Fc domain suitable for the present invention comprises one or more mutations that prolong the half-life of the fusion protein comprising a human C1-inhibitor polypeptide and an Fc domain. In some embodiments of the present disclosure, the mutation of the Fc domain comprises one or more mutations are selected from one or more positions corresponding to Thr 250, Met 252, Ser 254, Thr 256, Thr 307, Glu 380, Met 428, His 433, and/or Asn 434 of human IgG1.

In some embodiments of the present disclosure, an Fc domain suitable for the present invention comprises a H433K mutation. In some embodiments of the present disclosure, an Fc domain suitable for the present invention comprises a N434F mutation. In some embodiments of the present disclosure, an Fc domain suitable for the present invention comprises a H433K mutation and a N434F mutation. In some embodiments of the present disclosure, an Fc domain suitable for the present invention comprises a H433K mutation or a N434F mutation.

In some embodiments of the fusion protein comprising a human C1-inhibitor polypeptide and an Fc domain, the Fc domain is attached to the N-terminus of the human C1-inhibitor polypeptide. In some embodiments the Fc domain is a human IgG4 Fc domain. In some embodiments of the fusion protein comprising a human C1-inhibitor polypeptide and an Fc domain, the Fc domain is attached to the N-terminus of the human C1-inhibitor polypeptide. In some embodiments the Fc domain is derived from a human IgG4 Fc domain.

In some embodiments of the present disclosure, an Fc domain suitable for the present invention comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:9. In some embodiments, the fusion protein comprising a human C1-inhibitor polypeptide and an Fc domain includes an amino acid sequence identical to the human IgG4 Fc domain SEQ ID NO:9. In some embodiments, an Fc domain suitable for the present invention comprises a S241P mutation. In some embodiments of the present disclosure, fusion protein comprising a human C1-inhibitor polypeptide and an Fc domain comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:10. In some embodiments, the fusion protein comprising a human C1-inhibitor polypeptide and an Fc domain includes an amino acid sequence identical to SEQ ID NO:10.

In some embodiments of the present disclosure, fusion protein comprising a human C1-inhibitor polypeptide and an Fc domain comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:11. In some embodiments, the fusion protein comprising a human C1-inhibitor polypeptide and an Fc domain includes an amino acid sequence identical to SEQ ID NO:11. In some embodiments of the present disclosure, fusion protein comprising a human C1-inhibitor polypeptide and an Fc domain comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:13. In some embodiments, the fusion protein comprising a human C1-inhibitor polypeptide and an Fc domain includes an amino acid sequence identical to SEQ ID NO: 13.

In some embodiments of the present disclosure, fusion protein comprising a human C1-inhibitor polypeptide and an Fc domain comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:14. In some embodiments, the fusion protein comprising a human C1-inhibitor polypeptide and an Fc domain includes an amino acid sequence identical to SEQ ID NO:14. In some embodiments of the present disclosure, fusion protein comprising a human C1-inhibitor polypeptide and an Fc domain comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO: 15In some embodiments, the fusion protein comprising a human C1-inhibitor polypeptide and an Fc domain includes an amino acid sequence identical to SEQ ID NO:15.

In some embodiments of the present disclosure, fusion protein comprising a human C1-inhibitor polypeptide and an Fc domain comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:16. In some embodiments, the fusion protein comprising a human C1-inhibitor polypeptide and an Fc domain includes an amino acid sequence identical to SEQ ID NO:16. In some embodiments of the fusion protein of the disclosure, comprising a human C1-inhibitor polypeptide and an Fc domain, the fusion protein comprises a linker between the human C1-inhibitor polypeptide and the Fc domain. In some embodiments of the disclosure, the linker is a peptide comprising 3-100 amino acids.

In some embodiments of the fusion protein comprising a human C1-inhibitor polypeptide and an Fc domain, the fusion protein binds FcRN. In some embodiments of the fusion protein comprising a human C1-inhibitor polypeptide and an Fc domain, the fusion protein inhibits C1 esterase activity.

In some embodiments of the fusion protein comprising a human C1-inhibitor polypeptide and an Fc domain, the Fc domain comprises a mutation that reduces or eliminates ADCC activity. In some embodiments of the fusion protein of the disclosure, comprising a human C1-inhibitor polypeptide and an Fc domain, the Fc domain comprises one or more mutations that reduce or eliminate ADCC activity. In some embodiments of the fusion protein comprising a human C1-inhibitor polypeptide and an Fc domain, the Fc domain comprises a mutation that reduces or eliminates CDC activity. In some embodiments of the fusion protein of the disclosure, comprising a human C1-inhibitor polypeptide and an Fc domain, the Fc domain comprises one or more mutations that reduce or eliminate CDC activity.

In some embodiments of the fusion protein comprising a human C1-inhibitor polypeptide and an Fc domain, the Fc domain comprises a mutation that reduce or eliminate FcγR binding. In some embodiments of the fusion protein of the disclosure, comprising a human C1-inhibitor polypeptide and an Fc domain, the Fc domain comprises one or more mutations that reduce or eliminate FcγR binding. In some embodiments of the fusion protein comprising a human C1-inhibitor polypeptide and an Fc domain, the Fc domain comprises a mutation that reduces or eliminates FcγR effector function. In some embodiments of the fusion protein of the disclosure, comprising a human C1-inhibitor polypeptide and an Fc domain, the Fc domain comprises one or more mutations that reduce or eliminate FcγR effector function.

In some embodiments of the fusion protein comprising a human C1-inhibitor polypeptide and an Fc domain, the Fc domain comprises a mutation that reduces or eliminates C1q binding. In some embodiments of the fusion protein of the disclosure, comprising a human C1-inhibitor polypeptide and an Fc domain, the Fc domain comprises one or more mutations that reduce or eliminate C1q binding. In some embodiments of the fusion protein comprising a human C1-inhibitor polypeptide and an Fc domain, the mutation that reduces or eliminates C1q binding is in the Fc domain.

In some embodiments of the fusion protein comprising a human C1-inhibitor polypeptide and an Fc domain, the fusion protein inhibits and/or inactivates C1r and/or C1s protease activity. In some embodiments of the fusion protein comprising a human C1-inhibitor polypeptide and an Fc domain, the fusion protein inhibits the lysis of red blood cells *in vitro.*

In some embodiments of the fusion protein comprising a human C1-inhibitor polypeptide and an Fc domain, the fusion protein has a longer half-life than plasma-derived human C1-inhibitor. In some embodiments of the fusion protein comprising a human C1-inhibitor polypeptide and an Fc domain, the fusion protein has a half-life of at least four days. In some embodiments of the fusion protein comprising a human C1-inhibitor polypeptide and an Fc domain, the fusion protein has a half-life of at least five days. In some embodiments of the fusion protein comprising a human C1-inhibitor polypeptide and an Fc domain, the fusion protein has a half-life of at least six days. In some embodiments of the fusion protein comprising a human C1-inhibitor polypeptide and an Fc domain, the fusion protein has a half-life of at least seven days.

In some embodiments of the present disclosure, the fusion protein comprising a human C1-inhibitor polypeptide and an Fc domain, the fusion protein has an *in vivo* half-life of or greater than about 12 hours, 18 hours, 24 hours, 36 hours, 2 days, 2.5 days, 3 days, 3.5 days, 4 days, 4.5 days, 5 days, 5.5 days, 6 days, 6.5 days, 7 days, 7.5 days, 8 days, 8.5 days, 9 days, 9.5 days, or 10 days. In some embodiments of the present disclosure, a recombinant C1-INH fusion protein has an *in vivo* half-life of between 0.5 and 10 days, between 1 day and 10 days, between 1 day and 9 days, between 1 day and 8 days, between 1 day and 7 days, between 1 day and 6 days, between 1 day and 5 days, between 1 day and 4 days, between 1 day and 3 days, between 2 days and 10 days, between 2 days and 9 days, between 2 days and 8 days, between 2 days and 7 days, between 2 days and 6 days, between 2 days and 5 days, between 2 days and 4 days, between 2 day and 3 days, between 2.5 days and 10 days, between 2.5 days and 9 days, between 2.5 days and 8 days, between 2.5 days and 7 days, between 2.5 days and 6 days, between 2.5 days and 5 days, between 2.5 days and 4 days, between 3 days and 10 days, between 3 days and 9 days, between 3 days and 8 days, between 3 days and 7 days, between 3 days and 6 days, between 3 days and 5 days, between 3 days and 4 days, between 3.5 days and 10 days, between 3.5 days and 9 days, between 3.5 days and 8 days, between 3.5 days and 7 days, between 3.5 days and 6 days, between 3.5 days and 5 days, between 3.5 days and 4 days, between 4 days and 10 days, between 4 days and 9 days, between 4 days and 8 days, between 4 days and 7 days, between 4 days and 6 days, between 4 days and 5 days, between 4.5 days and 10 days, between 4.5 days and 9 days, between 4.5 days and 8 days, between 4.5 days and 7 days, between 4.5 days and 6 days, between 4.5 days and 5 days, between 5 days and 10 days, between 5 days and 9 days, between 5 days and 8 days, between 5 days and 7 days, between 5 days and 6 days, between 5.5 days and 10 days, between 5.5 days and 9 days, between 5.5 days and 8 days, between 5.5 days and 7 days, between 5.5 days and 6 days, between 6 days and 10 days, between 7 days and 10 days, between 8 days and 10 days, between 9 days and 10 days.

In some embodiments of the fusion protein comprising a human C1-inhibitor polypeptide and an Fc domain, the fusion protein is monovalent. In some embodiments of the fusion protein comprising a human C1-inhibitor polypeptide and an Fc domain, the fusion protein is dimeric.

The present disclosure further provides examples of a fusion protein comprising a human C1-inhibitor polypeptide and an albumin polypeptide. In one aspect the present invention provides a nucleic acid encoding a fusion protein of any one of the fusion proteins comprising a human C1-inhibitor polypeptide and an Fc domain of the invention.

In one aspect the present invention provides a cell comprising a nucleic acid encoding a fusion protein of any one of the fusion proteins comprising a human C1-inhibitor polypeptide and an Fc domain of the invention.

In some embodiments, the cell is a mammalian cell. In some embodiments, the mammalian cell is a human cell. In some embodiments, the mammalian cell is a Chinese Hamster Ovary (CHO) cell. In some embodiments, the cell is engineered to modify glycosylation of protein expressed by the cell. In some embodiments, the cell is engineered to modify glycosylation of protein expressed by the cell as compared to the same cell that has not been engineered. In some embodiments, the cell is engineered to enhance, improve, increase and/or humanize glycosylation of protein expressed by the cell. In some embodiments, the cell is engineered to enhance, improve, increase and/or humanize glycosylation of protein expressed by the cell as compared to the same cell that has not been engineered. In some embodiments, the cell is engineered to modify sialylation of protein expressed by the cell. In some embodiments, the cell is engineered to modify sialylation of protein expressed by the cell as compared to the same cell that has not been engineered. In some embodiments, the cell is engineered to enhance, improve, increase and/or humanize sialylation of protein expressed by the cell. In some embodiments, the cell is engineered to enhance, improve, increase and/or humanize sialylation of protein expressed by the cell as compared to the same cell that has not been engineered.

In another aspect, the invention provides a fusion protein comprising a human C1-inhibitor polypeptide and an Fc domain and expressed or produced by a cell engineered to modify, enhance, improve, increase and/or humanize glycosylation. In another aspect, the invention provides a fusion protein comprising a human C1-inhibitor polypeptide and an Fc domain and expressed or produced by a cell engineered to modify, enhance, improve, increase and/or humanize sialylation.

In another aspect, the invention provides a pharmaceutical composition comprising a fusion protein of any one of the fusion proteins comprising a human C1-inhibitor polypeptide and an Fc domain disclosed herein and a pharmaceutically acceptable carrierIn one aspect, the invention provides a method of treating a complement-mediated disorder comprising administering to a subject in need of treatment a pharmaceutical composition comprising a fusion protein of any one of the fusion proteins comprising a human C1-inhibitor polypeptide and an Fc domain of the invention and a pharmaceutically acceptable carrier.

In some embodiments, the subject in need of treatment has a complement-mediated disorder. In some embodiments, the complement-mediated disorder is selected from hereditary angioedema, antibody mediated rejection, neuromyelitis optica spectrum disorders, traumatic brain injury, spinal cord injury, ischemic brain injury, burn injury, toxic epidermal necrolysis, multiple sclerosis, amyotrophic lateral sclerosis (ALS), Parkinson's disease, stroke, chronic inflammatory demyelinating polyneuropathy (CIDP), myasthenia gravis, multifocal motor neuropathy.

### BRIEF DESCRIPTION OF THE DRAWINGS

The drawings are for illustration purposes only, not for limitation.

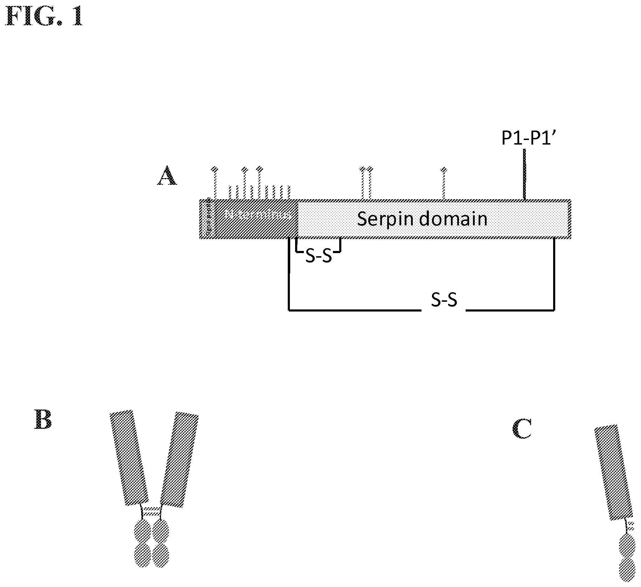

FIG. 1A is a schematic representation of C1-INH. From right to left the three domains are the signal peptide, the N-terminus, also referred to as N-terminal domain, and the serpin domain. N-linked glycans are shown as long vertical lines with diamond heads and O-linked glycans are shown as short vertical lines. FIG. 1B is a schematic representation of an exemplary dimeric C1-INH Fc fusion protein. The C1-INH polypeptide portions are indicated by the rectangles and the Fc portion is indicated by the oval segments. FIG. 1C is a schematic representation of an exemplary monomeric C1-INH Fc fusion protein.

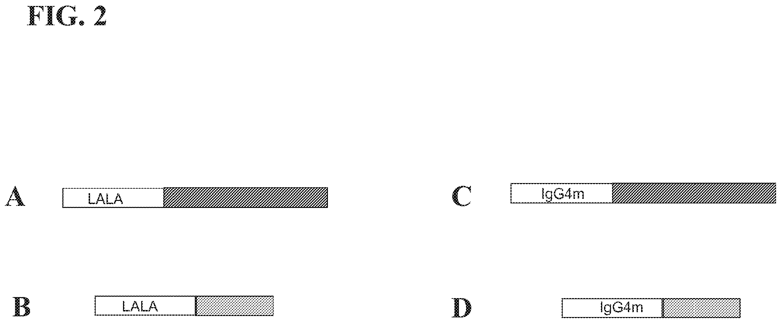

FIG. 2A-2D are a schematic representations of exemplary C1-INH fusion proteins. FIG. 2A is a representation of an IgG1 Fc with LALA mutation fused to a full length C1-INH polypeptide (shown as shaded rectangle). FIG. 2B is a representation of an IgG1 Fc with LALA mutation fused to a truncated C1-INH polypeptide (shown as shaded rectangle). FIG. 2C is a representation of an IgG4 Fc with S241P mutation fused to a full length C1-INH polypeptide (shown as shaded rectangle). FIG. 2D is a representation of an IgG4 Fc with S241P mutation fused to a truncated C1-INH polypeptide (shown as shaded rectangle).

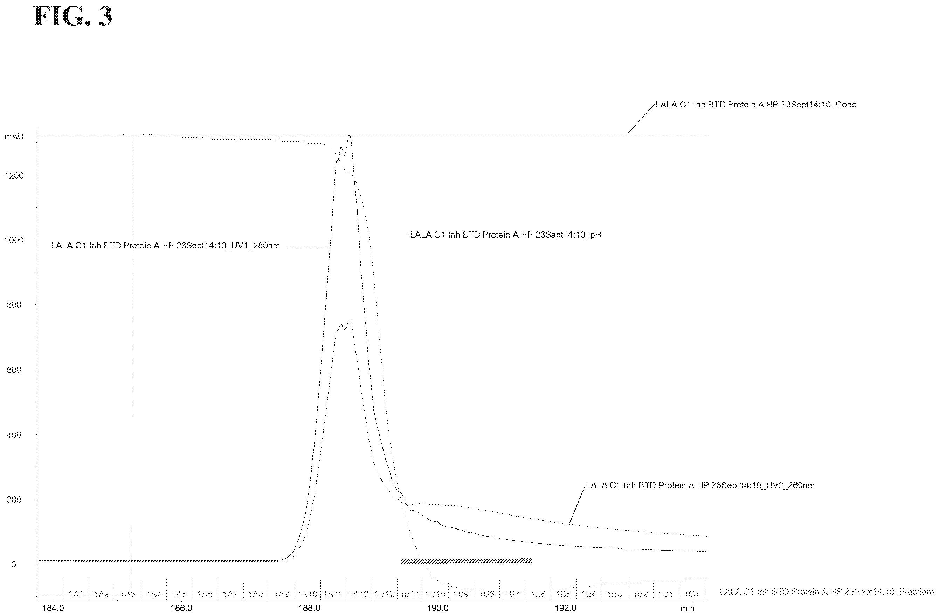

FIG. 3 shows the results of an exemplary protein A purification of full length C1-INH human Fc (hFc) IgG1 LALA fusion protein. The graph shows the results of UV 280 nm, UV 260 nm, concentration, and pH over time during the capture and elution of the fusion protein during the purification process.

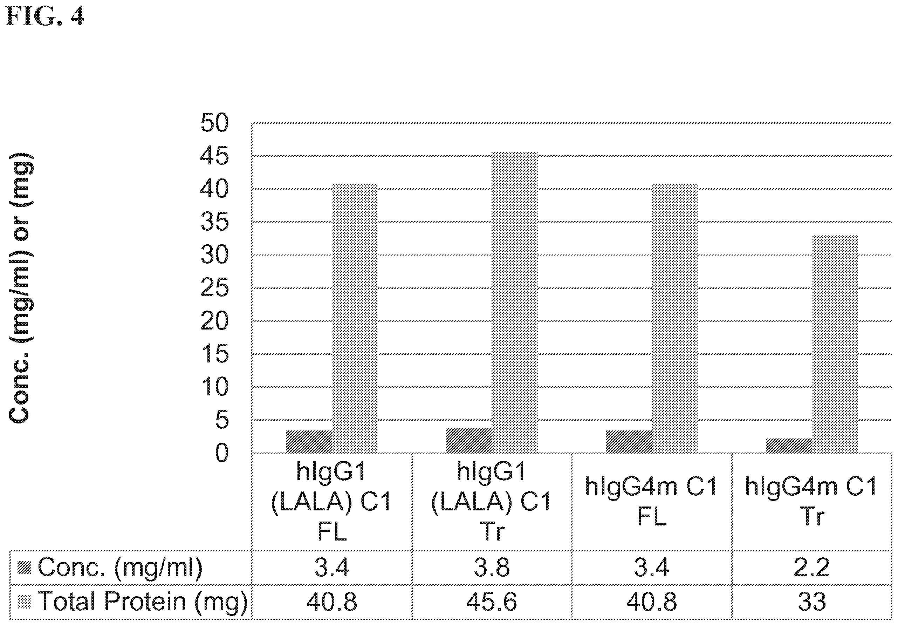

FIG. 4 is a chart of the concentration and total protein collected during prufication of four exemplary Fc fusion constructs: full length (FL) C1-INH hFc IgG1 LALA fusion protein; truncated (Tr) C1-INH hFc IgG1 LALA fusion protein; full length (FL) C1-INH hFc IgG4m (IgG4 S241P) fusion protein; truncated (Tr) C1-INH hFc IgG4m (IgG4 S241P) fusion protein.

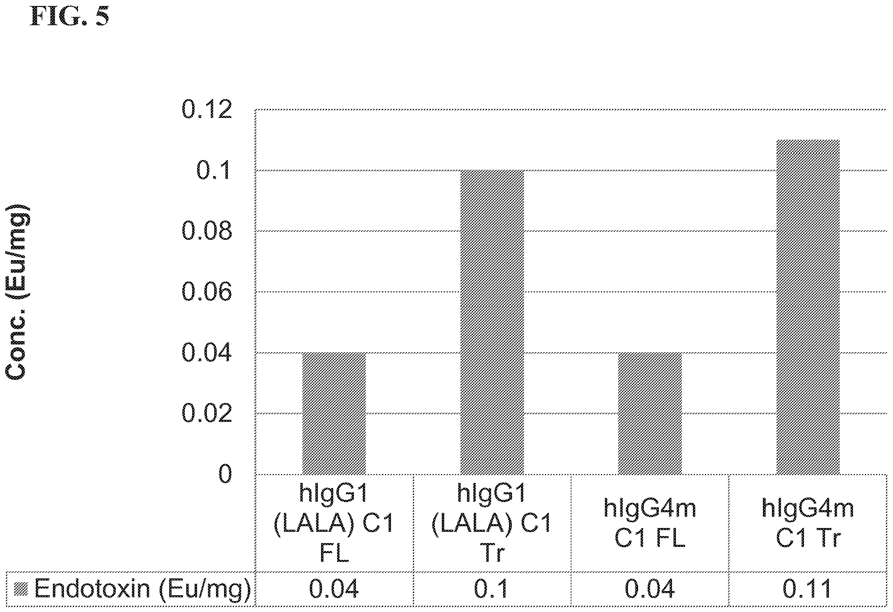

FIG. 5 is a chart depicting the amount of endotoxin detected in the purified samples of full length (FL) C1-INH hFc IgG1 LALA fusion protein; truncated (Tr) C1-INH hFc IgG1 LALA fusion protein; full length (FL) C1-INH hFc IgG4m (IgG4 S241P) fusion protein; truncated (Tr) C1-INH hFc IgG4m (IgG4 S241P) fusion protein.

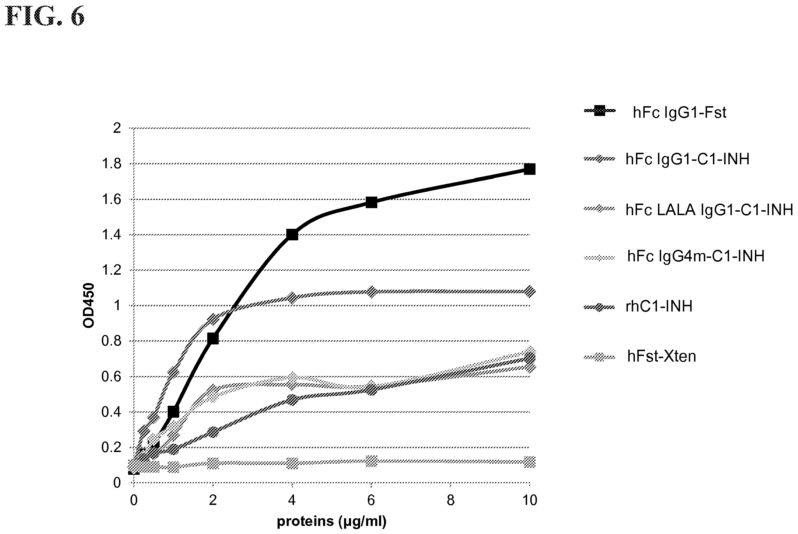

FIG. 6 shows the results of an exemplary C1q binding ELISA for the hFc IgG1 - C1-INH, hFc LALA IgG1-C1-INH, and hFc IgG4m-C1-INH fusions, recombinant human C1-INH (rhC1-INH) (expressed in 1080 cells), and IgG1 Fc-human follistatin(hFc IgG1-hFst-XTEN) fusion as a positive control and human follistatin-Xten (hFst-XTEN) fusion as a negative control.

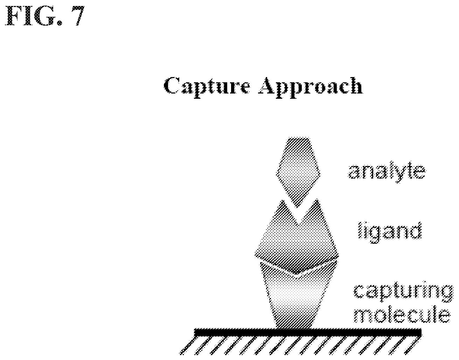

FIG. 7 is a schematic of the surface plasmon resonance (SPR) Biacore capture approach utilized to measure binding of the exemplary fusion constructs to the extracellular domain of the FcγR1.

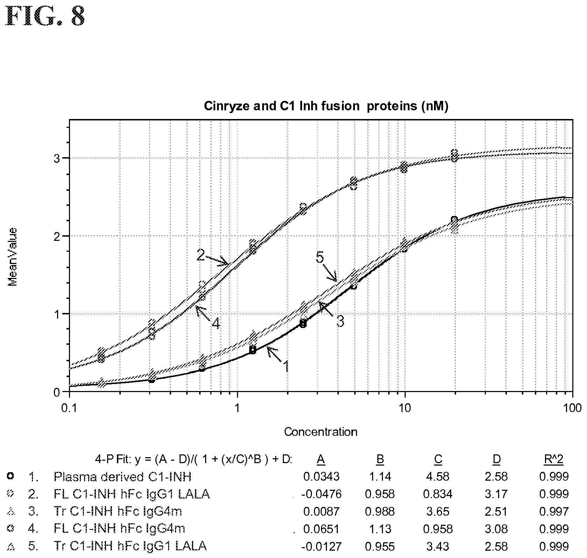

FIG. 8 shows the results of SPR analysis of exemplary effector dead Fc fusion constructs: full length (FL) C1-INH hFc IgG1 LALA fusion protein; truncated (Tr) C1-INH hFc IgG1 LALA fusion protein; full length (FL) C1-INH hFc IgG4m (IgG4 S241P) fusion protein; truncated (Tr) C1-INH hFc IgG4m (IgG4 S241P) fusion protein. Plasma-derived C1-INH was included as a positive control.

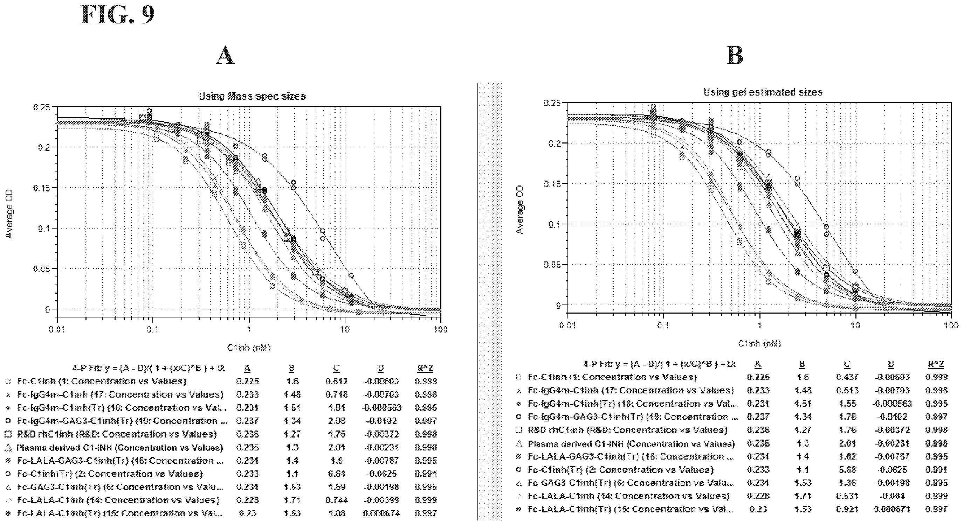

FIGs. 9A and 9B present the results of an assay to measure the ability of the effector dead constructs to inhibit C1s cleavage of a colorimetric peptide. FIG. 9A shows the titration curve for each of the exemplary effector dead constructs tested using the mass spectrometry calculated molecular weights for each of the constructs. FIG. 9B shows the titration curve for each of the exemplary effector dead constructs tested using the gel estimated molecular weights for each of the constructs.

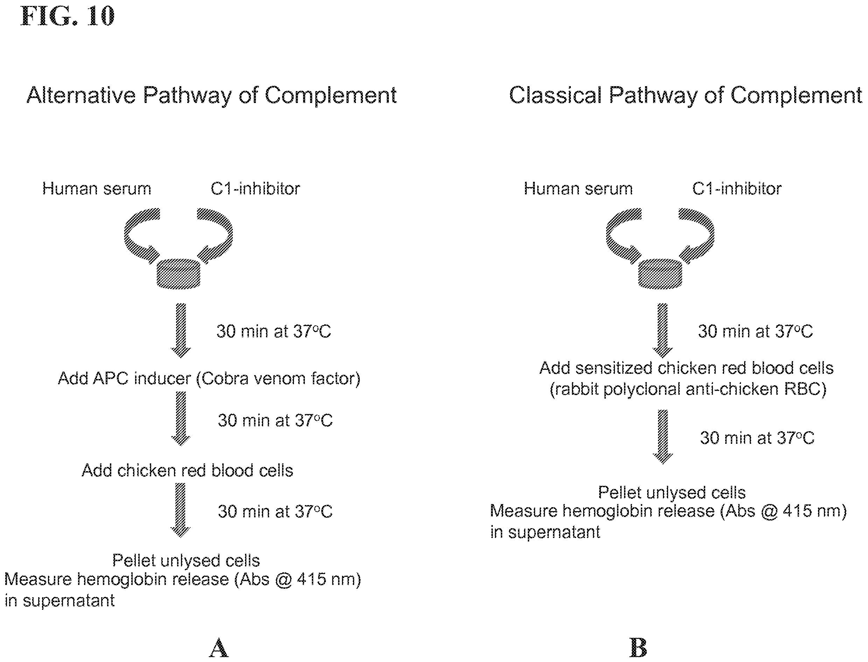

FIG. 10A depicts a schematic overview of a hemolysis assay to measure the activation of the alternative pathway of complement (APC). FIG. 10B depicts a schematic overview of a hemolysis assay to measure the activation of the classical pathway of complement.

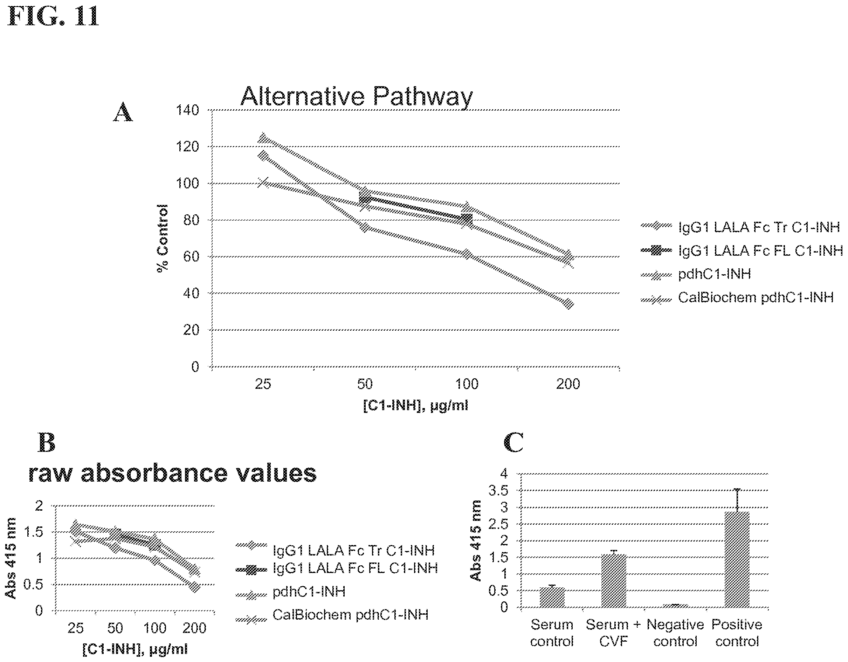

FIGs. 11A, 11B, and 11C show the results of a hemolysis assay to measure the activation of the alternative pathway of complement. FIG. 11A shows the results comparing truncated (Tr) C1-INH hFc IgG1 LALA fusion protein, full length (FL) C1-INH hFc IgG1 LALA fusion protein , a plasma-derived C1-INH preparation, and CalBiochem commercially available plasma-derived C1-INH. The absorbance readings are plotted as a percentage of the control by the concentration of the tested proteins. FIG. 11B shows the plot of raw data collected for each of the samples. FIG. 11C shows the plot of the controls run in the assay.

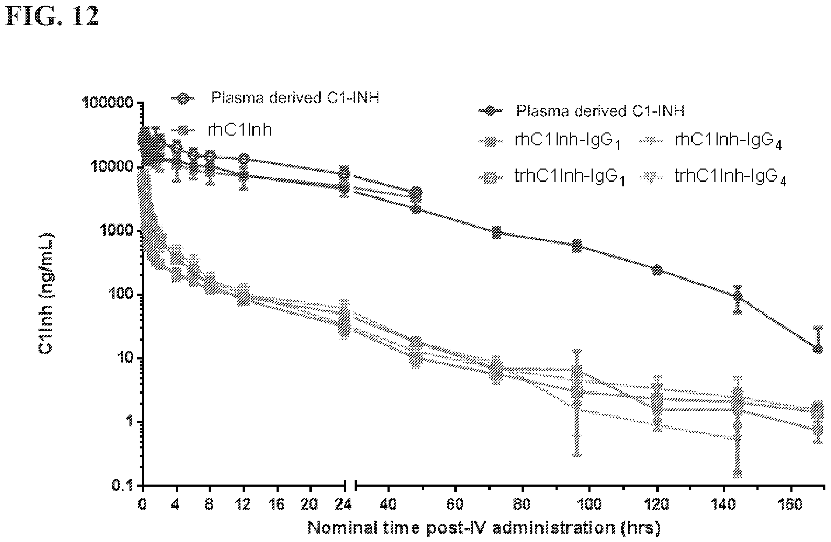

FIG. 12 shows exemplary results of a Rabbit PK study of the effector dead fusion constructs compared with two preparations of plasma-derived C1-inhibitor and HT1080 expressed recombinant C1-INH. IV administration of plasma-derived C1-INH exhibits a monophasic serum concentration-time profile.

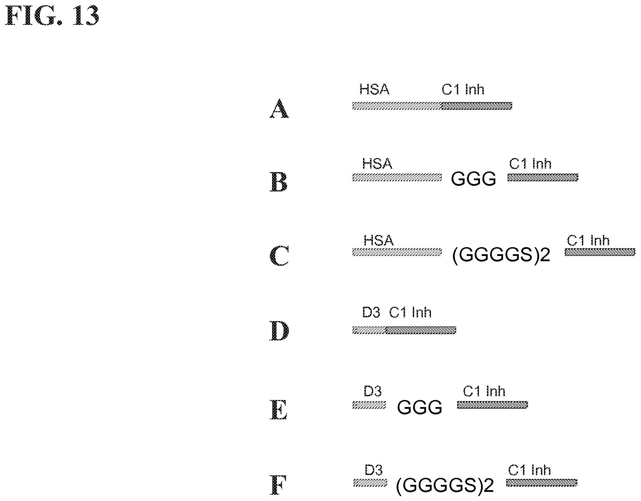

FIGs. 13A-13F are schematic representations of exemplary albumin fusion constructs generated. FIG. 13A is a schematic representation of a human serum albumin (HSA) fused to C1-INH. FIG. 13B is a schematic representation of HSA joined to C1-INH via a GGG linker. FIG. 13C is a schematic representation of HSA joined to C1-INH via a (GGGGS)2 linker. FIG. 13D is a schematic representation of the D3 domain of HSA fused to C1-INH. FIG. 13E is a schematic representation of the D3 domain of HSA joined to C1-INH via a GGG linker. FIG. 13F is a schematic representation of the D3 domain of HSA joined to C1-INH via a (GGGGS)2 linker.

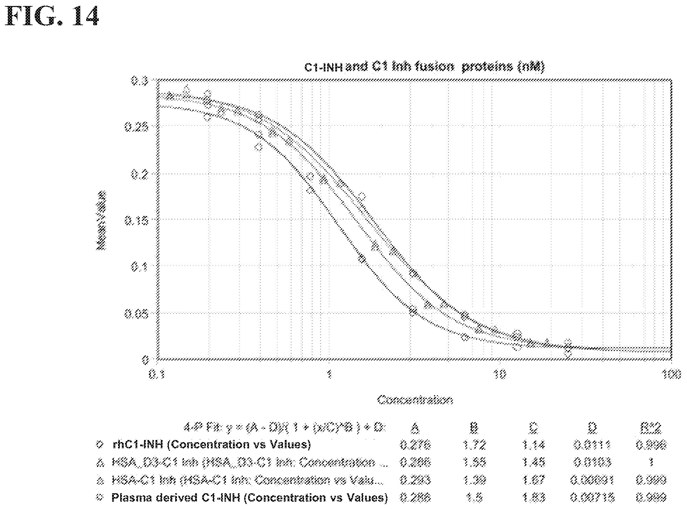

FIG. 14 present the results of an assay to measure the ability of some exemplary albumin C1-INH fusion constructs to inhibit C1s cleavage of a colorimetric peptide, including a plasma-derived C1-INH and HT1080 expressed recombinant C1-INH for comparison.

### DEFINITIONS

In order for the present invention to be more readily understood, certain terms are first defined below. Additional definitions for the following terms and other terms are set forth throughout the specification.

*Animal*: As used herein, the term "animal" refers to any member of the animal kingdom. In some embodiments, "animal" refers to humans, at any stage of development. In some embodiments, "animal" refers to non-human animals, at any stage of development. In certain embodiments, the non-human animal is a mammal (e.g., a rodent, a mouse, a rat, a rabbit, a monkey, a dog, a cat, a sheep, cattle, a primate, and/or a pig). In some embodiments, animals include, but are not limited to, mammals, birds, reptiles, amphibians, fish, insects, and/or worms. In some embodiments, an animal may be a transgenic animal, genetically-engineered animal, and/or a clone.

*Approximately or about:* As used in this application, the terms "about" and "approximately" are used as equivalents. Any numerals used in this application with or without about/approximately are meant to cover any normal fluctuations appreciated by one of ordinary skill in the relevant art. As used herein, the term "approximately" or "about," as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

*Bioavailability*: As used herein, the term "bioavailability" generally refers to the percentage of the administered dose that reaches the blood stream of a subject.

*Biologically active:* As used herein, the phrase "biologically active" refers to a characteristic of any agent that has activity in a biological system, and particularly in an organism. For instance, an agent that, when administered to an organism, has a biological effect on that organism, is considered to be biologically active. In particular embodiments, where a peptide is biologically active, a portion of that peptide that shares at least one biological activity of the peptide is typically referred to as a "biologically active" portion.

*Carrier or diluent*: As used herein, the terms "carrier" and "diluent" refers to a pharmaceutically acceptable (e.g., safe and non-toxic for administration to a human) carrier or diluting substance useful for the preparation of a pharmaceutical formulation. Exemplary diluents include sterile water, bacteriostatic water for injection (BWFI), a pH buffered solution (e.g. phosphate-buffered saline), sterile saline solution, Ringer's solution or dextrose solution.

*C1-inhibitor or C1 esterase inhibitor or C1-INH*: As used herein, the term "C1-inhibitor" or "C1 esterase inhibitor" or "C1-INH" can all be used interchangeably and refer to any wild-type or modified C1-INH polypeptides (e.g., C1-INH proteins with amino acid mutations, deletions, insertions, and/or fusion proteins) that retain substantial C1-INH biological activity unless otherwise specified. C1-INH may be recombinantly expressed in cells. In certain embodiments, the C1-INH is expressed in mammalian cells, preferably CHO cells or human cells.

*Functional equivalent or derivative:* As used herein, the term "functional equivalent" or "functional derivative" denotes, in the context of a functional derivative of an amino acid sequence, a molecule that retains a biological activity (either function or structural) that is substantially similar to that of the original sequence. A functional derivative or equivalent may be a natural derivative or is prepared synthetically. Exemplary functional derivatives include amino acid sequences having substitutions, deletions, or additions of one or more amino acids, provided that the biological activity of the protein is conserved. The substituting amino acid desirably has chemico-physical properties which are similar to that of the substituted amino acid. Desirable similar chemico-physical properties include, similarities in charge, bulkiness, hydrophobicity, hydrophilicity, and the like.

*Fusion protein:* As used herein, the term "fusion protein" or "chimeric protein" refers to a protein created through the joining of two or more originally separate proteins, or portions thereof. In some embodiments, a linker or spacer will be present between each protein.

*Half-Life*: As used herein, the term "half-life" is the time required for a quantity such as protein concentration or activity to fall to half of its value as measured at the beginning of a time period.

*Hereditary angioedema or HAE:* As used herein, the term "hereditary angioedema" or "HAE" refers to a blood disorder characterized by unpredictable and recurrent attacks of inflammation. HAE is typically associated with C1-INH deficiency, which may be the result of low levels of C1-INH or C1-INH with impaired or decreased activity. Symptoms include, but are not limited to, swelling that can occur in any part of the body, such as the face, extremities, genitals, gastrointestinal tract and upper airways.

*Improve, increase, or reduce:* As used herein, the terms "improve," "increase" or "reduce," or grammatical equivalents, indicate values that are relative to a baseline measurement, such as a measurement in the same individual prior to initiation of the treatment described herein, or a measurement in a control subject (or multiple control subject) in the absence of the treatment described herein. A "control subject" is a subject afflicted with the same form of disease as the subject being treated, who is about the same age as the subject being treated.

*In Vitro:* As used herein, the term *"in vitro"* refers to events that occur in an artificial environment, *e.g*., in a test tube or reaction vessel, in cell culture, *etc*., rather than within a multi-cellular organism.

*In Vivo*: As used herein, the term *"in vivo"* refers to events that occur within a multi-cellular organism, such as a human and a non-human animal. In the context of cell-based systems, the term may be used to refer to events that occur within a living cell (as opposed to, for example, *in vitro* systems).

*Linker*: As used herein, the term "linker" refers to, in a fusion protein, an amino acid sequence other than that appearing at a particular position in the natural protein and is generally designed to be flexible or to interpose a structure, such as an α-helix, between two protein moieties. A linker is also referred to as a spacer. A linker or a spacer typically does not have biological function on its own.

*Polypeptide*: The term "polypeptide" as used herein refers to a sequential chain of amino acids linked together via peptide bonds. The term is used to refer to an amino acid chain of any length, but one of ordinary skill in the art will understand that the term is not limited to lengthy chains and can refer to a minimal chain comprising two amino acids linked together via a peptide bond. As is known to those skilled in the art, polypeptides may be processed and/or modified. As used herein, the terms "polypeptide" and "peptide" are used inter-changeably.

*Prevent*: As used herein, the term "prevent" or "prevention", when used in connection with the occurrence of a disease, disorder, and/or condition, refers to reducing the risk of developing the disease, disorder and/or condition. See the definition of "risk."

*Protein*: The term "protein" as used herein refers to one or more polypeptides that function as a discrete unit. If a single polypeptide is the discrete functioning unit and does not require permanent or temporary physical association with other polypeptides in order to form the discrete functioning unit, the terms "polypeptide" and "protein" may be used interchangeably. If the discrete functional unit is comprised of more than one polypeptide that physically associate with one another, the term "protein" refers to the multiple polypeptides that are physically coupled and function together as the discrete unit.

*Risk*: As will be understood from context, a "risk" of a disease, disorder, and/or condition comprises a likelihood that a particular individual will develop a disease, disorder, and/or condition (e.g., muscular dystrophy). In some embodiments, risk is expressed as a percentage. In some embodiments, risk is from 0,1, 2, 3, 4, 5, 6, 7, 8, 9, 10, 20, 30, 40, 50, 60, 70, 80, 90 up to 100%. In some embodiments risk is expressed as a risk relative to a risk associated with a reference sample or group of reference samples. In some embodiments, a reference sample or group of reference samples have a known risk of a disease, disorder, condition and/or event (e.g., muscular dystrophy). In some embodiments a reference sample or group of reference samples are from individuals comparable to a particular individual. In some embodiments, relative risk is 0,1, 2, 3, 4, 5, 6, 7, 8, 9, 10, or more.

*Subject*: As used herein, the term "subject" refers to a human or any non-human animal (e.g., mouse, rat, rabbit, dog, cat, cattle, swine, sheep, horse or primate). A human includes pre- and post-natal forms. In many embodiments, a subject is a human being. A subject can be a patient, which refers to a human presenting to a medical provider for diagnosis or treatment of a disease. The term "subject" is used herein interchangeably with "individual" or "patient." A subject can be afflicted with or is susceptible to a disease or disorder but may or may not display symptoms of the disease or disorder.

*Substantially:* As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

*Substantial homology*: The phrase "substantial homology" is used herein to refer to a comparison between amino acid or nucleic acid sequences. As will be appreciated by those of ordinary skill in the art, two sequences are generally considered to be "substantially homologous" if they contain homologous residues in corresponding positions. Homologous residues may be identical residues. Alternatively, homologous residues may be non-identical residues will appropriately similar structural and/or functional characteristics. For example, as is well known by those of ordinary skill in the art, certain amino acids are typically classified as "hydrophobic" or "hydrophilic" amino acids, and/or as having "polar" or "non-polar" side chains Substitution of one amino acid for another of the same type may often be considered a "homologous" substitution.

As is well known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences. Exemplary such programs are described in Altschul, et al., Basic local alignment search tool, J. Mol. Biol., 215(3): 403-410, 1990; Altschul, et al., Methods in Enzymology; Altschul, et al., "Gapped BLAST and PSI-BLAST: a new generation of protein database search programs", Nucleic Acids Res. 25:3389-3402, 1997; Baxevanis, et al., Bioinformatics : A Practical Guide to the Analysis of Genes and Proteins, Wiley, 1998; and Misener, et al., (eds.), Bioinformatics Methods and Protocols (Methods in Molecular Biology, Vol. 132), Humana Press, 1999. In addition to identifying homologous sequences, the programs mentioned above typically provide an indication of the degree of homology. In some embodiments, two sequences are considered to be substantially homologous if at least 50%, 55%, 60%, 65%, 70%, 75%, 80%, 85%, 90%, 91%, 92%, 93%, 94%, 95%, 96%, 97%, 98%, 99% or more of their corresponding residues are homologous over a relevant stretch of residues. In some embodiments, the relevant stretch is a complete sequence. In some embodiments, the relevant stretch is at least 10, 15, 20, 25, 30, 35, 40, 45, 50, 55, 60, 65, 70, 75, 80, 85, 90, 95, 100, 125, 150, 175, 200, 225, 250, 275, 300, 325, 350, 375, 400, 425, 450, 475, 500 or more residues.

*Substantial identity*: The phrase "substantial identity" is used herein to refer to a comparison between amino acid or nucleic acid sequences. As will be appreciated by those of ordinary skill in the art, two sequences are generally considered to be "substantially identical" if they contain identical residues in corresponding positions. As is well known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences. Exemplary such programs are described in Altschul, et al., Basic local alignment search tool, J. Mol. Biol., 215(3): 403-410, 1990; Altschul, et al., Methods in Enzymology; Altschul et al., Nucleic Acids Res. 25:3389-3402, 1997; Baxevanis et al., Bioinformatics : A Practical Guide to the Analysis of Genes and Proteins, Wiley, 1998; and Misener, et al., (eds.), Bioinformatics Methods and Protocols (Methods in Molecular Biology, Vol. 132), Humana Press, 1999. In addition to identifying identical sequences, the programs mentioned above typically provide an indication of the degree of identity. In some embodiments, two sequences are considered to be substantially identical if at least 50%, 55%, 60%, 65%, 70%, 75%, 80%, 85%, 90%, 91%, 92%, 93%, 94%, 95%, 96%, 97%, 98%, 99% or more of their corresponding residues are identical over a relevant stretch of residues. In some embodiments, the relevant stretch is a complete sequence. In some embodiments, the relevant stretch is at least 10, 15, 20, 25, 30, 35, 40, 45, 50, 55, 60, 65, 70, 75, 80, 85, 90, 95, 100, 125, 150, 175, 200, 225, 250, 275, 300, 325, 350, 375, 400, 425, 450, 475, 500 or more residues.

*Suffering from:* An individual who is "suffering from" a disease, disorder, and/or condition has been diagnosed with or displays one or more symptoms of the disease, disorder, and/or condition.

*Susceptible to:* An individual who is "susceptible to" a disease, disorder, and/or condition has not been diagnosed with the disease, disorder, and/or condition. In some embodiments, an individual who is susceptible to a disease, disorder, and/or condition may not exhibit symptoms of the disease, disorder, and/or condition. In some embodiments, an individual who is susceptible to a disease, disorder, condition, or event (for example, DMD) may be characterized by one or more of the following: (1) a genetic mutation associated with development of the disease, disorder, and/or condition; (2) a genetic polymorphism associated with development of the disease, disorder, and/or condition; (3) increased and/or decreased expression and/or activity of a protein associated with the disease, disorder, and/or condition; (4) habits and/or lifestyles associated with development of the disease, disorder, condition, and/or event (5) having undergone, planning to undergo, or requiring a transplant. In some embodiments, an individual who is susceptible to a disease, disorder, and/or condition will develop the disease, disorder, and/or condition. In some embodiments, an individual who is susceptible to a disease, disorder, and/or condition will not develop the disease, disorder, and/or condition.

*Therapeutically effective amount*: As used herein, the term "therapeutically effective amount" of a therapeutic agent means an amount that is sufficient, when administered to a subject suffering from or susceptible to a disease, disorder, and/or condition, to treat, diagnose, prevent, and/or delay the onset of the symptom(s) of the disease, disorder, and/or condition. It will be appreciated by those of ordinary skill in the art that a therapeutically effective amount is typically administered via a dosing regimen comprising at least one unit dose.

*Treating*: As used herein, the term "treat," "treatment," or "treating" refers to any method used to partially or completely alleviate, ameliorate, relieve, inhibit, prevent, delay onset of, reduce severity of and/or reduce incidence of one or more symptoms or features of a particular disease, disorder, and/or condition. Treatment may be administered to a subject who does not exhibit signs of a disease and/or exhibits only early signs of the disease for the purpose of decreasing the risk of developing pathology associated with the disease.

### DETAILED DESCRIPTION

The present invention provides, among other things, methods and compositions for treating complement-mediated disorders, including Hereditary angioedema (HAE), based on C1-INH as a protein therapeutic.

Purpose of fusion is to extend half-life. Result is decrease in dose frequency, increase in prophylactic efficacy.

Various aspects of the invention are described in detail in the following sections. The use of sections is not meant to limit the invention. Each section can apply to any aspect of the invention. In this application, the use of "or" means "and/or" unless stated otherwise.

### C1-INH

Human C1-INH is an important anti-inflammatory plasma protein with a wide range of inhibitory and non-inhibitory biological activities. By sequence homology, structure of its C-terminal domain, and mechanism of protease inhibition, it belongs to the serpin superfamily, the largest class of plasma protease inhibitors, which also includes antithrombin, α1-proteinase inhibitor, plasminogen activator inhibitor, and many other structurally similar proteins that regulate diverse physiological systems. C1-INH is an inhibitor of proteases in the complement system, the contact system of kinin generation, and the intrinsic coagulation pathway. Cai, S. & Davis, A. E., Complement Regulatory Protein C1 Inhibitor Binds to Selectins and Interferes with Endothelial-Leukocyte Adhesion, J Immunol, 171:4786-4791 (2003). Specifically, C1-INH has been shown to inhibit C1r and C1s of the complement system. C1-INH is also a major regulator of coagulation factors XI and XII, as well as kallikrein and other serine proteases of the coagulation and fibrinolytic systems including tissue type plasminogen activator and plasmin.

Low plasma content of C1-INH or its dysfunction result in the activation of both complement and contact plasma cascades, and may affect other systems as well. A decrease in C1-INH plasma content to levels lower than 55 µg/mL (~25% of normal) has been shown to induce spontaneous activation of C1.

A schematic depicting the structure of C1-INH is provided in FIG. 1A. The signal peptide, N-terminal domain, and serpin domain are shown. C1-INH is The 22 amino acid signal peptide is required for secretion and cleaved from the rest of the C1-INH protein. C1-INH has two domains: a C-terminal domain having 365 amino acids, which is a typical serpin domain, and an N-terminal domain having 113 amino acids. The protein is stabilized by two disulfide bridges which connect the domains. These disulfide bridges are formed by Cys101 of the N-terminal domain which forms a disulfide bond with Cys406 of the C-terminal (serpin) domain and Cys108 of the N-terminal domain which forms a disulfide bond with Cys183 of C-terminal domain. The serpin domain is responsible for the protease activity of C1-INH. P1-P1' denotes the Arg444-Thr445 scissile bond.

More than 26% of the weight of the glycosylated protein is carbohydrate. The glycans are unevenly distributed over human C1-INH. The N-terminus is heavily glycosylated, having three N-linked (shown as long vertical lines with diamond heads) and at least seven O-linked (shown as short vertical lines) carbohydrate groups. Three N-attached glycans are attached to asparagine residues Asn216, Asn231, and Asn330 in the serpin domain (shown as long vertical lines with diamond heads). Although the functional role of the exceptionally long and heavily glycosylated N-terminal domain is still unclear, it may be essential for the protein's conformational stability, recognition, affinity to endotoxins and selectins, and clearance. The intrinsic heterogeneity of the carbohydrate moiety greatly contributes to the heterogeneity of the whole C1-INH, one of the reasons why production of a recombinant C1-INH mimicking the properties of plasma-derived C1-INH is difficult.

As used herein, C1-INH fusion proteins suitable for the present invention comprise any wild-type and modified C1-INH polypeptides (e.g., C1-INH proteins with amino acid mutations, deletions, truncations, and/or insertions) that retain substantial C1-INH biological activity. Typically, a C1-INH fusion protein is produced using recombinant technology.

Typically, a suitable recombinant C1-INH fusion protein has an *in vivo* half-life of or greater than about 12 hours, 18 hours, 24 hours, 36 hours, 2 days, 2.5 days, 3 days, 3.5 days, 4 days, 4.5 days, 5 days, 5.5 days, 6 days, 6.5 days, 7 days, 7.5 days, 8 days, 8.5 days, 9 days, 9.5 days, or 10 days. In some embodiments of the present disclosure, a recombinant C1-INH fusion protein has an *in vivo* half-life of between 0.5 and 10 days, between 1 day and 10 days, between 1 day and 9 days, between 1 day and 8 days, between 1 day and 7 days, between 1 day and 6 days, between 1 day and 5 days, between 1 day and 4 days, between 1 day and 3 days, between 2 days and 10 days, between 2 days and 9 days, between 2 days and 8 days, between 2 days and 7 days, between 2 days and 6 days, between 2 days and 5 days, between 2 days and 4 days, between 2 day and 3 days, between 2.5 days and 10 days, between 2.5 days and 9 days, between 2.5 days and 8 days, between 2.5 days and 7 days, between 2.5 days and 6 days, between 2.5 days and 5 days, between 2.5 days and 4 days, between 3 days and 10 days, between 3 days and 9 days, between 3 days and 8 days, between 3 days and 7 days, between 3 days and 6 days, between 3 days and 5 days, between 3 days and 4 days, between 3.5 days and 10 days, between 3.5 days and 9 days, between 3.5 days and 8 days, between 3.5 days and 7 days, between 3.5 days and 6 days, between 3.5 days and 5 days, between 3.5 days and 4 days, between 4 days and 10 days, between 4 days and 9 days, between 4 days and 8 days, between 4 days and 7 days, between 4 days and 6 days, between 4 days and 5 days, between 4.5 days and 10 days, between 4.5 days and 9 days, between 4.5 days and 8 days, between 4.5 days and 7 days, between 4.5 days and 6 days, between 4.5 days and 5 days, between 5 days and 10 days, between 5 days and 9 days, between 5 days and 8 days, between 5 days and 7 days, between 5 days and 6 days, between 5.5 days and 10 days, between 5.5 days and 9 days, between 5.5 days and 8 days, between 5.5 days and 7 days, between 5.5 days and 6 days, between 6 days and 10 days, between 7 days and 10 days, between 8 days and 10 days, between 9 days and 10 days.

Typically, a suitable recombinant C1-INH fusion protein has an *in vivo* half-life of or greater than about 4 days, 4.5 days, 5 days, 5.5 days, 6 days, 6.5 days, 7 days, 7.5 days, 8 days, 8.5 days, 9 days, 9.5 days, or 10 days. In some embodiments of the present disclosure, a recombinant C1-INH fusion protein has an *in vivo* half-life of between 4 days and 9 days, between 4 days and 8 days, between 4 days and 7 days, between 4 days and 6 days, between 4 days and 5 days, between 4.5 days and 10 days, between 4.5 days and 9 days, between 4.5 days and 8 days, between 4.5 days and 7 days, between 4.5 days and 6 days, between 4.5 days and 5 days, between 5 days and 10 days, between 5 days and 9 days, between 5 days and 8 days, between 5 days and 7 days, between 5 days and 6 days, between 5.5 days and 10 days, between 5.5 days and 9 days, between 5.5 days and 8 days, between 5.5 days and 7 days, between 5.5 days and 6 days, between 6 days and 10 days, between 7 days and 10 days, between 8 days and 10 days, between 9 days and 10 days.

In some embodiments of the present disclosure, a recombinant C1-INH polypeptide includes an amino acid sequence at least 50% (e.g., at least 55%, 60%, 65%, 70%, 75%, 80%, 85%, 90%, 95%, 96%, 97%, 98%, 99%, or 100%) identical to the wild-type human C1-INH protein (amino acids 1-478) (amino acids 1-97 are underlined):

NPNATSSSSQDPESLQDRGEGKVATTVISKMLFVEPILEVSSLPTTNSTTNSATKITANTT
DEPTTQPTTEPTTQPTIQPTQPTTQLPTDSPTQPTTGSFCPGPVTLCSDLESHSTEAVLGDA
LVDFSLKLYHAFSAMKKVETNMAFSPFSIASLLTQVLLGAGENTKTNLESILSYPKDFTC
VHQALKGFTTKGVTSVSQIFHSPDLAIRDTFVNASRTLYSSSPRVLSNNSDANLELINTW
VAKNTNNKISRLLDSLPSDTRLVLLNAIYLSAKWKTTFDPKKTRMEPFHFKNSVIKVPM
MNSKKYPVAHFIDQTLKAKVGQLQLSHNLSLVILVPQNLKHRLEDMEQALSPSVFKAIM
EKLEMSKFQPTLLTLPRIKVTTSQDMLSIMEKLEFFDFSYDLNLCGLTEDPDLQVSAMQH
QTVLELTETGVEAAAASAISVARTLLVFEVQQPFLFVLWDQQHKFPVFMGRVYDPRA
(SEQ ID NO:1).

In some embodiments of the present disclosure, a recombinant C1-INH polypeptide includes an amino acid sequence at least 50% (e.g., at least 55%, 60%, 65%, 70%, 75%, 80%, 85%, 90%, 95%, 96%, 97%, 98%, 99%, or 100%) identical to the wild-type human C1-INH protein (amino acids 98-478):

GSFCPGPVTLCSDLESHSTEAVLGDALVDFSLKLYHAFSAMKKVETNMAFSPFSIASLLT
QVLLGAGENTKTNLESILSYPKDFTCVHQALKGFTTKGVTSVSQIFHSPDLAIRDTFVNA
SRTLYSSSPRVLSNNSDANLELINTWVAKNTNNKISRLLDSLPSDTRLVLLNAIYLSAKW
KTTFDPKKTRMEPFHFKNSVIKVPMMNSKKYPVAHFIDQTLKAKVGQLQLSHNLSLVIL
VPQNLKHRLEDMEQALSPSVFKAIMEKLEMSKFQPTLLTLPRIKVTTSQDMLSIMEKLEF
FDFSYDLNLCGLTEDPDLQVSAMQHQTVLELTETGVEAAAASAISVARTLLVFEVQQPF
LFVLWDQQHKFPVFMGRVYDPRA (SEQ ID NO:2).

As disclosed herein, SEQ ID NO: 1 represents the canonical amino acid sequence for the human C1-INH protein. In some embodiments, a C1-INH polypeptide may be a truncated C1-INH such as SEQ ID NO:2. In some embodiments of the present disclosure, a suitable recombinant C1-INH polypeptide may be a homologue or an analogue of a wild-type or naturally-occurring protein. For example, a homologue or an analogue of human wild-type or naturally-occurring C1-INH polypeptide may contain one or more amino acid or domain substitutions, deletions, and/or insertions as compared to a wild-type or naturally-occurring C1-INH protein (e.g., SEQ ID NO:1), while retaining substantial C1-INH protein activity. Thus, in some embodiments of the present disclosure, a recombinant C1-INH polypeptide is substantially homologous to human C1-INH protein (SEQ ID NO:1). In some embodiments of the present disclosure, a recombinant C1-INH polypeptide has an amino acid sequence at least 50%, 55%, 60%, 65%, 70%, 75%, 80%, 85%, 90%, 91%, 92%, 93%, 94%, 95%, 96%, 97%, 98%, 99% or more homologous to SEQ ID NO:1. In some embodiments, a recombinant C1-INH polypeptide suitable for the present invention is substantially identical to human C1-INH protein (SEQ ID NO:1). In some embodiments of the present disclosure, a recombinant C1-INH polypeptide has an amino acid sequence at least 50%, 55%, 60%, 65%, 70%, 75%, 80%, 85%, 90%, 91%, 92%, 93%, 94%, 95%, 96%, 97%, 98%, 99% or more identical to SEQ ID NO:1.

Homologues or analogues of human C1-INH proteins can be prepared according to methods for altering polypeptide sequence known to one of ordinary skill in the art such as are found in references that compile such methods. As will be appreciated by those of ordinary skill in the art, two sequences are generally considered to be "substantially homologous" if they contain homologous residues in corresponding positions. Homologous residues may be identical residues. Alternatively, homologous residues may be non-identical residues will appropriately similar structural and/or functional characteristics. For example, as is well known by those of ordinary skill in the art, certain amino acids are typically classified as "hydrophobic" or "hydrophilic" amino acids, and/or as having "polar" or "non-polar" side chains. Substitution of one amino acid for another of the same type may often be considered a "homologous" substitution. In some embodiments, conservative substitutions of amino acids include substitutions made among amino acids within the following groups: (a) M, I, L, V; (b) F, Y, W; (c) K, R, H; (d) A, G; (e) S, T; (f) Q, N; and (g) E, D. In some embodiments, a "conservative amino acid substitution" refers to an amino acid substitution that does not alter the relative charge or size characteristics of the protein in which the amino acid substitution is made.

As is well known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences. Exemplary such programs are described in Altschul, et al., Basic local alignment search tool, J. Mol. Biol., 215(3): 403-410, 1990; Altschul, et al., Methods in Enzymology*;* Altschul, et al., "Gapped BLAST and PSI-BLAST: a new generation of protein database search programs", Nucleic Acids Res. 25:3389-3402, 1997; Baxevanis, et al., Bioinformatics : A Practical Guide to the Analysis of Genes and Proteins, Wiley, 1998; and Misener, et al., (eds.), Bioinformatics Methods and Protocols (Methods in Molecular Biology, Vol. 132), Humana Press, 1999. In addition to identifying homologous sequences, the programs mentioned above typically provide an indication of the degree of homology.

In some embodiments of the present disclosure, a recombinant C1-INH polypeptide suitable for the present disclosure contains one or more amino acid deletions, insertions or replacement as compared to a wild-type human C1-INH protein. For example, a suitable recombinant C1-INH polypeptide may be truncated, such as the polypeptide of SEQ ID NO:2.

In some embodiments of the present disclosure, a recombinant C1-INH polypeptide includes an amino acid sequence at least 50% (e.g., at least 55%, 60%, 65%, 70%, 75%, 80%, 85%, 90%, 95%, 96%, 97%, 98%, 99%, or 100%) identical to the truncated wild-type human C1-INH protein (amino acids 98-478):

GSFCPGPVTLCSDLESHSTEAVLGDALVDFSLKLYHAFSAMKKVETNMAFSPFSIASLLT
QVLLGAGENTKTNLESILSYPKDFTCVHQALKGFTTKGVTSVSQIFHSPDLAIRDTFVNA
SRTLYSSSPRVLSNNSDANLELINTWVAKNTNNKISRLLDSLPSDTRLVLLNAIYLSAKW
KTTFDPKKTRMEPFHFKNSVIKVPMMNSKKYPVAHFIDQTLKAKVGQLQLSHNLSLVIL
VPQNLKHRLEDMEQALSPSVFKAIMEKLEMSKFQPTLLTLPRIKVTTSQDMLSIMEKLEF
FDFSYDLNLCGLTEDPDLQVSAMQHQTVLELTETGVEAAAASAISVARTLLVFEVQQPF
LFVLWDQQHKFPVFMGRVYDPRA (SEQ ID NO:2).

In some embodiments of the present disclosure, a C1-INH polypeptide may be a truncated C1-INH while retaining substantial C1-INH protein activity, such as SEQ ID NO:2. Thus, in some embodiments of the present disclosure, a recombinant C1-INH polypeptide is substantially homologous to human C1-INH protein (SEQ ID NO:2). In some embodiments of the present disclosure, a recombinant C1-INH polypeptide has an amino acid sequence at least 50%, 55%, 60%, 65%, 70%, 75%, 80%, 85%, 90%, 91%, 92%, 93%, 94%, 95%, 96%, 97%, 98%, 99% or more homologous to SEQ ID NO:2. In some embodiments, a recombinant C1-INH polypeptide suitable for the present invention is substantially identical to human C1-INH protein (SEQ ID NO:2). In some embodiments of the present disclosure, a recombinant C1-INH polypeptide has an amino acid sequence at least 50%, 55%, 60%, 65%, 70%, 75%, 80%, 85%, 90%, 91%, 92%, 93%, 94%, 95%, 96%, 97%, 98%, 99% or more identical to SEQ ID NO:2.

The recombinant C1-INH fusion protein suitable for the present disclosure is a fusion protein between a C1-INH domain and another domain or moiety that typically can facilitate a therapeutic effect of C1-INH by, for example, enhancing or increasing half-life, stability, potency, and/or delivery of C1-INH protein, or reducing or eliminating immunogenicity, clearance, or toxicity. Such domains or moieties for a C1-INH fusion protein suitable for the present disclosure include but are not limited to Fc domains and albumin domains.

### Fc Domains

In some embodiments of the present disclosure, a suitable C1-INH fusion protein contains an Fc domain or a portion thereof that binds to the FcRn receptor. As a non-limiting example, a suitable Fc domain may be derived from an immunoglobulin subclass such as IgG. In some embodiments, a suitable Fc domain is derived from IgG1, IgG2, IgG3, or IgG4. In some embodiments of the present disclosure, a suitable Fc domain is derived from IgM, IgA, IgD, or IgE. Particularly suitable Fc domains include those derived from human or humanized antibodies. In some embodiments, a suitable Fc domain is a modified Fc portion, such as a modified human Fc portion.

### A. IgG1

C1-inhibitor Fc fusion proteins may exist as dimers, as shown in FIG. 1A.

In some embodiments of the present disclosure, an Fc domain includes an amino acid sequence at least 50% (e.g., at least 55%, 60%, 65%, 70%, 75%, 80%, 85%, 90%, 95%, 96%, 97%, 98%, 99%, or 100%) identical to the wild-type human IgG1 Fc domain:

DKTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGK (SEQ ID
NO:3).

### i. LALA

Wild type IgG1 has a low level of complement cascade activation, any may undesirable for patients suffering from a complement-mediated disorder. IgG choice for effector dead constructs which reduce or eliminate complement activation and antibody-dependent cell-mediated cytotoxicity (ADCC) activity may be important. Suitable Fc domains include IgG1 with mutations of L234A and L235A (LALA).

In some embodiments of the present disclosure, an Fc domain includes an amino acid sequence at least 50% (e.g., at least 55%, 60%, 65%, 70%, 75%, 80%, 85%, 90%, 95%, 96%, 97%, 98%, 99%, or 100%) identical to a human IgG1 Fc domain having a LALA mutation (mutated residues underlined):

DKTHTCPPCPAPEAAGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGK (SEQ ID
NO:4).

### ii. NHance

It is contemplated that improved binding between Fc domain and the FcRn receptor results in prolonged serum half-life. Thus, in some embodiments of the present disclosure, a suitable Fc domain comprises one or more amino acid mutations that lead to improved binding to FcRn. Various mutations within the Fc domain that effect improved binding to FcRn are known in the art and can be adapted to practice the present disclosure. In some embodiments of the present disclosure, a suitable Fc domain comprises one or more mutations at one or more positions corresponding to Thr 250, Met 252, Ser 254, Thr 256, Thr 307, Glu 380, Met 428, His 433, and/or Asn 434 of human IgG1.

For example, a suitable Fc domain for the present disclosure may contain mutations of H433K (His433Lys) and/or N434F (Asn434Phe). As a non-limiting example, a suitable Fc domain for the present disclosure may contain mutations H433K (His433Lys) and N434F (Asn434Phe) (Nhance). Additional amino acid substitutions that can be included in a Fc domain according to the present disclosure include those described in, e.g., U.S. Patent Nos. 6,277,375; 8,012,476; and 8,163,881.

In some embodiments of the present disclosure, an Fc domain includes an amino acid sequence at least 50% (e.g., at least 55%, 60%, 65%, 70%, 75%, 80%, 85%, 90%, 95%, 96%, 97%, 98%, 99%, or 100%) identical to a human IgG1 Fc domain having Nhance mutation (mutated residues underlined):

DKTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP

VLDSDGSFFLYSKLTVDKSRWQQGNVFSCSVMHEALKFHYTQKSLSLSPGK (SEQ ID
NO:5).

In some embodiments of the present disclosure, an Fc domain includes an amino acid sequence at least 50% (e.g., at least 55%, 60%, 65%, 70%, 75%, 80%, 85%, 90%, 95%, 96%, 97%, 98%, 99%, or 100%) identical to a human IgG1 Fc domain having both LALA and Nhance mutations (mutated residues underlined):

DKTHTCPPCPAPEAAGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALKFHYTQKSLSLSPGK (SEQ ID
NO:6).

In some embodiments of the present disclosure, an Fc domain includes an amino acid sequence at least 50% (e.g., at least 55%, 60%, 65%, 70%, 75%, 80%, 85%, 90%, 95%, 96%, 97%, 98%, 99%, or 100%) identical to a human IgG1 Fc domain with a signal peptide, and having Nhance mutation (signal peptide and mutated residues underlined):

METPAQLLFLLLLWLPDTTGDKTHTCPPCPAPELLGGPSVFLFPPKPKDTLMISRTPEVTC
VVVDVSHEDPEVKFNWYVDGVEVHNAKTKPREEQYNSTYRVVSVLTVLHQDWLNGK
EYKCKVSNKALPAPIEKTISKAKGQPREPQVYTLPPSRDELTKNQVSLTCLVKGFYPSDI
AVEWESNGQPENNYKTTPPVLDSDGSFFLYSKLTVDKSRWQQGNVFSCSVMHEALKFH
YTQKSLSLSPGK (SEQ ID NO:7).

In some embodiments of the present disclosure, an Fc domain includes an amino acid sequence at least 50% (e.g., at least 55%, 60%, 65%, 70%, 75%, 80%, 85%, 90%, 95%, 96%, 97%, 98%, 99%, or 100%) identical to a human IgG1 Fc domain with a signal peptide, and having both LALA and Nhance mutations (mutated residues underlined):

METPAQLLFLLLLWLPDTTGDKTHTCPPCPAPEAAGGPSVFLFPPKPKDTLMISRTPEVT
CVVVDVSHEDPEVKFNWYVDGVEVHNAKTKPREEQYNSTYRVVSVLTVLHQDWLNG
KEYKCKVSNKALPAPIEKTISKAKGQPREPQVYTLPPSRDELTKNQVSLTCLVKGFYPSD
IAVEWESNGQPENNYKTTPPVLDSDGSFFLYSKLTVDKSRWQQGNVFSCSVMHEALKF
HYTQKSLSLSPGK (SEQ ID NO:8).

In some embodiments of the present disclosure, an Fc domain includes an amino acid sequence at least 50% (e.g., at least 55%, 60%, 65%, 70%, 75%, 80%, 85%, 90%, 95%, 96%, 97%, 98%, 99%, or 100%) identical to the wild-type human IgG4 Fc domain:

ESKYGPPCPSCPAPEFLGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPP

VLDSDGSFFLYSRLTVDKSRWQEGNVFSCSVMHEALHNHYTQKSLSLSLGK (SEQ ID
NO:9).

In some embodiments of the present disclosure, an Fc domain includes an amino acid sequence at least 50% (e.g., at least 55%, 60%, 65%, 70%, 75%, 80%, 85%, 90%, 95%, 96%, 97%, 98%, 99%, or 100%) identical to the human IgG4 Fc domain having an S241P mutation (mutated residue underlined):

ESKYGPPCPPCPAPEFLGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSLGK (SEQ ID
NO: 10).

In some embodiments of the present disclosure, a suitable Fc domain comprises an amino acid sequence at least 50%, 55%, 60%, 65%, 70%, 75%, 80%, 85%, 90%, 91%, 92%, 93%, 94%, 95%, 96%, 97%, 98%, 99% or more homologous or identical to SEQ ID NO:3, SEQ ID NO:4, or SEQ ID NO:5, SEQ ID NO:6, SEQ ID NO:7, SEQ ID NO:8, SEQ ID NO:9, or SEQ ID NO:10.

### B. IgG4

In some embodiments IgG4 IgG choice for "effector dead" Fc fusion proteins is important in HAE (eliminate complement activation and ADCC). Specifically, IgG4 is reported to have lower complement activation than WT IgG1. Expression of native IgG4 results in mixed species of construct sizing. Accordingly, IgG4 S241P was chosen for this program due to the increased stability and maintenance of dimeric structure exhibited by IgG4 S241P mutants based on prior art.

The human IgG4 core hinge region sequence according to some embodiments of the invention comprises an S228P substitution according to the EU index as in Kabat. This substitution has also been referred to as S241P according to Kabat et al (1987 Sequences of proteins of immunological interest. United States Department of Health and Human Services, Washington DC.). The substitution has the effect of making the sequence of the core of the hinge region the same as that of a Wild-type IgG1 or IgG2 isotype antibody. With respect to the IgG4 isotype antibody, it results in the production of the homogenous form of the IgG4 antibody and hence abrogates the dissociation and reassociation of the heavy chains which often leads to the production of heterodimeric IgG4 antibodies. In some embodiments, IgG4 is preferred for stability at high concentrations.

### C. Exemplary C1-INH Fusion Proteins

In particular embodiments of the present disclosure, a suitable recombinant C1-INH fusion protein includes a C1-INH polypeptide, an Fc domain. wherein the C1-INH polypeptide comprises an amino acid sequence at least 50%, 55%, 60%, 65%, 70%, 75%, 80%, 85%, 90%, 91%, 92%, 93%, 94%, 95%, 96%, 97%, 98%, 99% or 100% identical to the wild-type human C1-INH polypeptide (SEQ ID NO:1) or a truncated C1-INH polypeptide (SEQ ID NO:2). In some embodiments of the present disclosure there is a linker that associates the C1-INH polypeptide with the Fc domain. In some embodiments of the present disclosure, a suitable recombinant C1-INH fusion protein has an *in vivo* half-life ranging from about 0.5-10 days (e.g., about 0.5-5.5 days, about 0.5-5 days, about 1-5 days, about 1.5-5 days, about 1.5-4.5 days, about 1.5-4.0 days, about 1.5-3.5 days, about 1.5-3 days, about 1.5-2.5 days, about 2-6 days, about 2-5.5 days, about 2-5 days, about 2-4.5 days, about 2-4 days, about 2-3.5 days, about 2-3 days). In some embodiments of the present disclosure, a suitable recombinant C1-INH fusion protein has an *in vivo* half-life ranging from about 2-10 days (e.g., ranging from about 2.5-10 days, from about 3-10 days, from about 3.5-10 days, from about 4-10 days, from about 4.5-10 days, from about 5-10 days, from about 3-8 days, from about 3.5-8 days, from about 4-8 days, from about 4.5-8 days, from about 5-8 days, from about 3-6 days, from about 3.5-6 days, from about 4-6 days, from about 4.5-6 days, from about 5-6 days).

Typically, a suitable recombinant C1-INH fusion protein has an *in vivo* half-life of or greater than about 12 hours, 18 hours, 24 hours, 36 hours, 2 days, 2.5 days, 3 days, 3.5 days, 4 days, 4.5 days, 5 days, 5.5 days, 6 days, 6.5 days, 7 days, 7.5 days, 8 days, 8.5 days, 9 days, 9.5 days, or 10 days. In some embodiments of the present disclosure, a recombinant C1-INH fusion protein has an *in vivo* half-life of between 0.5 and 10 days, between 1 day and 10 days, between 1 day and 9 days, between 1 day and 8 days, between 1 day and 7 days, between 1 day and 6 days, between 1 day and 5 days, between 1 day and 4 days, between 1 day and 3 days, between 2 days and 10 days, between 2 days and 9 days, between 2 days and 8 days, between 2 days and 7 days, between 2 days and 6 days, between 2 days and 5 days, between 2 days and 4 days, between 2 day and 3 days, between 2.5 days and 10 days, between 2.5 days and 9 days, between 2.5 days and 8 days, between 2.5 days and 7 days, between 2.5 days and 6 days, between 2.5 days and 5 days, between 2.5 days and 4 days, between 3 days and 10 days, between 3 days and 9 days, between 3 days and 8 days, between 3 days and 7 days, between 3 days and 6 days, between 3 days and 5 days, between 3 days and 4 days, between 3.5 days and 10 days, between 3.5 days and 9 days, between 3.5 days and 8 days, between 3.5 days and 7 days, between 3.5 days and 6 days, between 3.5 days and 5 days, between 3.5 days and 4 days, between 4 days and 10 days, between 4 days and 9 days, between 4 days and 8 days, between 4 days and 7 days, between 4 days and 6 days, between 4 days and 5 days, between 4.5 days and 10 days, between 4.5 days and 9 days, between 4.5 days and 8 days, between 4.5 days and 7 days, between 4.5 days and 6 days, between 4.5 days and 5 days, between 5 days and 10 days, between 5 days and 9 days, between 5 days and 8 days, between 5 days and 7 days, between 5 days and 6 days, between 5.5 days and 10 days, between 5.5 days and 9 days, between 5.5 days and 8 days, between 5.5 days and 7 days, between 5.5 days and 6 days, between 6 days and 10 days, between 7 days and 10 days, between 8 days and 10 days, between 9 days and 10 days.

In certain embodiments, as shown in FIG. 2A and FIG. 2B, Fc moieties may be directly fused to the N-terminal region of the full length (1-478 aa) as well as truncated (98-478) C1-inhibitor. As non-limiting examples of fusion proteins described herein, suitable C1-INH Fc fusion proteins may have an amino acid sequence shown below: 
DKTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGKNPNATSSS
SQDPESLQDRGEGKVATTVISKMLFVEPILEVSSLPTTNSTTNSATKITANTTDEPTTQPTT
EPTTQPTIQPTQPTTQLPTDSPTQPTTGSFCPGPVTLCSDLESHSTEAVLGDALVDFSLKL
YHAFSAMKKVETNMAFSPFSIASLLTQVLLGAGENTKTNLESILSYPKDFTCVHQALKG
FTTKGVTSVSQIFHSPDLAIRDTFVNASRTLYSSSPRVLSNNSDANLELINTWVAKNTNN
KISRLLDSLPSDTRLVLLNAIYLSAKWKTTFDPKKTRMEPFHFKNSVIKVPMMNSKKYP
VAHFIDQTLKAKVGQLQLSHNLSLVILVPQNLKHRLEDMEQALSPSVFKAIMEKLEMSK
FQPTLLTLPRIKVTTSQDMLSIMEKLEFFDFSYDLNLCGLTEDPDLQVSAMQHQTVLELT
ETGVEAAAASAISVARTLLVFEVQQPFLFVLWDQQHKFPVFMGRVYDPRA (SEQ ID
NO:11)
 or 
DKTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGKGSFCPGP
VTLCSDLESHSTEAVLGDALVDFSLKLYHAFSAMKKVETNMAFSPFSIASLLTQVLLGA
GENTKTNLESILSYPKDFTCVHQALKGFTTKGVTSVSQIFHSPDLAIRDTFVNASRTLYSS
SPRVLSNNSDANLELINTWVAKNTNNKISRLLDSLPSDTRLVLLNAIYLSAKWKTTFDPK
KTRMEPFHFKNSVIKVPMMNSKKYPVAHFIDQTLKAKVGQLQLSHNLSLVILVPQNLKH
RLEDMEQALSPSVFKAIMEKLEMSKFQPTLLTLPRIKVTTSQDMLSIMEKLEFFDFSYDL
NLCGLTEDPDLQVSAMQHQTVLELTETGVEAAAASAISVARTLLVFEVQQPFLFVLWD
QQHKFPVFMGRVYDPRA (SEQ ID NO:12)
 or 
DKTHTCPPCPAPEAAGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGKNPNATSSS
SQDPESLQDRGEGKVATTVISKMLFVEPILEVSSLPTTNSTTNSATKITANTTDEPTTQPTT
EPTTQPTIQPTQPTTQLPTDSPTQPTTGSFCPGPVTLCSDLESHSTEAVLGDALVDFSLKL
YHAFSAMKKVETNMAFSPFSIASLLTQVLLGAGENTKTNLESILSYPKDFTCVHQALKG
FTTKGVTSVSQIFHSPDLAIRDTFVNASRTLYSSSPRVLSNNSDANLELINTWVAKNTNN
KISRLLDSLPSDTRLVLLNAIYLSAKWKTTFDPKKTRMEPFHFKNSVIKVPMMNSKKYP
VAHFIDQTLKAKVGQLQLSHNLSLVILVPQNLKHRLEDMEQALSPSVFKAIMEKLEMSK
FQPTLLTLPRIKVTTSQDMLSIMEKLEFFDFSYDLNLCGLTEDPDLQVSAMQHQTVLELT
ETGVEAAAASAISVARTLLVFEVQQPFLFVLWDQQHKFPVFMGRVYDPRA (SEQ ID
NO:13)
 or 
DKTHTCPPCPAPEAAGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGKGSFCPGP
VTLCSDLESHSTEAVLGDALVDFSLKLYHAFSAMKKVETNMAFSPFSIASLLTQVLLGA
GENTKTNLESILSYPKDFTCVHQALKGFTTKGVTSVSQIFHSPDLAIRDTFVNASRTLYSS
SPRVLSNNSDANLELINTWVAKNTNNKISRLLDSLPSDTRLVLLNAIYLSAKWKTTFDPK
KTRMEPFHFKNSVIKVPMMNSKKYPVAHFIDQTLKAKVGQLQLSHNLSLVILVPQNLKH
RLEDMEQALSPSVFKAIMEKLEMSKFQPTLLTLPRIKVTTSQDMLSIMEKLEFFDFSYDL
NLCGLTEDPDLQVSAMQHQTVLELTETGVEAAAASAISVARTLLVFEVQQPFLFVLWD
QQHKFPVFMGRVYDPRA (SEQ ID NO:14)
 or 
ESKYGPPCPSCPAPEFLGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSLGKNPNATSSS
 
SQDPESLQDRGEGKVATTVISKMLFVEPILEVSSLPTTNSTTNSATKITANTTDEPTTQPTT
EPTTQPTIQPTQPTTQLPTDSPTQPTTGSFCPGPVTLCSDLESHSTEAVLGDALVDFSLKL
YHAFSAMKKVETNMAFSPFSIASLLTQVLLGAGENTKTNLESILSYPKDFTCVHQALKG
FTTKGVTSVSQIFHSPDLAIRDTFVNASRTLYSSSPRVLSNNSDANLELINTWVAKNTNN
KISRLLDSLPSDTRLVLLNAIYLSAKWKTTFDPKKTRMEPFHFKNSVIKVPMMNSKKYP
VAHFIDQTLKAKVGQLQLSHNLSLVILVPQNLKHRLEDMEQALSPSVFKAIMEKLEMSK
FQPTLLTLPRIKVTTSQDMLSIMEKLEFFDFSYDLNLCGLTEDPDLQVSAMQHQTVLELT
ETGVEAAAASAISVARTLLVFEVQQPFLFVLWDQQHKFPVFMGRVYDPRA (SEQ ID
NO:32)
 or 
ESKYGPPCPSCPAPEFLGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSLGKGSFCPGPV
TLCSDLESHSTEAVLGDALVDFSLKLYHAFSAMKKVETNMAFSPFSIASLLTQVLLGAG
ENTKTNLESILSYPKDFTCVHQALKGFTTKGVTSVSQIFHSPDLAIRDTFVNASRTLYSSS
PRVLSNNSDANLELINTWVAKNTNNKISRLLDSLPSDTRLVLLNAIYLSAKWKTTFDPKK
TRMEPFHFKNSVIKVPMMNSKKYPVAHFIDQTLKAKVGQLQLSHNLSLVILVPQNLKHR
LEDMEQALSPSVFKAIMEKLEMSKFQPTLLTLPRIKVTTSQDMLSIMEKLEFFDFSYDLN
LCGLTEDPDLQVSAMQHQTVLELTETGVEAAAASAISVARTLLVFEVQQPFLFVLWDQ
QHKFPVFMGRVYDPRA (SEQ ID NO:33)
 or 
ESKYGPPCPPCPAPEFLGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSLGKNPNATSSS
SQDPESLQDRGEGKVATTVISKMLFVEPILEVSSLPTTNSTTNSATKITANTTDEPTTQPTT
EPTTQPTIQPTQPTTQLPTDSPTQPTTGSFCPGPVTLCSDLESHSTEAVLGDALVDFSLKL
YHAFSAMKKVETNMAFSPFSIASLLTQVLLGAGENTKTNLESILSYPKDFTCVHQALKG
FTTKGVTSVSQIFHSPDLAIRDTFVNASRTLYSSSPRVLSNNSDANLELINTWVAKNTNN
KISRLLDSLPSDTRLVLLNAIYLSAKWKTTFDPKKTRMEPFHFKNSVIKVPMMNSKKYP
VAHFIDQTLKAKVGQLQLSHNLSLVILVPQNLKHRLEDMEQALSPSVFKAIMEKLEMSK
FQPTLLTLPRIKVTTSQDMLSIMEKLEFFDFSYDLNLCGLTEDPDLQVSAMQHQTVLELT
ETGVEAAAASAISVARTLLVFEVQQPFLFVLWDQQHKFPVFMGRVYDPRA (SEQ ID
NO:15)
 or

ESKYGPPCPPCPAPEFLGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSLGKGSFCPGPV
TLCSDLESHSTEAVLGDALVDFSLKLYHAFSAMKKVETNMAFSPFSIASLLTQVLLGAG
ENTKTNLESILSYPKDFTCVHQALKGFTTKGVTSVSQIFHSPDLAIRDTFVNASRTLYSSS
PRVLSNNSDANLELINTWVAKNTNNKISRLLDSLPSDTRLVLLNAIYLSAKWKTTFDPKK

TRMEPFHFKNSVIKVPMMNSKKYPVAHFIDQTLKAKVGQLQLSHNLSLVILVPQNLKHR
LEDMEQALSPSVFKAIMEKLEMSKFQPTLLTLPRIKVTTSQDMLSIMEKLEFFDFSYDLN
LCGLTEDPDLQVSAMQHQTVLELTETGVEAAAASAISVARTLLVFEVQQPFLFVLWDQ
QHKFPVFMGRVYDPRA (SEQ ID NO:16).

In some embodiments of the present disclosure, a suitable recombinant C1-INH Fc fusion protein has an amino acid sequence at least 50%, 55%, 60%, 65%, 70%, 75%, 80%, 85%, 90%, 91%, 92%, 93%, 94%, 95%, 96%, 97%, 98%, 99% or more homologous or identical to SEQ ID NO:11, SEQ ID NO:12, SEQ ID NO:13, SEQ ID NO:14, SEQ ID NO:15, SEQ ID NO:16, SEQ ID NO:32, or SEQ ID NO:33.

It is contemplated that a C1-INH-Fc fusion protein may be provided in various configurations including homodimeric or monomeric configurations. For example, a suitable homodimeric configuration may be designed to have the C-terminal end of fusion partner (e.g., a C1-INH polypeptide plus linker) attached to the N-terminal end of both Fc polypeptide strands. A suitable monomeric configuration may be designed to have the C-terminal end of fusion partner (e.g., a C1-INH polypeptide plus linker) fused to one Fc dimer.

Monomeric, also referred to herein as monovalent, forms may be preferred for certain applications and routes of administration, e.g., subcutaneous administration. A monomeric configuration may decrease steric hindrance, increase half-life, and/or may increase bioavailability.

Monovalent forms may also be preferred for C1-INH Fc fusion constructs because C1-INH is a suicide inhibitor. Since it is a suicide inhibitor, the binding of one C1-INH "arm" of a dimer Fc fusion will result in increased rate of clearance of the bound C1-INH fusion protein, even in the event that a second arm remain unbound.

An advantage of the Fc fusion proteins, both monomeric and dimeric, is that Fc expression was found to occur at higher levels than expression of C1-INH alone. Activity assays comparing the dimeric C1-INH Fc constructs with recombinant C1-INH have been shown to have similar C1q binding activity. The inclusion of a linker was also tested and found not to affect the ability of Fc-C1-INH fusion protein to bind its target.

Methods of making monomeric antibody fusion proteins include those described in, e.g., PCT Publication Nos. WO2011/063348; WO2012/020096; WO2013/138643; WO2014087299; Dumont, J. et al., Monomeric Fc Fusions: Impact on Pharmacokinetic and Biological Activity of Protein Therapeutics, Biodrugs, 20(3): 151-160 (2006); Ishino, T. et al, Protein Structure and Folding: Half-life Extension of Biotherapeutics Modality by N-Glycosylation for the Engineering a Monomeric Fc Domain, J. Biol. Chem., 288:16529-16537 (2013).

Monovalent C1-inhibitor can be made by using a plasmid containing the Fc-C1 co transfected with a plasmid expressing Fc alone. In addition, it could be made by using a dual promoter plasmid with one promoter generating Fc-C1 and the other promoter generating Fc alone. Monovalent Fc could also be made using bispecific technology where specific amino acids in the hinge region of the Fc are mutated to impart stability of the Fc region (e.g. Knob and hole technology or other stabilizing mutations which drive formation of the monovalent C1).

As used herein, "percent (%) amino acid sequence identity" with respect to a reference protein sequence (e.g., a reference C1-INH protein sequence) identified herein is defined as the percentage of amino acid residues in a candidate sequence that are identical with the amino acid residues in the reference sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, ALIGN or Megalign (DNASTAR) software. Those skilled in the art can determine appropriate parameters for measuring alignment, including any algorithms needed to achieve maximal alignment over the full length of the sequences being compared. Preferably, the WU-BLAST-2 software is used to determine amino acid sequence identity (Altschul et al., Methods in Enzymology 266, 460-480 (1996); http://blast.wustl/edu/blast/README.html). WU-BLAST-2 uses several search parameters, most of which are set to the default values. The adjustable parameters are set with the following values: overlap span=1, overlap fraction=0.125, world threshold (T)=11. HSP score (S) and HSP S2 parameters are dynamic values and are established by the program itself, depending upon the composition of the particular sequence, however, the minimum values may be adjusted and are set as indicated above.

### Albumin Domains

Albumin is a soluble, monomeric protein which comprises about one-half of the blood serum protein. Albumin functions primarily as a carrier protein for steroids, fatty acids, and thyroid hormones and plays a role in stabilizing extracellular fluid volume. Alubumin has a globular unglycosylated serum protein of molecular weight 66,500. Albumin is synthesized in the liver as preproalbumin which has an N-terminal peptide that is removed before the nascent protein is released from the rough endoplasmic reticulum. The product, proalbumin, is in turn cleaved in the Golgi vesicles to produce the secreted albumin.

Albumin is made up of three homologous domains (I-III), and each of these is comprised of two subdomains (A and B). The principal regions of ligand binding to human serum albumin are located in cavities in subdomains IIA and IIIA, which are formed mostly of hydrophobic and positively charged residues and exhibit similar chemistry. Human serum albumin has 585 amino acids and a molecular mass of 66,500 Da. The amino acids include 35 cysteines, all but one of which are involved in the formation of 17 stabilizing disulfide bonds.

Albumin has a prolonged serum half-life of 19 days. FcRn controls the long serum half-life of albumin. FcRn is a dual binding receptor that, in addition to albumin, binds IgG, and protects both proteins from intracellular degradation. The C-terminal domain of the albumin molecule has been shown to be crucial for binding to FcRn. Lack of domain IIIB or mutations of 464His, 510His, and 535His almost completely abolish FcRn binding.

Albumin fusion proteins of the invention are monomeric. In some embodiments, this feature may be an advantage over the dimeric Fc fusion embodiments for the reasons described above with regard to monomeric Fc fusion embodiments.

In some embodiments of the present disclosure, an albumin polypeptide suitable for the present disclosure includes an amino acid sequence at least 50% (e.g., at least 55%, 60%, 65%, 70%, 75%, 80%, 85%, 90%, 95%, 96%, 97%, 98%, 99%, or 100%) identical to the wild-type human serum albumin:












 (SEQ ID NO:17).

In some embodiments of the present disclosure, an albumin polypeptide suitable for the present disclosure includes an amino acid sequence at least 50% (e.g., at least 55%, 60%, 65%, 70%, 75%, 80%, 85%, 90%, 95%, 96%, 97%, 98%, 99%, or 100%) identical to the D3 domain of wild-type human serum albumin:

METPAQLLFLLLLWLPDTTGVEEPQNLIKQNCELFEQLGEYKFQNALLVRYTKKVPQVS
TPTLVEVSRNLGKVGSKCCKHPEAKRMPCAEDYLSVVLNQLCVLHEKTPVSDRVTKCC
TESLVNRRPCFSALEVDETYVPKEFNAETFTFHADICTLSEKERQIKKQTALVELVKHKP
KATKEQLKAVMDDFAAFVEKCCKADDKETCFAEEGKKLVAASRAALGL (SEQ ID
NO:20).

### Linker or Spacer

A C1-INH domain according to the present disclosure may be directly or indirectly linked to an Fc domain or an albumin domain. In some embodiments of the present disclosure, a suitable recombinant C1-INH fusion protein contains a linker or spacer that joins a C1-INH polypeptide and an Fc domain. In some embodiments of the present disclosure, a suitable recombinant C1-INH fusion protein contains a linker or spacer that joins a C1-INH polypeptide and an albumin polypeptide. An amino acid linker or spacer is generally designed to be flexible or to interpose a structure, such as an alpha-helix, between the two protein moieties. A linker or spacer can be relatively short, or can be longer. Typically, a linker or spacer contains for example 3-100 (e.g., 5-100, 10-100, 20-100 30-100, 40-100, 50-100, 60-100, 70-100, 80-100, 90-100, 5-55, 10-50, 10-45, 10-40, 10-35, 10-30, 10-25, 10-20) amino acids in length. In some embodiments of the present disclosure, a linker or spacer is equal to or longer than 2, 3, 4, 5, 6, 7, 8, 9, 10, 15, 20, 25, 30, 35, 40, 45, 50, 55, 60, 65, 70, 75, 80, 85, 90, 95, or 100 amino acids in length. Typically, a longer linker may decrease steric hindrance. In some embodiments of the present disclosure, a linker will comprise a mixture of glycine and serine residues. In some embodiments of the present disclosure, the linker may additionally comprise threonine, proline, and/or alanine residues. Thus, in some embodiments of the present disclosure, the linker comprises between 10-100, 10-90, 10-80, 10-70, 10-60, 10-50, 10-40, 10-30, 10-20, 10-15 amino acids. In some embodiments of the present disclosure, the linker comprises at least 10, 15, 20, 25, 30, 35, 40, 45, 50, 55, 60, 65, 70, 75, 80, 85, 90, or 95 amino acids. In some embodiments of the present disclosure, the linker is not a linker consisting of ALEVLFQGP.

As non-limiting examples, linkers or spacers suitable for the present disclosure include but are not limited to GGG linker and GGGGSGGGGS ((GGGGS)2 linker SEQ ID NO:27). In some embodiments, the linker comprises the sequence GGG and/or the sequence of SEQ ID NO:27.

Other suitable linkers include GAPGGGGGAAAAAGGGGGGAP (GAG linker, SEQ ID NO:34); GAPGGGGGAAAAAGGGGGGAPGGGGGAAAAAGGGGGGAP (GAG2 linker, SEQ ID NO:35); and

GAPGGGGGAAAAAGGGGGGAPGGGGGAAAAAGGGGGGAPGGGGGAAAAAGGGGG
GAP (GAG3 linker, SEQ ID NO:36).

Suitable linkers or spacers also include those having an amino acid sequence at least 50%, 55%, 60%, 65%, 70%, 75%, 80%, 85%, 90%, 91%, 92%, 93%, 94%, 95%, 96%, 97%, 98%, 99% or more homologous or identical to the above exemplary linkers, e.g., GGG linker, GGGGSGGGGS ((GGGGS)2 linker SEQ ID NO:27), GAG linker (SEQ ID NO:34), GAG2 linker (SEQ ID NO:35), or GAG3 linker (SEQ ID NO:36). Additional linkers suitable for use with some embodiments of the present disclosure may be found in US2012/0232021, filed on March 2, 2012.

In some embodiments of the present disclosure, a linker is provided that associates the C1-INH polypeptide with the albumin domain without substantially affecting the ability of the C1-INH polypeptide to bind to any of its cognate ligands (e.g., C1s, etc.). In some embodiments of the present disclosure, a linker is provided such that the binding of a C1-INH peptide to one or more of its cognate ligands is not altered as compared to the C1-INH polypeptide alone. In some embodiments of the present disclosure, a linker is provided such that the binding of a C1-INH peptide to one or more of its cognate ligands is not reduced or decreased as compared to the C1-INH polypeptide alone.

### Production of Recombinant C1-INH Fusion Proteins

A recombinant C1-INH fusion protein suitable for the present invention may be produced by any available means. For example, a recombinant C1-INH fusion protein may be recombinantly produced by utilizing a host cell system engineered to express a recombinant C1-INH fusion protein-encoding nucleic acid. Alternatively or additionally, a recombinant C1-INH fusion protein may be produced by activating endogenous genes. Alternatively or additionally, a recombinant C1-INH fusion protein may be partially or fully prepared by chemical synthesis.

Where proteins are recombinantly produced, any expression system can be used. To give but a few examples, known expression systems include, for example, *E.coli*, egg, baculovirus, plant, yeast, or mammalian cells.

In some embodiments, recombinant C1-INH fusion proteins suitable for the present invention are produced in mammalian cells. Non-limiting examples of mammalian cells that may be used in accordance with the present invention include BALB/c mouse myeloma line (NSO/1, ECACC No: 85110503); human retinoblasts (PER.C6, CruCell, Leiden, The Netherlands); monkey kidney CV1 line transformed by SV40 (COS-7, ATCC CRL 1651); human embryonic kidney line (HEK293 or 293 cells subcloned for growth in suspension culture, Graham et al., J. Gen Virol., 36:59,1977); human fibrosarcoma cell line (e.g., HT1080); baby hamster kidney cells (BHK21, ATCC CCL 10); Chinese hamster ovary cells +/-DHFR (CHO, Urlaub and Chasin, Proc. Natl. Acad. Sci. USA, 77:4216, 1980); mouse sertoli cells (TM4, Mather, Biol. Reprod., 23:243-251, 1980); monkey kidney cells (CV1 ATCC CCL 70); African green monkey kidney cells (VERO-76, ATCC CRL-1 587); human cervical carcinoma cells (HeLa, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci., 383:44-68, 1982); MRC 5 cells; FS4 cells; and a human hepatoma line (Hep G2).

In some embodiments, the present invention provides recombinant C1-INH fusion proteins produced from human cells. In some embodiments, the present invention provides recombinant C1-INH fusion proteins produced from CHO cells or HT1080 cells.

Typically, cells that are engineered to express a recombinant C1-INH fusion protein may comprise a transgene that encodes a recombinant C1-INH fusion protein described herein. It should be appreciated that the nucleic acids encoding recombinant C1-INH fusion protein may contain regulatory sequences, gene control sequences, promoters, non-coding sequences and/or other appropriate sequences for expressing the recombinant C1-INH fusion protein. Typically, the coding region is operably linked with one or more of these nucleic acid components.

The coding region of a transgene may include one or more silent mutations to optimize codon usage for a particular cell type. For example, the codons of a C1-INH fusion transgene may be optimized for expression in a vertebrate cell. In some embodiments, the codons of a C1-INH fusion transgene may be optimized for expression in a mammalian cell. In some embodiments, the codons of a C1-INH fusion transgene may be optimized for expression in a human cell. In some embodiments, the codons of a C1-INH fusion transgene may be optimized for expression in a CHO cell.

### Nucleic Acids Encoding C1-Inhibitor Fc Fusion Proteins and/or C1-Inhibitor Albumin Fusion Proteins

In some embodiments, nucleic acid molecules are provided comprising nucleic acid sequences encoding for a recombinant gene of interest (herein referred to as a transgene) which encodes for a C1-inhibitor Fc fusion protein having an amino acid sequence selected from: SEQ ID NO:11, SEQ ID NO:13, SEQ ID NO:14, SEQ ID NO:15, or SEQ ID NO:16.. In some embodiments of the present disclosure, the nucleic acid encoding a transgene may be modified to provide increased expression of the encoded C1-inhibitor Fc fusion protein and/or C1-inhibitor albumin fusion protein, which is also referred to as codon optimization. For example, the nucleic acid encoding a transgene can be modified by altering the open reading frame for the coding sequence. As used herein, the term "open reading frame" is synonymous with "ORF" and means any nucleotide sequence that is potentially able to encode a protein, or a portion of a protein. An open reading frame usually begins with a start codon (represented as, e.g. AUG for an RNA molecule and ATG in a DNA molecule in the standard code) and is read in codon-triplets until the frame ends with a STOP codon (represented as, e.g. UAA, UGA or UAG for an RNA molecule and TAA, TGA or TAG in a DNA molecule in the standard code). As used herein, the term "codon" means a sequence of three nucleotides in a nucleic acid molecule that specifies a particular amino acid during protein synthesis; also called a triplet or codon-triplet. For example, of the 64 possible codons in the standard genetic code, two codons, GAA and GAG encode the amino acid Glutamine whereas the codons AAA and AAG specify the amino acid Lysine. In the standard genetic code three codons are stop codons, which do not specify an amino acid. As used herein, the term "synonymous codon" means any and all of the codons that code for a single amino acid. Except for Methionine and Tryptophan, amino acids are coded by two to six synonymous codons. For example, in the standard genetic code the four synonymous codons that code for the amino acid Alanine are GCA, GCC, GCG and GCU, the two synonymous codons that specify Glutamine are GAA and GAG and the two synonymous codons that encode Lysine are AAA and AAG.

In some embodiments of the present disclosure, a nucleic acid encoding the open reading frame of a C1-inhibitor Fc fusion protein and/or C1-inhibitor albumin fusion protein may be modified using standard codon optimization methods. Various commercial algorithms for codon optimization are available and can be used to practice the present invention. Typically, codon optimization does not alter the encoded amino acid sequences. In some embodiments of the present disclosure, codon optimization may lead to amino acids alteration such as substitution, deletion or insertion. Typically, such amino acid alteration does not substantially alter the protein activity.
Exemplary nucleic acid sequences according to the present disclosure encode for C1-inhibitor Fc fusion proteins and C1-inhibitor albumin fusion proteins having an amino acid sequence at least 50%, 55%, 60%, 65%, 70%, 75%, 80%, 85%, 90%, 91%, 92%, 93%, 94%, 95%, 96%, 97%, 98%, 99% or more identical or homologous to SEQ ID NO:11, SEQ ID NO:12, SEQ ID NO:13, SEQ ID NO:14, SEQ ID NO:15, SEQ ID NO:16, SEQ ID NO:18, SEQ ID NO:19, SEQ ID NO:21, SEQ ID NO:22, SEQ ID NO:23, SEQ ID NO:24, SEQ ID NO:25, SEQ ID NO:26, SEQ ID NO:28, SEQ ID NO:29, SEQ ID NO:30, SEQ ID NO:31, SEQ ID NO:32, or SEQ ID NO:33.

In some embodiments of the present disclosure, a nucleotide change may alter a synonymous codon within the open reading frame in order to agree with the endogenous codon usage found in a particular heterologous cell selected to express C1-inhibitor Fc fusion protein and/or C1-inhibitor albumin fusion protein. Alternatively or additionally, a nucleotide change may alter the G+C content within the open reading frame to better match the average G+C content of open reading frames found in endogenous nucleic acid sequence present in the heterologous host cell. A nucleotide change may also alter a polymononucleotide region or an internal regulatory or structural site found within a C1-inhibitor Fc fusion protein or C1-inhibitor albumin fusion sequence. Thus, a variety of modified or optimized nucleotide sequences are envisioned according to the present disclosure including, without limitation, nucleic acid sequences providing increased expression of C1-inhibitor Fc fusion proteins and/or C1-inhibitor albumin fusion proteins in a prokaryotic cell; yeast cell; insect cell; and in a mammalian cell.

Typically, a modified nucleic acid according to the present disclosure encodes a C1-inhibitor Fc fusion protein and/or C1-inhibitor albumin fusion protein with or without amino acid sequence alteration. In the event there is amino acid alteration, such alteration typically does not substantially decrease the C1-inhibitor Fc fusion protein or C1-inhibitor albumin fusion protein activity. In some embodiments of the present disclosure, such alteration increases and/or enhances the C1-inhibitor Fc fusion protein or C1-inhibitor albumin fusion protein activity. Activity may refer to the following non-limiting list of parameters: increased half-life, increased/elevated protein expression by host cells, increased stability of expressed protein, increased solubility of expressed protein, decreased aggregation of expressed protein, simpler formulation of expressed protein, simpler purification of expressed protein, increased tolerance of expressed protein to changes in pH, increased ability of protein to tolerate high and low pH conditions, expressed protein that is suitable for formulating at high concentrations.

### Expression Vectors

A nucleic acid sequence encoding a C1-inhibitor Fc fusion protein and/or C1-inhibitor albumin fusion protein as disclosed in the present application, can be molecularly cloned (inserted) into a suitable vector for propagation or expression in a host cell. A wide variety of expression vectors can be used to practice the present disclosure, including, without limitation, a prokaryotic expression vector; a yeast expression vector; an insect expression vector and a mammalian expression vector. Exemplary vectors suitable for the present disclosure include, but are not limited to, viral based vectors (e.g., AAV based vectors, retrovirus based vectors, plasmid based vectors). In some embodiments of the present disclosure, a nucleic acid sequence encoding a C1-inhibitor Fc fusion protein can be inserted into a suitable vector. In some embodiments of the present disclosure, a nucleic acid sequence encoding a C1-inhibitor albumin fusion protein can be inserted into a suitable vector. Typically, a nucleic acid encoding a C1-inhibitor Fc fusion protein or C1-inhibitor albumin fusion protein is operably linked to various regulatory sequences or elements.

### Regulatory Sequences or Elements

Various regulatory sequences or elements may be incorporated in an expression vector suitable for the present disclosure. Exemplary regulatory sequences or elements include, but are not limited to, promoters, enhancers, repressors or suppressors, 5' untranslated (or non-coding) sequences, introns, 3' untranslated (or non-coding) sequences.

As used herein, a "Promoter" or "Promoter sequence" is a DNA regulatory region capable of binding an RNA polymerase in a cell (e.g., directly or through other promoter bound proteins or substances) and initiating transcription of a coding sequence. A promoter sequence is, in general, bound at its 3' terminus by the transcription initiation site and extends upstream (5' direction) to include the minimum number of bases or elements necessary to initiate transcription at any level. The promoter may be operably associated with or operably linked to the expression control sequences, including enhancer and repressor sequences or with a nucleic acid to be expressed. In some embodiments of the present disclosure, the promoter may be inducible. In some embodiments of the present disclosure, the inducible promoter may be unidirectional or bio-directional. In some embodiments of the present disclosure, the promoter may be a constitutive promoter. In some embodiments of the present disclosure, the promoter can be a hybrid promoter, in which the sequence containing the transcriptional regulatory region is obtained from one source and the sequence containing the transcription initiation region is obtained from a second source. Systems for linking control elements to coding sequence within a transgene are well known in the art (general molecular biological and recombinant DNA techniques are described in Sambrook, Fritsch, and Maniatis, Molecular Cloning: A Laboratory Manual, Second Edition, Cold Spring Harbor Laboratory Press, Cold Spring Harbor, NY, 1989). Commercial vectors suitable for inserting a transgene for expression in various host cells under a variety of growth and induction conditions are also well known in the art.

In some embodiments of the present disclosure, a specific promoter may be used to control expression of the transgene in a mammalian host cell such as, but are not limited to, SRα-promoter (Takebe et al., Molec. and Cell. Bio. 8:466-472 (1988)), the human CMV immediate early promoter (Boshart et al., Cell 41:521-530 (1985); Foecking et al., Gene 45:101-105 (1986)), human CMV promoter, the human CMV5 promoter, the murine CMV immediate early promoter, the EF1-α-promoter, a hybrid CMV promoter for liver specific expression (e.g., made by conjugating CMV immediate early promoter with the transcriptional promoter elements of either human α-1-antitrypsin (HAT) or albumin (HAL) promoter), or promoters for hepatoma specific expression (e.g., wherein the transcriptional promoter elements of either human albumin (HAL; about 1000 bp) or human α-1-antitrypsin (HAT, about 2000 bp) are combined with a 145 long enhancer element of human α-1-microglobulin and bikunin precursor gene (AMBP); HAL-AMBP and HAT-AMBP); the SV40 early promoter region (Benoist at al., Nature 290:304-310 (1981)), the Orgyia pseudotsugata immediate early promoter, the herpes thymidine kinase promoter (Wagner at al., Proc. Natl. Acad. Sci. USA 78:1441-1445 (1981)); or the regulatory sequences of the metallothionein gene (Brinster et al., Nature 296:39-42 (1982)). In some embodiments of the present disclosure, the mammalian promoter is a is a constitutive promoter such as, but not limited to, the hypoxanthine phosphoribosyl transferase (HPTR) promoter, the adenosine deaminase promoter, the pyruvate kinase promoter, the beta-actin promoter as well as other constitutive promoters known to those of ordinary skill in the art.

In some embodiments of the present disclosure, a specific promoter may be used to control expression of a transgene in a prokaryotic host cell such as, but are not limited to, the β-lactamase promoter (Villa-Komaroff et al., Proc. Natl. Acad. Sci. USA 75:3727-3731 (1978)); the tac promoter (DeBoer et al., Proc. Natl. Acad. Sci. USA 80:21-25 (1983)); the T7 promoter, the T3 promoter, the M13 promoter or the M16 promoter; in a yeast host cell such as, but are not limited to, the GAL1, GAL4 or GAL10 promoter, the ADH (alcohol dehydrogenase) promoter, PGK (phosphoglycerol kinase) promoter, alkaline phosphatase promoter, glyceraldehyde-3-phosphate dehydrogenase III (TDH3) promoter, glyceraldehyde-3-phosphate dehydrogenase II (TDH2) promoter, glyceraldehyde-3-phosphate dehydrogenase I (TDH1) promoter, pyruvate kinase (PYK), enolase (ENO), or triose phosphate isomerase (TPI).

In some embodiments of the present disclosure, the promoter may be a viral promoter, many of which are able to regulate expression of a transgene in several host cell types, including mammalian cells. Viral promoters that have been shown to drive constitutive expression of coding sequences in eukaryotic cells include, for example, simian virus promoters, herpes simplex virus promoters, papilloma virus promoters, adenovirus promoters, human immunodeficiency virus (HIV) promoters, Rous sarcoma virus promoters, cytomegalovirus (CMV) promoters, the long terminal repeats (LTRs) of Moloney murine leukemia virus and other retroviruses, the thymidine kinase promoter of herpes simplex virus as well as other viral promoters known to those of ordinary skill in the art.

In some embodiments of the present disclosure, the gene control elements of an expression vector may also include 5' non-transcribing and 5' non-translating sequences involved with the initiation of transcription and translation, respectively, such as a TATA box, capping sequence, CAAT sequence, Kozak sequence and the like. Enhancer elements can optionally be used to increase expression levels of a polypeptide or protein to be expressed. Examples of enhancer elements that have been shown to function in mammalian cells include the SV40 early gene enhancer, as described in Dijkema et al., EMBO J. (1985) 4: 761 and the enhancer/promoter derived from the long terminal repeat (LTR) of the Rous Sarcoma Virus (RSV), as described in Gorman et al., Proc. Natl. Acad. Sci. USA (1982b) 79:6777 and human cytomegalovirus, as described in Boshart et al., Cell (1985) 41:521. Genetic control elements of an expression vector will also include 3' non-transcribing and 3' non-translating sequences involved with the termination of transcription and translation. Respectively, such as a poly polyadenylation (polyA) signal for stabilization and processing of the 3' end of an mRNA transcribed from the promoter. Poly A signals included, for example, the rabbit beta globin polyA signal, bovine growth hormone polyA signal, chicken beta globin terminator/polyA signal, or SV40 late polyA region.

### Selectable Markers

Expression vectors will preferably but optionally include at least one selectable marker. In some embodiments of the present disclosure, the selectable maker is a nucleic acid sequence encoding a resistance gene operably linked to one or more genetic regulatory elements, to bestow upon the host cell the ability to maintain viability when grown in the presence of a cytotoxic chemical and/or drug. In some embodiments of the present disclosure, a selectable agent may be used to maintain retention of the expression vector within the host cell. In some embodiments, the selectable agent is may be used to prevent modification (i.e. methylation) and/or silencing of the transgene sequence within the expression vector. In some embodiments of the present disclosure, a selectable agent is used to maintain episomal expression of the vector within the host cell. In some embodiments of the present disclosure, the selectable agent is used to promote stable integration of the transgene sequence into the host cell genome. In some embodiments of the present disclosure, an agent and/or resistance gene may include, but is not limited to, methotrexate (MTX), dihydrofolate reductase (DHFR, U.S. Pat. Nos. 4,399,216; 4,634,665; 4,656,134; 4,956,288; 5,149,636; 5,179,017, ampicillin, neomycin (G418), zeomycin, mycophenolic acid, or glutamine synthetase (GS, U.S. Pat. Nos. 5,122,464; 5,770,359; 5,827,739) for eukaryotic host cell; tetracycline, ampicillin, kanamycin or chlorampenichol for a prokaryotic host cell; and URA3, LEU2, HIS3, LYS2, HIS4, ADE8, CUP1 or TRP1 for a yeast host cell.

Expression vectors may be transfected, transformed or transduced into a host cell. As used herein, the terms "transfection," "transformation" and "transduction" all refer to the introduction of an exogenous nucleic acid sequence into a host cell. In some embodiments of the present disclosure, expression vectors containing nucleic acid sequences encoding for a C1-inhibitor Fc fusion protein and/or a C1-inhibitor albumin fusion protein are transfected, transformed or transduced into a host cell at the same time. In some embodiments of the present disclosure, expression vectors containing nucleic acid sequences encoding for a C1-inhibitor Fc fusion protein and/or a C1-inhibitor albumin fusion protein are transfected, transformed or transduced into a host cell sequentially. For example, a vector encoding an C1-inhibitor fusion protein may be transfected, transformed or transduced into a host cell first, followed by the transfection, transformation or transduction of a vector encoding one or more proteins which enhance glycosylation, preferably by increased sialylation, of the expressed C1-inhibitor fusion protein, and vice versa.

Examples of transformation, transfection and transduction methods, which are well known in the art, include liposome delivery, i.e., lipofectamine™ (Gibco BRL) Method of Hawley-Nelson, Focus 15:73 (1193), electroporation, CaPO₄ delivery method of Graham and van der Erb, Virology, 52:456-457 (1978), DEAE-Dextran medicated delivery, microinjection, biolistic particle delivery, polybrene mediated delivery, cationic mediated lipid delivery, transduction, and viral infection, such as, e.g., retrovirus, lentivirus, adenovirus adeno-associated virus and Baculovirus (Insect cells). General aspects of cell host transformations have been described in the art, such as by Axel in U.S. Pat. No. 4,399,216; Sambrook, supra, Chapters 1-4 and 16-18; Ausubel, supra, chapters 1, 9, 13, 15, and 16. For various techniques for transforming mammalian cells, see Keown et al., Methods in Enzymology (1989), Keown et al., Methods in Enzymology, 185:527-537 (1990), and Mansour et al., Nature, 336:348-352 (1988).

Once introduced inside cells, expression vectors may be integrated stably in the genome or exist as extra-chromosomal constructs. Vectors may also be amplified and multiple copies may exist or be integrated in the genome. In some embodiments of the present disclosure, cells of the invention may contain 1, 2, 3, 4, 5, 6, 7, 8, 9, 10, 15, 20 or more copies of nucleic acids encoding a C1-inhibitor fusion protein. In some embodiments of the present disclosure, cells of the invention may contain 1, 2, 3, 4, 5, 6, 7, 8, 9, 10, 15, 20 or more copies of nucleic acids encoding a C1-inhibitor fusion protein. In some embodiments of the present disclosure, cells of the invention may contain multiple copies (e.g., 2, 3, 4, 5, 6, 7, 8, 9, 10, 15, 20 or more) of nucleic acids encoding both C1-inhibitor Fc fusion protein and one or more proteins which enhance glycosylation, preferably by increased sialylation, of the expressed C1-inhibitor fusion protein. Sialylation can also be manipulated through the various methods described herein. Without wishing to be bound by any theory, decreased sialylation was found to be associated with increased heparin binding of the disclosed constructs, thereby preventing the C1-INH binding site from binding to targets. Heparin binding also increases likelihood of internalization into the lysosome, thereby increasing the rate of clearance.

### Host Cells

As used herein, the term "host cells" refers to cells that can be used to produce recombinant C1-inhibitor fusion protein. In particular, host cells are suitable for producing recombinant C1-inhibitor fusion protein at a large scale. Suitable host cells can be derived from a variety of organisms, including, but not limited to, mammals, plants, birds (e.g., avian systems), insects, yeast, and bacteria. In some embodiments, host cells are mammalian cells. In some embodiments, a suitable host cell is engineered to improve the glycosylation profile of the expressed recombinant C1-inhibitor fusion protein. In some embodiments of the present disclosure, the C1-INH polypeptide has the same or similar glycosylation profile to the analogous portions of native plasma-derived C1-INH. In some embodiments of the present disclosure, the C1-INH polypeptide has at least 50%, 55%, 60%, 65%, 70%, 75%, 80%, 85%, 90%, 91%, 92%, 93%, 94%, 95%, 96%, 97%, 98%, 99% glycans equivalent to native plasma-derived C1-INH. In some embodiments of the present disclosure, the glycosylation profile of the C1-INH fusion protein has a humanized glycosylation profile. In some embodiments of the present disclosure, the C1-INH fusion protein has increased sialylation compared to native plasma-derived C1-INH.

Improvement of the glycosylation profile may refer to increasing sialylation and/or humanizing the glycosylation profile, e.g., expressing in an engineered cell a recombinant C1-inhibitor fusion protein comprising a C1-INH polypeptide, thereby producing a glycosylation profile more similar to native human C1-INH than a C1-INH polypeptide expressed in a non-engineered cell. Improvement may also refer to increasing, enhancing, and/or optimizing the glycosylation profile of the C1-INH fusion compared to Ruconest.

Various methods of changing, controlling, manipulating, improving, enhancing, and/or optimizing the glycosylation profile of proteins are known in the art. The glycosylation profile characterisitcs that may be optimized include the number of glycan residues, location of glycan residue attachment, manner of glycan attachment (e.g., type of bond), process of glycan attachment, and identity of glycan residues attached to the protein or polypeptide. The glycosylation profile of any portion of the disclosed fusion proteins are contemplated as suitable targets for glycosylation optimization. Portions of the disclosed fusion proteins that may be optimized for glycosylation, include but are not limited to, the C1-INH polypeptide, the Fc domain, albumin polypeptides, and/or linkers disclosed herein. These methods of controlling the glycosylation profile, as well as others yet to be discovered that a person of skill would understand as having utility in view of the instant disclosure in optimizing glycosylation of the disclosed fusion proteins, are contemplated. Methods of changing, controlling, manipulating, improving, enhancing, and/or optimizing the glycosylation profile of the disclosed C1-INH proteins and polypeptides include *in vitro, in situ,* and *in vivo* methods.

In some embodiments of the present disclosure the glycosylation profile of expressed proteins or polypeptides is altered through post-translational and/or chemical modification of the expressed protein or polypeptide.

In some embodiments of the present disclosure, the C1-INH fusion protein that has undergone post-translational and/or chemical modification has a glycosylation profile that is changed, improved, enhanced, humanized, and/or optimized compared to a C1-INH fusion protein having the same sequence that has not undergone said post-translational and/or chemical modification. In some embodiments of the present disclosure, the C1-INH fusion protein that has undergone post-translational and/or chemical modification has a sialylation profile that is changed, improved, enhanced, humanized, and/or optimized compared to a C1-INH fusion protein having the same sequence that has not undergone said post-translational and/or chemical modification.

In some embodiments of the present disclosure, the C1-INH fusion protein is expressed from a cell line engineered to enhance glycosylation has a glycosylation profile that is changed, improved, enhanced, humanized, and/or optimized compared to a C1-INH fusion protein having the same sequence that is expressed from the same cell line that has not been engineered to enhance glycosylation. In some embodiments of the present disclosure, the C1-INH fusion protein is expressed from a cell line engineered to enhance sialylation has a sialylation profile that is changed, improved, enhanced, humanized, and/or optimized compared to a C1-INH fusion protein having the same sequence that is expressed from the same cell line that has not been engineered to enhance sialylation.

In some embodiments of the present disclosure, the C1-INH fusion protein is expressed from a cell line a cell line cultured under conditions to enhance glycosylation has a glycosylation profile that is changed, improved, enhanced, humanized, and/or optimized compared to a C1-INH fusion protein having the same sequence that is expressed from a cell line cultured under conditions to enhance glycosylation. In some embodiments of the present disclosure, the C1-INH fusion protein is expressed from a cell line a cell line cultured under conditions to enhance sialylation has a sialylation profile that is changed, improved, enhanced, humanized, and/or optimized compared to a C1-INH fusion protein having the same sequence that is expressed from a cell line cultured under conditions to enhance sialylation.

In some embodiments of the present disclosure the C1-INH fusion protein has a glycosylation profile that is changed, improved, enhanced, humanized, and/or optimized compared to Ruconest. In some embodiments of the present disclosure the C1-INH fusion protein has a glycosylation profile that is changed, improved, enhanced, humanized, and/or optimized compared to human plasma-derived C1-INH.

In some embodiments of the present disclosure the cell culture conditions are manipulated to achieve expression of proteins having a desired glycosylation profile. These cell culture conditions include control of the production and culture process including length of culture, additives to culture medium, co-expression of genes to enhance glycosylation via increased glycosylation, and/or engineering of cells to knock out, prevent expression of, inactivate, or disrupt enzymes associated with glycan degradation (e.g., sialyladase). Suitable methods for manipulation of glycosylation include, but are not limited to, those described in, e.g., U.S. Patent Nos. 5,047,335; 5,096,816; 5,705,364; 7,645,609; 8,273,723 ; 8,524,477; 8,617,878; 8,871,723; PCT Publication Nos. WO2006/106348; WO2007/095506; WO2008/025856; WO2010/007214; WO2010/099394; and WO2013/093760.

Selection of host cells and specific clones of transfected host cells may also be used to enhance glycosylation. Other methods of enhancing glycosylation according to the present disclosure include development of purification processes to enrich for proteins or polypeptides having the desired glycosylation profile.

Various methods of manipulating the sialylation profile of proteins are known in the art. These methods as well as others yet to be discovered are contemplated by the instant disclosure. Methods of manipulating the sialylation profile of C1-INH proteins and polypeptides of the disclosure include in vitro, in situ, and in vivo methods. In some embodiments the sialylation profile of expressed proteins or polypeptides is altered through post-expression chemical modification of the expressed protein or polypeptide. In some embodiments of the present disclosure the cell culture conditions are manipulated to achieve expression of proteins having a desired sialylation profile. These cell culture conditions include control of the production and culture process including length of culture, additives to culture medium, and/or co-expression of genes to enhance sialylation. Selection of host cells and specific clones of transfected host cells may also be used to enhance sialylation. Other methods of enhancing sialylation according to the present disclosure include development of purification processes to enrich for proteins or polypeptides having the desired sialylation profile.

Sialylation can also be manipulated through the various methods described herein. Without wishing to be bound by any theory, decreased sialylation was found to be associated with increased heparin binding of the disclosed constructs, thereby preventing the C1-INH binding site from binding to targets. Heparin binding also increases likelihood of internalization into the lysosome, thereby increasing the rate of clearance.

### Mammalian Cell Lines

Any mammalian cell or cell type susceptible to cell culture, and to expression of polypeptides, may be utilized in accordance with the present invention as a host cell. Non-limiting examples of mammalian cells that may be used in accordance with the present invention include human embryonic kidney 293 cells (HEK293), HeLa cells; BALB/c mouse myeloma line (NSO/1, ECACC No: 85110503); human retinoblasts (PER.C6 (CruCell, Leiden, The Netherlands)); monkey kidney CV1 line transformed by SV40 (COS-7, ATCC CRL 1651); human embryonic kidney line (293 or 293 cells subcloned for growth in suspension culture, Graham et al., J. Gen Virol., 36:59 (1977)); baby hamster kidney cells (BHK, ATCC CCL 10); Chinese hamster ovary cells +/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); monkey kidney cells (CV1 ATCC CCL 70); African green monkey kidney cells (VERO-76, ATCC CRL-1 587); human cervical carcinoma cells (HeLa, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci., 383:44-68 (1982)); MRC 5 cells; FS4 cells; and a human hepatoma line (Hep G2). In some embodiments of the present disclosure, a suitable mammalian cell is not an endosomal acidification-deficient cell.

Additionally, any number of commercially and non-commercially available hybridoma cell lines that express polypeptides or proteins may be utilized in accordance with the present invention. One skilled in the art will appreciate that hybridoma cell lines might have different nutrition requirements and/or might require different culture conditions for optimal growth and polypeptide or protein expression, and will be able to modify conditions as needed.

### Non-Mammalian Cell Lines

Any non-mammalian derived cell or cell type susceptible to cell culture, and to expression of polypeptides, may be utilized in accordance with the present invention as a host cell. Non-limiting examples of non-mammalian host cells and cell lines that may be used in accordance with the present invention include cells and cell lines derived from *Pichia pastoris, Pichia methanolica, Pichia angusta, Schizosacccharomyces pombe, Saccharomyces cerevisiae,* and *Yarrowia lipolytica* for yeast; *Sodoptera frugiperda, Trichoplusis ni, Drosophila melangoster* and *Manduca sexta* for insects; and *Escherichia coli, Salmonella typhimurium, Bacillus subtilis, Bacillus lichenifonnis, Bacteroides fragilis, Clostridia perfringens, Clostridia difficile* for bacteria; and *Xenopus Laevis* from amphibian.

### Adaptable to Adherent vs Suspension Growth

In certain embodiments, a host cell is selected for generating a cell line based on certain preferable attributes or growth under particular conditions chosen for culturing cells. It will be appreciated by one skilled in the art, such attributes may be ascertained based on known characteristic and/or traits of an established line (i.e. a characterized commercially available cell line) or though empirical evaluation. In some embodiments, a cell line may be selected for its ability to grow on a feeder layer of cells. In some embodiments, a cell line may be selected for its ability to grow in suspension. In some embodiments, a cell line may be selected for its ability to grow as an adherent monolayer of cells. In some embodiments, such cells can be used with any tissue culture vessel or any vessel treated with a suitable adhesion substrate. In some embodiments, a suitable adhesion substrate is selected from the group consisting of collagen (e.g. collagen I, II, II, or IV), gelatin, fibronectin, laminin, vitronectin, fibrinogen, BD Matrigel™, basement membrane matrix, dermatan sulfate proteoglycan, Poly-D-Lysine and/or combinations thereof. In some embodiments, an adherent host cell may be selected and modified under specific growth conditions to grow in suspension. Such methods of modifying an adherent cell to grown in suspension are known in the art. For example, a cell may be conditioned to grow in suspension culture, by gradually removing animal serum from the growth media over time.

### Cell Line Selection and Evaluation

According to the present disclosure, a cell engineered to express recombinant C1-INH fusion protein is selected for its ability to produce the recombinant C1-INH fusion protein at commercially viable scale. In particular, engineered cells according to the present disclosure are able to produce recombinant C1-INH fusion protein at a high level and/or with high enzymatic activity. In some embodiments of the present disclosure, desirable cells, once cultivated under a cell culture condition (e.g., a standard large scale suspension or adherent culture condition), can produce C1-INH fusion protein in an amount of or greater than about 5 picogram/cell/day (e.g., greater than about 10, 15, 20, 25, 30, 35, 40, 45, 50, 55, 60, 65, 70, 75, 80, 85, 90, 95, or 100 picogram/cell/day). In some embodiments of the present disclosure, desired cells, once cultivated under a cell culture condition (e.g., a standard large scale suspension or adherent culture condition), are able to produce C1-INH fusion protein in an amount ranging from about 5-100 picogram/cell/day (e.g., about 5-90 picogram/cell/day, about 5-80 picogram/cell/day, about 5-70 picogram/cell/day, about 5-60 picogram/cell/day, about 5-50 picogram/cell/day, about 5-40 picogram/cell/day, about 5-30 picogram/cell/day, about 10-90 picogram/cell/day, about 10-80 picogram/cell/day, about 10-70 picogram/cell/day, about 10-60 picogram/cell/day, about 10-50 picogram/cell/day, about 10-40 picogram/cell/day, about 10-30 picogram/cell/day, about 20-90 picogram/cell/day, about 20-80 picogram/cell/day, about 20-70 picogram/cell/day, about 20-60 picogram/cell/day, about 20-50 picogram/cell/day, about 20-40 picogram/cell/day, about 20-30 picogram/cell/day).

The half-life of C1-INH may be affected by the glycosylation profile. As discussed above, cells of the invention may be engineered to improve the glycosylation profile of the expressed C1-INH fusion protein. For example, Ruconest® (Pharming N.V.), a recombinant C1-INH polypeptide that is less sialylated and/or has a different sialylation distribution from plasma-derived human C1-INH has been shown to have a shorter half-life than plasma-derived human C1-INH. Davis, B. & Bernstein, J. A., Conestat alfa for the treatment of angioedema attacks, Ther Clin Risk Manag. 7: 265-273 (2011); Koles, K. et al., Influence of lactation parameters on the N-glycosylation of recombinant human C1 inhibitor isolated from the milk of transgenic rabbits, Glycobiology, 14(11):979-986 (2004); Koles, K. et al., N- and O-glycans of recombinant human C1 inhibitor expressed in the milk of transgenic rabbits, Glycobiology, 14(1):51-64 (2004).

### Cell Culture Medium and Condition

Various cell culture medium and conditions may be used to produce a recombinant C1-INH fusion protein using engineered cells according to the present invention. For example, a recombinant C1-INH fusion protein may be produced in serum-containing or serum-free medium. In some embodiments, a recombinant C1-INH fusion protein is produced in serum-free medium. In some embodiments, a recombinant C1-INH fusion protein is produced in an animal free medium, i.e., a medium that lacks animal-derived components. In some embodiments, a recombinant C1-INH fusion protein is produced in a chemically defined medium. As used herein, the term "chemically-defined nutrient medium" refers to a medium of which substantially all of the chemical components are known. In some embodiments, a chemically defined nutrient medium is free of animal-derived components such as serum, serum derived proteins (e.g., albumin or fetuin), and other components. In some cases, a chemically-defined medium comprises one or more proteins (*e.g*., protein growth factors or cytokines.) In some cases, a chemically-defined nutrient medium comprises one or more protein hydrolysates. In other cases, a chemically-defined nutrient medium is a protein-free media, i.e., a serum-free media that contains no proteins, hydrolysates or components of unknown composition.

In some embodiments, a chemically defined medium may be supplemented by one or more animal derived components. Such animal derived components include, but are not limited to, fetal calf serum, horse serum, goat serum, donkey serum, human serum, and serum derived proteins such as albumins (e.g., bovine serum albumin or human serum albumin).

Various cell culture conditions may be used to produce recombinant C1-INH fusion proteins at large scale including, but not limited to, roller bottle cultures, bioreactor batch cultures, perfusion cultures, and bioreactor fed-batch cultures. In some embodiments, recombinant C1-INH fusion protein is produced by cells cultured in suspension. In some embodiments, recombinant C1-INH fusion protein is produced by adherent cells. In some embodiments, perfusion culture is used to control glycosylation of expressed protein. Exemplary methods of perfusion culture include, but are not limited to, those described in, e.g., U.S. Patent Nos. 6,528,286 and PCT Publication No. WO1996/039488A1.

Exemplary cell media and culture conditions are described in the Examples sections. The examples are not intended to be limiting.

### Purification of Expressed C1-INH Fusion Protein

Various methods may be used to purify or isolate C1-INH fusion protein produced according to various methods described herein. In some embodiments, the expressed C1-INH fusion protein is secreted into the medium and thus cells and other solids may be removed, as by centrifugation or filtering for example, as a first step in the purification process. Alternatively or additionally, the expressed C1-INH fusion protein is bound to the surface of the host cell. In this embodiment, the host cells expressing the polypeptide or protein are lysed for purification. Lysis of mammalian host cells can be achieved by any number of means well known to those of ordinary skill in the art, including physical disruption by glass beads and exposure to high pH conditions.

The C1-INH fusion protein may be isolated and purified by standard methods including, but not limited to, chromatography (e.g., ion exchange, affinity, size exclusion, and hydroxyapatite chromatography), gel filtration, centrifugation, or differential solubility, ethanol precipitation or by any other available technique for the purification of proteins (See, e.g., Scopes, Protein Purification Principles and Practice 2nd Edition, Springer-Verlag, New York, 1987; Higgins, S. J. and Hames, B. D. (eds.), Protein Expression: A Practical Approach, Oxford Univ Press, 1999; and Deutscher, M. P., Simon, M. I., Abelson, J. N. (eds.), Guide to Protein Purification: Methods in Enzymology (Methods in Enzymology Series, Vol 182), Academic Press, 1997). For immunoaffinity chromatography in particular, the protein may be isolated by binding it to an affinity column comprising antibodies that were raised against that protein and were affixed to a stationary support. Alternatively, affinity tags such as an influenza coat sequence, poly-histidine, or glutathione-S-transferase can be attached to the protein by standard recombinant techniques to allow for easy purification by passage over the appropriate affinity column. Protease inhibitors such as phenyl methyl sulfonyl fluoride (PMSF), leupeptin, pepstatin, or aprotinin may be added at any or all stages in order to reduce or eliminate degradation of the polypeptide or protein during the purification process. Protease inhibitors are particularly desired when cells must be lysed in order to isolate and purify the expressed polypeptide or protein.

Protein A HP Purification of IgG1 LALA Fc full length C1-inhibitor is shown in FIG. 3. Protein A capture of fusion proteins was successful on small scale, but aggregation was observed when scaled up. Without wishing to be bound by any theory, the low pH necessary for elution appeared to cause the aggregation and inactivate C1-INH. In some embodiments purification does not involve a low pH step. In some embodiments, the fusion proteins are mutated in order to stabilize the protein at low pH. In other embodiments, additives are used to protect the C1-INH fusion proteins from aggregation during the low pH elution step. In still other embodiments, non-protein A resins are used to purify proteins,

Exemplary purification methods that avoid the problems associated with Protein A purification of C1-INH Fc fusion proteins are described in the Examples sections below. Suitable resins for purification include, but are not limited to, anion exchange resin, albumin affinity resin, C1 inhibitor affinity resin, and protein A resin. In some embodiments, a purification method that does not require a drop in pH is preferred. In some embodiments, a purification method that does not require an acidic pH for elution is preferred. In other embodiments, a stabilizer that prevents aggregation is utilized. In some embodiments, the stabilizer that prevents aggregation is used in a method requiring a drop in pH. Non-limiting examples of suitable stabilizers include EDTA.

Suitable methods for purification of the C1-INH fusion proteins of the invention include, but are not limited to, those described in, e.g., U.S. Patent Nos. 5,276,141; US7384754; 8,802,816; PCT Publication Nos. WO2012107572; and WO2013009526.

### Pharmaceutical compositions

The present invention further provides a pharmaceutical composition containing a recombinant C1-INH fusion protein having an amino acid sequence selected from: SEQ ID NO:11, SEQ ID NO:13, SEQ ID NO:14, SEQ ID NO:15, or SEQ ID NO:16 and a physiologically acceptable carrier or excipient. The carrier and recombinant C1-INH fusion protein can be sterile. The formulation should suit the mode of administration.

Suitable pharmaceutically acceptable carriers include but are not limited to water, salt solutions (*e.g*., NaCl), saline, buffered saline, alcohols, glycerol, ethanol, gum arabic, vegetable oils, benzyl alcohols, polyethylene glycols, gelatin, carbohydrates such as lactose, amylose or starch, sugars such as mannitol, sucrose, or others, dextrose, magnesium stearate, talc, silicic acid, viscous paraffin, perfume oil, fatty acid esters, hydroxymethylcellulose, polyvinyl pyrrolidone, *etc*., as well as combinations thereof. The pharmaceutical preparations can, if desired, be mixed with auxiliary agents (*e.g*., lubricants, preservatives, stabilizers, wetting agents, emulsifiers, salts for influencing osmotic pressure, buffers, coloring, flavoring and/or aromatic substances and the like) which do not deleteriously react with the active compounds or interference with their activity. In a preferred embodiment, a water-soluble carrier suitable for intravenous administration is used.

A suitable pharmaceutical composition or medicament, if desired, can also contain minor amounts of wetting or emulsifying agents, or pH buffering agents. A composition can be a liquid solution, suspension, emulsion, tablet, pill, capsule, sustained release formulation, or powder. A composition can also be formulated as a suppository, with traditional binders and carriers such as triglycerides. Oral formulations can include standard carriers such as pharmaceutical grades of mannitol, lactose, starch, magnesium stearate, polyvinyl pyrrolidone, sodium saccharine, cellulose, magnesium carbonate, *etc.*

A pharmaceutical composition or medicament can be formulated in accordance with the routine procedures as a pharmaceutical composition adapted for administration to human beings. For example, in some embodiments, a composition for intravenous administration typically is a solution in sterile isotonic aqueous buffer. Where necessary, the composition may also include a solubilizing agent and a local anesthetic to ease pain at the site of the injection. Generally, the ingredients are supplied either separately or mixed together in unit dosage form, for example, as a dry lyophilized powder or water free concentrate in a hermetically sealed container such as an ampule or sachette indicating the quantity of active agent. Where the composition is to be administered by infusion, it can be dispensed with an infusion bottle containing sterile pharmaceutical grade water, saline or dextrose/water. Where the composition is administered by injection, an ampule of sterile water for injection or saline can be provided so that the ingredients may be mixed prior to administration.

A recombinant C1-INH fusion protein described herein can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, *etc.*

A preferred formulation comprises 50 mM NaPO4 (pH 7.2), 50 mM Sorbitol, and 150 mM Glycine. The formulation may be liquid, or may be lyophilized and reconstituted prior to administration.

### Routes of Administration

A recombinant C1-INH fusion protein described herein (or a composition or medicament containing a recombinant C1-INH fusion protein described herein) is administered by any appropriate route. In some embodiments, a recombinant C1-INH fusion protein or a pharmaceutical composition containing the same is administered systemically. Systemic administration may be intravenous, intradermal, intracranial, intrathecal, inhalation, transdermal (topical), intraocular, intramuscular, subcutaneous, intramuscular, oral, and/or transmucosal administration. In some embodiments, a recombinant C1-INH fusion protein or a pharmaceutical composition containing the same is administered subcutaneously. As used herein, the term "subcutaneous tissue", is defined as a layer of loose, irregular connective tissue immediately beneath the skin. For example, the subcutaneous administration may be performed by injecting a composition into areas including, but not limited to, the thigh region, abdominal region, gluteal region, or scapular region. In some embodiments, a recombinant C1-INH fusion protein or a pharmaceutical composition containing the same is administered intravenously. In some embodiments, a recombinant C1-INH fusion protein or a pharmaceutical composition containing the same is administered orally. In some embodiments, a recombinant C1-INH fusion protein or a pharmaceutical composition containing the same is administered intracranially. In some embodiments, a recombinant C1-INH fusion protein or a pharmaceutical composition containing the same is administered intrathecally. More than one route can be used concurrently, if desired.

In some embodiments, a recombinant C1-INH fusion protein or a pharmaceutical composition containing the same is administered to a subject by intrathecal administration. As used herein, the term "intrathecal administration" or "intrathecal injection" refers to an injection into the spinal canal (intrathecal space surrounding the spinal cord). Various techniques may be used including, without limitation, lateral cerebroventricular injection through a burrhole or cisternal or lumbar puncture or the like. In some embodiments, "intrathecal administration" or "intrathecal delivery" according to the present invention refers to IT administration or delivery via the lumbar area or region, i.e., lumbar IT administration or delivery. As used herein, the term "lumbar region" or "lumbar area" refers to the area between the third and fourth lumbar (lower back) vertebrae and, more inclusively, the L2-S1 region of the spine.

In some embodiments, a recombinant C1-INH fusion protein or a pharmaceutical composition containing the same is administered to the subject by subcutaneous (i.e., beneath the skin) administration. For such purposes, the formulation may be injected using a syringe. However, other devices for administration of the formulation are available such as injection devices (e.g., the Inject-ease™ and Genject™ devices); injector pens (such as the GenPen™); needleless devices (e.g., MediJector™ and BioJector™); and subcutaneous patch delivery systems.

In some embodiments, intrathecal administration may be used in conjunction with other routes of administration (e.g., intravenous, subcutaneously, intramuscularly, parenterally, transdermally, or transmucosally (*e.g*., orally or nasally)).

The present disclosure contemplates single as well as multiple administrations of a therapeutically effective amount of a recombinant C1-INH fusion protein or a pharmaceutical composition containing the same described herein. A recombinant C1-INH fusion protein or a pharmaceutical composition containing the same can be administered at regular intervals, depending on the nature, severity and extent of the subject's condition (e.g., a lysosomal storage disease). In some embodiments, a therapeutically effective amount of a recombinant C1-INH fusion protein or a pharmaceutical composition containing the same may be administered periodically at regular intervals (e.g., once every year, once every six months, once every five months, once every three months, bimonthly (once every two months), monthly (once every month), biweekly (once every two weeks), weekly, daily or continuously).

In some embodiments, administration results only in a localized effect in an individual, while in other embodiments, administration results in effects throughout multiple portions of an individual, for example, systemic effects. Typically, administration results in delivery of a recombinant C1-INH fusion protein to one or more target tissues. In some embodiments, the recombinant C1-INH fusion protein is delivered to one or more target tissues including, but not limited to, heart, brain, skin, blood, spinal cord, striated muscle (e.g., skeletal muscle), smooth muscle, kidney, liver, lung, and/or spleen. In some embodiments, the recombinant C1-INH fusion protein is delivered to the heart. In some embodiments, the recombinant C1-INH fusion protein is delivered to the central nervous system, particularly the brain and/or spinal cord. In some embodiments, the recombinant C1-INH fusion protein is delivered to triceps, tibialis anterior, soleus, gastrocnemius, biceps, trapezius, deltoids, quadriceps, and/or diaphragm.

### Dosage Forms and Dosing Regimen

In some embodiments of the present disclosure, a composition is administered in a therapeutically effective amount and/or according to a dosing regimen that is correlated with a particular desired outcome (e.g., with prophylaxis of a complement-mediated chronic disease, such as HAE).

Particular doses or amounts to be administered in accordance with the present disclosure may vary, for example, depending on the nature and/or extent of the desired outcome, on particulars of route and/or timing of administration, and/or on one or more characteristics (e.g., weight, age, personal history, genetic characteristic, lifestyle parameter, severity of cardiac defect and/or level of risk of cardiac defect, etc., or combinations thereof). Such doses or amounts can be determined by those of ordinary skill. In some embodiments, an appropriate dose or amount is determined in accordance with standard clinical techniques. Alternatively or additionally, in some embodiments, an appropriate dose or amount is determined through use of one or more *in vitro* or *in vivo* assays to help identify desirable or optimal dosage ranges or amounts to be administered.

In various embodiments of the present disclosure, a recombinant C1-INH fusion protein is administered at a therapeutically effective amount. Generally, a therapeutically effective amount is sufficient to achieve a meaningful benefit to the subject (e.g., prophylaxis, treating, modulating, curing, preventing and/or ameliorating the underlying disease or condition). Generally, the amount of a therapeutic agent (e.g., a recombinant C1-INH fusion protein) administered to a subject in need thereof will depend upon the characteristics of the subject. Such characteristics include the condition, disease severity, general health, age, sex and body weight of the subject. One of ordinary skill in the art will be readily able to determine appropriate dosages depending on these and other related factors. In addition, both objective and subjective assays may optionally be employed to identify optimal dosage ranges. In some particular embodiments, appropriate doses or amounts to be administered may be extrapolated from dose-response curves derived from *in vitro* or animal model test systems.

In some embodiments of the present disclosure, a composition is provided as a pharmaceutical formulation. In some embodiments of the present disclosure, a pharmaceutical formulation is or comprises a unit dose amount for administration in accordance with a dosing regimen correlated with achievement of the reduced incidence or risk of an HAE attack.

In some embodiments of the present disclosure, a formulation comprising a recombinant C1-INH fusion protein described herein administered as a single dose. In some embodiments of the present disclosure, a formulation comprising a recombinant C1-INH fusion protein described herein is administered at regular intervals. Administration at an "interval," as used herein, indicates that the therapeutically effective amount is administered periodically (as distinguished from a one-time dose). The interval can be determined by standard clinical techniques. In some embodiments of the present disclosure, a formulation comprising a recombinant C1-INH fusion protein described herein is administered bimonthly, monthly, twice monthly, triweekly, biweekly, weekly, twice weekly, thrice weekly, daily, twice daily, or every six hours. The administration interval for a single individual need not be a fixed interval, but can be varied over time, depending on the needs of the individual.

A therapeutically effective amount is commonly administered in a dosing regimen that may comprise multiple unit doses. For any particular therapeutic protein, a therapeutically effective amount (and/or an appropriate unit dose within an effective dosing regimen) may vary, for example, depending on route of administration, on combination with other pharmaceutical agents. Also, the specific therapeutically effective amount (and/or unit dose) for any particular patient may depend upon a variety of factors including the disorder being treated and the severity of the disorder; the activity of the specific pharmaceutical agent employed; the specific composition employed; the age, body weight, general health, sex and diet of the patient; the time of administration, route of administration, and/or rate of excretion or metabolism of the specific fusion protein employed; the duration of the treatment; and like factors as is well known in the medical arts.

As used herein, the term "bimonthly" means administration once per two months (*i.e.,* once every two months); the term "monthly" means administration once per month; the term "triweekly" means administration once per three weeks (*i.e*., once every three weeks); the term "biweekly" means administration once per two weeks (*i.e*., once every two weeks); the term "weekly" means administration once per week; and the term "daily" means administration once per day.

In some embodiments of the present disclosure, a formulation comprising a recombinant C1-INH fusion protein described herein is administered at regular intervals indefinitely. In some embodiments of the present disclosure, a formulation comprising a recombinant C1-INH fusion protein described herein is administered at regular intervals for a defined period.

It is to be further understood that for any particular subject, specific dosage regimens should be adjusted over time according to the individual need and the professional judgment of the person administering or supervising the administration of the enzyme replacement therapy and that dosage ranges set forth herein are exemplary only and are not intended to limit the scope or practice of the claimed invention.

### Combination Therapy

In some embodiments of the present disclosure, a recombinant C1-INH fusion protein is administered in combination with one or more known therapeutic agents (e.g., corticosteroids) currently used for treatment of a complement-mediated disease. In some embodiments of the present disclosure, the known therapeutic agent(s) is/are administered according to its standard or approved dosing regimen and/or schedule. In some embodiments of the present disclosure, the known therapeutic agent(s) is/are administered according to a regimen that is altered as compared with its standard or approved dosing regimen and/or schedule. In some embodiments of the present disclosure, such an altered regimen differs from the standard or approved dosing regimen in that one or more unit doses is altered (e.g., reduced or increased) in amount, and/or in that dosing is altered in frequency (e.g., in that one or more intervals between unit doses is expanded, resulting in lower frequency, or is reduced, resulting in higher frequency).

### A. Disorders

Preferred embodiment is treatment of chronic disorders

In some embodiments, the fusion proteins provided by the invention are suitable for acute attacks associated with complement-mediated disorders, e.g., NMOSD AMR, and HAE events. These attacks may be long or short. In some embodiments, the disease or disorder is chronic. In some embodiments of the present disclosure the compositions and methods of the invention are used prophylactically. Exemplary complement-mediated disease that may be treated using the compositions and methods disclosed herein include, but are not limited to, hereditary angioedema, antibody mediated rejection, neuromyelitis optica spectrum disorders, traumatic brain injury, spinal cord injury, ischemic brain injury, burn injury, toxic epidermal necrolysis, multiple sclerosis, amyotrophic lateral sclerosis (ALS), Parkinson's disease, stroke, chronic inflammatory demyelinating polyneuropathy (CIDP), myasthenia gravis, multifocal motor neuropathy.

### EXAMPLES

Other features, objects, and advantages of the present invention are apparent in the examples that follow. It should be understood, however, that the examples, while indicating embodiments of the present invention, are given by way of illustration only, not limitation. Various changes and modifications within the scope of the invention will become apparent to those skilled in the art from the examples.

### Example 1. Fc Fusion Proteins

This example illustrates various exemplary fusion protein constructs and transient or scale-up expression of such fusion protein constructs in mammalian cells. As shown below, wild-type or mutant Fc moieties are fused to full length (1-478 aa) or truncated (98-478 aa) human C1-inhibitor.

### A. Fc-C1-Inhibitor Fusion Expression Constructs

A fusion protein of full Length C1-inhibitor with an N-terminal IgG1 Fc was made according to the methods described above, having the amino acid sequence (the Fc moiety is underlined):

DKTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP

VLDSDGSFFLYSKLTVDKSRWQQGNVFSCSVMHEALHNHYTQKSLSLSPGKNPNATSSS
SQDPESLQDRGEGKVATTVISKMLFVEPILEVSSLPTTNSTTNSATKITANTTDEPTTQPTT
EPTTQPTIQPTQPTTQLPTDSPTQPTTGSFCPGPVTLCSDLESHSTEAVLGDALVDFSLKL
YHAFSAMKKVETNMAFSPFSIASLLTQVLLGAGENTKTNLESILSYPKDFTCVHQALKG
FTTKGVTSVSQIFHSPDLAIRDTFVNASRTLYSSSPRVLSNNSDANLELINTWVAKNTNN
KISRLLDSLPSDTRLVLLNAIYLSAKWKTTFDPKKTRMEPFHFKNSVIKVPMMNSKKYP
VAHFIDQTLKAKVGQLQLSHNLSLVILVPQNLKHRLEDMEQALSPSVFKAIMEKLEMSK
FQPTLLTLPRIKVTTSQDMLSIMEKLEFFDFSYDLNLCGLTEDPDLQVSAMQHQTVLELT
ETGVEAAAASAISVARTLLVFEVQQPFLFVLWDQQHKFPVFMGRVYDPRA (SEQ ID
NO:11).

A fusion protein of truncated C1 -inhibitor with an N-terminal IgG1 Fc was made according to the methods described above, having the amino acid sequence (the Fc moiety is underlined):

DKTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGKGSFCPGP
VTLCSDLESHSTEAVLGDALVDFSLKLYHAFSAMKKVETNMAFSPFSIASLLTQVLLGA
GENTKTNLESILSYPKDFTCVHQALKGFTTKGVTSVSQIFHSPDLAIRDTFVNASRTLYSS
SPRVLSNNSDANLELINTWVAKNTNNKISRLLDSLPSDTRLVLLNAIYLSAKWKTTFDPK
KTRMEPFHFKNSVIKVPMMNSKKYPVAHFIDQTLKAKVGQLQLSHNLSLVILVPQNLKH
RLEDMEQALSPSVFKAIMEKLEMSKFQPTLLTLPRIKVTTSQDMLSIMEKLEFFDFSYDL
NLCGLTEDPDLQVSAMQHQTVLELTETGVEAAAASAISVARTLLVFEVQQPFLFVLWD
QQHKFPVFMGRVYDPRA (SEQ ID NO:12).

Truncated C1-inhibitor with an N-terminal IgG1 Fc eliminates a large amount of the carbohydrate sites due to the removal of the serine and threonine residues on the truncated portion of the C1-INH polypeptide. These amino acids have OH moieties that become glycosylated in post-translational processing. Without wishing to be bound by any theory, the elimination of this carbohydrate reduces potential clearance of the molecule via the asialoglycoprotein receptor, which may extend half-life of the Fc-C1-inhibitor fusion protein.

Without wishing to be bound by any theory, asialoglycoprotein is not the only clearance mechanism. There are also active clearance pathways based on presence of other glycans. The Fc domain does not bind the FcRN receptor until the complex has been internalized in an endosome. The drop in pH within the endosome allows for the binding to occur. In some embodiments, the active clearance processes are reduced, slowed, and/or eliminated by manipulation of the glycosylation profile of the C1-INH fusion construct. This allows for the passive Fc recycling process to be effective in increasing half-life. In some embodiments, a truncated C1-INH polypeptide is preferred. The truncation may remove glycosylation moieties associated with active clearance. Further, the truncated form is smaller in size which may increase absorption, particularly when administered subcutaneously.

IgG1 LALA mutation eliminates the complement activation and ADCC that can happen from wild type IgG1 Fc sequence. Both full length and truncated C1-INH IgG1 Fc effector dead constructs were made.

Full-length C1-inhibitor with an N-terminal IgG1 LALA Fc was made according to the methods described above having the amino acid sequence (the Fc moiety is underlined, LALA mutation bolded):

DKTHTCPPCPAPEAAGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGKNPNATSSS
SQDPESLQDRGEGKVATTVISKMLFVEPILEVSSLPTTNSTTNSATKITANTTDEPTTQPTT
EPTTQPTIQPTQPTTQLPTDSPTQPTTGSFCPGPVTLCSDLESHSTEAVLGDALVDFSLKL
YHAFSAMKKVETNMAFSPFSIASLLTQVLLGAGENTKTNLESILSYPKDFTCVHQALKG
FTTKGVTSVSQIFHSPDLAIRDTFVNASRTLYSSSPRVLSNNSDANLELINTWVAKNTNN
KISRLLDSLPSDTRLVLLNAIYLSAKWKTTFDPKKTRMEPFHFKNSVIKVPMMNSKKYP
VAHFIDQTLKAKVGQLQLSHNLSLVILVPQNLKHRLEDMEQALSPSVFKAIMEKLEMSK
FQPTLLTLPRIKVTTSQDMLSIMEKLEFFDFSYDLNLCGLTEDPDLQVSAMQHQTVLELT
ETGVEAAAASAISVARTLLVFEVQQPFLFVLWDQQHKFPVFMGRVYDPRA (SEQ ID
NO:13).

Truncated C1-inhibitor with an N-terminal IgG1 LALA Fc was made according to the methods described above having the amino acid sequence (the Fc moiety is underlined, LALA mutation bolded):

DKTHTCPPCPAPEAAGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGKGSFCPGP
VTLCSDLESHSTEAVLGDALVDFSLKLYHAFSAMKKVETNMAFSPFSIASLLTQVLLGA
GENTKTNLESILSYPKDFTCVHQALKGFTTKGVTSVSQIFHSPDLAIRDTFVNASRTLYSS
SPRVLSNNSDANLELINTWVAKNTNNKISRLLDSLPSDTRLVLLNAIYLSAKWKTTFDPK
KTRMEPFHFKNSVIKVPMMNSKKYPVAHFIDQTLKAKVGQLQLSHNLSLVILVPQNLKH
RLEDMEQALSPSVFKAIMEKLEMSKFQPTLLTLPRIKVTTSQDMLSIMEKLEFFDFSYDL
NLCGLTEDPDLQVSAMQHQTVLELTETGVEAAAASAISVARTLLVFEVQQPFLFVLWD
QQHKFPVFMGRVYDPRA (SEQ ID NO:14).

A fusion protein of truncated C1 -inhibitor with an N-terminal IgG4 Fc is made according to the methods described above, having the amino acid sequence (the Fc moiety is underlined):

ESKYGPPCPSCPAPEFLGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSLGKNPNATSSS
SQDPESLQDRGEGKVATTVISKMLFVEPILEVSSLPTTNSTTNSATKITANTTDEPTTQPTT
EPTTQPTIQPTQPTTQLPTDSPTQPTTGSFCPGPVTLCSDLESHSTEAVLGDALVDFSLKL
YHAFSAMKKVETNMAFSPFSIASLLTQVLLGAGENTKTNLESILSYPKDFTCVHQALKG
FTTKGVTSVSQIFHSPDLAIRDTFVNASRTLYSSSPRVLSNNSDANLELINTWVAKNTNN
KISRLLDSLPSDTRLVLLNAIYLSAKWKTTFDPKKTRMEPFHFKNSVIKVPMMNSKKYP
VAHFIDQTLKAKVGQLQLSHNLSLVILVPQNLKHRLEDMEQALSPSVFKAIMEKLEMSK
FQPTLLTLPRIKVTTSQDMLSIMEKLEFFDFSYDLNLCGLTEDPDLQVSAMQHQTVLELT
ETGVEAAAASAISVARTLLVFEVQQPFLFVLWDQQHKFPVFMGRVYDPRA (SEQ ID
NO:32).

A fusion protein of truncated C1 -inhibitor with an N-terminal IgG4 Fc is made according to the methods described above, having the amino acid sequence (the Fc moiety is underlined):

ESKYGPPCPSCPAPEFLGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSLGKGSFCPGPV
TLCSDLESHSTEAVLGDALVDFSLKLYHAFSAMKKVETNMAFSPFSIASLLTQVLLGAG
ENTKTNLESILSYPKDFTCVHQALKGFTTKGVTSVSQIFHSPDLAIRDTFVNASRTLYSSS
PRVLSNNSDANLELINTWVAKNTNNKISRLLDSLPSDTRLVLLNAIYLSAKWKTTFDPKK
TRMEPFHFKNSVIKVPMMNSKKYPVAHFIDQTLKAKVGQLQLSHNLSLVILVPQNLKHR
LEDMEQALSPSVFKAIMEKLEMSKFQPTLLTLPRIKVTTSQDMLSIMEKLEFFDFSYDLN
LCGLTEDPDLQVSAMQHQTVLELTETGVEAAAASAISVARTLLVFEVQQPFLFVLWDQ
QHKFPVFMGRVYDPRA (SEQ ID NO:33).

Full-length C1-inhibitor with an N-terminal IgG4 S241P Fc was made according to the methods described above having the amino acid sequence (the Fc moiety is underlined, S241P mutation bolded):

ESKYGPPCPPCPAPEFLGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSLGKNPNATSSS
SQDPESLQDRGEGKVATTVISKMLFVEPILEVSSLPTTNSTTNSATKITANTTDEPTTQPTT
EPTTQPTIQPTQPTTQLPTDSPTQPTTGSFCPGPVTLCSDLESHSTEAVLGDALVDFSLKL
YHAFSAMKKVETNMAFSPFSIASLLTQVLLGAGENTKTNLESILSYPKDFTCVHQALKG

FTTKGVTSVSQIFHSPDLAIRDTFVNASRTLYSSSPRVLSNNSDANLELINTWVAKNTNN
KISRLLDSLPSDTRLVLLNAIYLSAKWKTTFDPKKTRMEPFHFKNSVIKVPMMNSKKYP
VAHFIDQTLKAKVGQLQLSHNLSLVILVPQNLKHRLEDMEQALSPSVFKAIMEKLEMSK
FQPTLLTLPRIKVTTSQDMLSIMEKLEFFDFSYDLNLCGLTEDPDLQVSAMQHQTVLELT
ETGVEAAAASAISVARTLLVFEVQQPFLFVLWDQQHKFPVFMGRVYDPRA (SEQ ID
NO:15).

Truncated C1 -inhibitor with an N-terminal IgG4 S241P Fc was made according to the methods described above having the amino acid sequence (the Fc moiety is underlined, S241P mutation bolded):

ESKYGPPCPPCPAPEFLGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPP
VLDSGSFFLYSRLTVDKSRWQEGNVFSCSVMHEALHNHYTQKSLSLSLGKGSFCPGPV
TLCSDLESHSTEAVLGDALVDFSLKLYHAFSAMKKVETNMAFSPFSIASLLTQVLLGAG
ENTKTNLESILSYPKDFTCVHQALKGFTTKGVTSVSQIFHSPDLAIRDTFVNASRTLYSSS
PRVLSNNSDANLELINTWVAKNTNNKISRLLDSLPSDTRLVLLNAIYLSAKWKTTFDPKK
TRMEPFHFKNSVIKVPMMNSKKYPVAHFIDQTLKAKVGQLQLSHNLSLVILVPQNLKHR
LEDMEQALSPSVFKAIMEKLEMSKFQPTLLTLPRIKVTTSQDMLSIMEKLEFFDFSYDLN
LCGLTEDPDLQVSAMQHQTVLELTETGVEAAAASAISVARTLLVFEVQQPFLFVLWDQ
QHKFPVFMGRVYDPRA (SEQ ID NO:16).

### B. Small Scale Transient Expression of Fc-C1-inhibitor Fusion Proteins

FreeStyle™ 293-F Cells (suspension human embryonal kidney cells, Invitrogen, Cat. No. R790-07), FreeStyle™ CHO-S Cells (suspension Chinese Hamster Ovary cells, Invitrogen, Cat. No. R800-07), and HT1080 cells were transfected with various C1-inhibitor fusion plasmids (N-hFc, N-hFcLALA and N-hFcIgG4m), and with no DNA as a control.

The transfections of the 293-F and CHO-S cells were carried out using 293-FreeStyle™ MAX Reagent (Invitrogen, Cat. No. 16447-500) following Invitrogen's protocol (Invitrogen Protocol Pub. No. MAN0007818 Rev. 1.0, available at https://tools.thermofisher.com/content/sfs/manuals/FreeStyle_MAX_Reagent_man.pdf). After transfection, the 293-F cells were seeded into serum-free FreeStyle™ 293 Expression Medium (Invitrogen, Cat. No. 12338-018) and the CHO-S cells were seeded into serum-free FreeStyle™ CHO Expression Medium (Invitrogen, Cat. No. 12651-014), both having a final culture volume of 30 mL.

Similarly, HT1080 cells were transfected using a standard transfection protocol (350V, 960uF, 12e6 cells, 35µg DNA).

Conditioned Medium (CM) from the transfected cell cultures was harvested 4 days and 7 days (3 day accumulation after re-feed) post transfection. Fusion protein expression was evaluated by SDS-PAGE (8-16% TG gel, 150V for 1.5h) and visualized with Coomassie blue.

In order to generate stable pools of effector dead constructs, the HT1080 transiently transfected cells were seeded at 1x10⁶ cells/mL at 37°C. After two days of recovery, cells wereselected for 2 weeks in a suitable chemically defined (CD) medium_with 250 µg/mL zeocin until cells exhibited 95% viability. Fusion protein expression was evaluated by SDS-PAGE and visualized with Coomassie blue. Expression of the C1-INH Fc fusion constructs was found to be greater than that of the C1-INH albumin constructs.

### C. Scale-up Expression using FreeStyle™ 293-F Cells

FreeStyle™ 293-F Cells were transfected with LALA-C1-inhibitor fusion plasmids (both full-length and truncated C1) as well as IgG4m-C1-inhibitor fusion plasmids (full-length and truncated C1). Transfections were carried out using 293-FreeStyle™ MAX Reagent as described above.

Cells were transfected in 1 L shake flasks with 320 mL working volume. DNA and FreeStyle™ MAX Reagent quantities were scaled-up proportionally from the 30 mL transfection volume of the transfections described above. CM from transfected cells was harvested 4 days post transfection and expression was evaluated with SDS-PAGE, as described above. Cells were re-fed for a second harvest (7 days post transfection, 3 day batch collection).

### C. Expression Data Summary

Fc fusions with full length C1-inhibitor consistently express at higher levels than native recombinant C1. Fc fusions with truncated C1-inhibitor express similarly to native recombinant C1-inhibitor. No severe clipping of the proteins was observed in any of the host cell culture samples for any of the tested constructs.

HEK293 transient cell expression was particularly increased compared to the other tested cell types. Placement of the Fc sequence at the N-terminus resulted in particularly increased expression than if on the C-terminus

### Example 2. Characterization of Fc-C1-INH Fusion Proteins

To characterize Fc-C1-inhibitor fusion proteins, the fusion proteins were first purified using a process involving protein A column. Exemplary purification results are shown in FIG. 3.

C1-inhibitor fusion composition concentrations and total protein detected in samples are depicted in FIG. 4. Highly purified material was obtained after a single affinity column purification. Excellent protein yields were achieved using the purification process described above.

C1-inhibitor fusion composition endotoxin concentrations detected in samples are depicted in FIG. 5. Low endotoxin levels were found in the final products, making them eminently suitable for pharmaceutical use.

The purified Fc Fusion proteins outlined above were analyzed for protein purity, SEC, thermal stability, binding to FcRN, binding to C1q, and binding to FcgR1.

All samples for PK analysis were above 97% pure by RP HPLC. Size exclusion chromatography of C1-inhibitor fusion proteins was performed under the following exemplary conditions: Column: Tosoh G3000swx1; Mobile Phase Buffer:0.2M sodium phosphate pH 6.8; Flow Rate:0.5 mL/min

Samples were injected at volume of 40 µL. The samples run were LALA C1-inhibitor full length(FL) R1; LALA C1-inhibitor truncated (TR) R1; IgG4 C1 -inhibitor full length (FL) R1; IgG4 C1-inhibitor truncated (TR) R1; and a 20 µL BSA control injection. results

Exemplary results of the SEC-MALS are depicted in Table 1.

**Table 1: SEC-MALS Results**

| **Sample Name** | **g/mol** |
|---|---|
| **LALA FL R1** | 195,600 |
| **LALA TR R1** | Peak1=385,000 Peak2=184,000 |
| **IgG4 FL R1** | Peak1=984,200 Peak2=212,300 |
| **IgG4 TR R1** | Peak1=360,300 Peak2=191,900 |

Thermal stability of C1-inhibitor fusion protein constructs was assessed by differential scanning calorimetry (DSC).

### A. Fc Fusion Protein Binding to FcRN and Fc Effector Function

Binding to the Fc neonatal receptor (FcRN) allows for recycling of the molecules and leads to an extended in vivo serum half-life of the Fc fusion proteins. Recycling occurs as the molecules are passively taken into the cells and the pH of the endosomes is lower. That leads to binding of the Fc portion of the molecule to the FcRN. When the FcRN recycles back to the surface of the cell, the pH is then neutral and the protein is released back into the serum.

Binding to the extracellular domain of the FcRN was measured by surface plasmon resonance (SPR) using a Biacore system.

Direct immobilization with FcRn was achieved via amine coupling of a CM5 chip with FcRn under the following conditions:
FcRn (Sino Biological Inc or in-house BD1) is diluted in Acetate buffer pH 5.0 (Cat#BR-1003-51) to 10 µg/mL.
Flow rate: 10 µl/min
Final coupling signal 354 Ru
Running buffer: PBS-P+, pHed to 6.0

The kinetic binding study was done using the following protocol. Samples were diluted in PBS-P+ to 100, 50, 25, 12.5, 6.25, 3.125, 0 nM. The parameters were set as follows:
Association 300s and Dissociation 600s at Flow rate 30 µL/min
Regeneration with 25 mM Tris, 150mM NaCl pH 8.0

A summary of exemplary kinetic binding data with FcRn is depicted in Table 2.

**Table 2: Summary of Kinetic binding data with FcRn**

| | **Constructs** | **ka (1/Ms)** | **kd (1/s)** | **KD** |
|---|---|---|---|---|
| **C1-Inh Full Length** | **N-hFcLala-C1-INH** | 2.19E+05 | 0.003019 | 1.38E-08 |
| | **N-IgG4m-C1-INH** | 1.87E+05 | 0.005616 | 3.00E-08 |
| **C1-Inh Truncated** | **N-hFcLala-C1-INH Tr** | 8.42E+04 | 0.008214 | 9.76E-08 |
| | **N-IgG4m-C1-INH Tr** | 1.34E+05 | 0.009462 | 7.07E-08 |

### B. Fc Fusion Protein Binding to C1q Binding

Wild Type Fc molecules have varying amounts of effector function, e.g., the ability to activate the complement cascade, ADCC activity, and/or to bind to specific Fc receptors that are not desired for the purposes of the present invention. Patients in need of the C1-INH fusion proteins of the invention suffer from complement-mediated disease, thus it is an object of the invention to reduce or eliminate effector functions associated with Fc domains. The C1-INH Fc fusion constructs disclosed herein preferably reduce or eliminate effector functions. The C1-INH Fc fusion constructs preferably reduce or eliminate complement activation, ADCC, and/or Fcγ Receptors. Effector function can be measured by binding to C1q, also by binding to Fcγ Receptors.

### C1q Binding ELISA

The C1q binding ELISA protocol used was:
- Protein was coated onto maxisorp plate and titrated from 100µg/mL to 0ug/mL in 50mM Carbonate Buffer pH 9.6 O/N at 4°C
- 3 washes with ELISA Wash Buffer (PBS/0.05% TWEEN-20)
- Add 2µg/mL C1q in Assay Buffer (PBS/0.05% TWEEN-20/0.1% fish gelatin) and incubate for 2h at RT
- 3 washes with ELISA Wash Buffer (PBS/0.05% TWEEN-20)
- Add anti-Clq-HRP antibody (Thermo PA1-84324) at 4µg/mL and incubate 1h at RT
- 3 washes with ELISA Wash Buffer (PBS/0.05% TWEEN-20)
- TMB for 15min at RT
- Read OD450

Exemplary C1q binding ELISA results for the hFc, hFcLALA and IgG4m fusions with C1-INH and rhC1 (plasma-derived commercial product) Inhibitor are depicted in FIG. 6 which plots the OD450 against the concentration of protein in each sample. The samples were hFc IgGl-Cl-INH, hFc LALA IgGl-Cl-INH, and hFc IgG4m-Cl-INH fusions, recombinant human C1-INH (rhCl-INH) (expressed in 1080 cells), and IgG1 Fc-human follistatin(hFc IgGl-hFst-XTEN) fusion as a positive control and human follistatin-Xten (hFst-XTEN) fusion as a negative control.

### Fc Fusion Protein Binding to FcγR1

Binding to the extracellular domain of the FcyRl was measured by surface plasmon resonance (SPR) using a Biacore system. The Biacore capture approach is depicted in FIG. 7. The assay setup was as follows:
HBS-P+ used as running buffer
Coupling anti-His (GE, anti-His Kit) on CM5 to 16000Ru

### Capture level

For FcγRI (5 µg/mL R&D system), capture level at ∼350 Ru

### Sample preparation

Positive control: N-hFc-Cl-INH is diluted in HBS-P+ buffer to 12.5, 6.25, 3.175, 1.59 and 0.79 nM
Other samples (N-hFcIgG4m-C1 Inh, N-hFcIgG4m-C1-INH Tr, N-hFcLala-C1-INH and
N-hFclala-C1-INH Tr) are diluted in HBS-P+buffer to 100, 50, 25, 12.5, 6.25 nM

### Binding Kinetic setup:

Capture: 12s injection at 10 µl/mL
Capture stabilization: 30s
Association: 300s at Flow rate 30 µl/mL
Dissociation: 600s at Flow rate 30 µl/mL
Regeneration: 40s at 30 µl/mL with 10 mM phosphate 500mM NaCl pH 2.5

Summary of exemplary data illustrating C1-INH constructs binding with FcγRI is shown in Table 3. N-hFcLala-Cl-INH abolished binding with FcγRI. N-hFcIgG4m-Cl-INH and N-hFcIgG4m-Cl-INH Tr binds to FcyRI, but weaker than N-hFc-Cl-INH. Truncated Cl-INH with hFcLala or hFcIgG4m fusion increases binding affinity with FcγRI.

**Table 3: Summary of C1-INH construct FcγRI binding**

| **Samples** | **ka1 (1/Ms)** | **kd1 (1/s)** | **KD** |
|---|---|---|---|
| **N-hFc-C1 Inh** | 2.496E+5 | 5.277E-4 | 2.114E-9 |
| **N-hFcLala-C1 Inh** | No binding | | |
| **N-hFcLala-C1-INH Tr** | 1.403E+4 | 0.001833 | 1.306E-7 |
| **N-hFcIgG4m-C1 Inh** | 7.232E+4 | 0.001673 | 2.313E-8 |
| **N-hFcIgG4m-C1-INH Tr** | 1.183E+5 | 8.616E-4 | 7.281E-9 |

### C1-inhibitor Activity of the C1-inhibitor Fc Fusion Proteins

C1-inhibitor is capable of inhibiting many enzymes from the coagulation, contact and complement pathways, and thus useful in the treatment of diseases such as HAE, AMR, NMOSD, and PNH.

C1-INH is a suicide inhibitor, meaning it is inactivated during the process of inhibition. It forms a 1:1 stoichiometric complex with the target protease, followed by clearance of the entire complex. The C1-INH is then no longer available to inhibit other enzymes. The following experiments examined the ability of the Fc-C1-inhibitor fusion proteins to inhibit C1s by two exemplary methods.

Two exemplary in vitro approaches were taken to examine the inhibitory activity of C1-inhibitor on C1s activity: measuring the complex of C1S and C1-inhibitor in an ELISA based method (functional ELISA)- only used on effector dead constructs and measuring the inhibition of the ability of C1s to cleave a colorimetric substrate. The two assays resulted in agreeing data.

### Functional ELISA measuring the complex of C1S and C1-inhibitor

This method was used to test the effector dead Fc constructs made for PK studies FIG. 8. The ELISA protocol was as follows:
- 60 µL diluted C1-INH + 12 µL biotin-Cls
   ∘ 30 min incubation at RT
- Transfer 50 µL of complexes to streptavidin coated plate
   ∘ 10 min incubation at RT, wash 5x
- Add 50 µL of anti-C 1 -INH-HRP
   ∘ 60 min incubation at RT, wash 5x
- Add 100 µL of TMB substrate
   ∘ 15 minute incubation at RT
- Add 100 µL of Stop solution (4%HCl)
- Read at OD450

Plasma-derived C1-INH molecular weight (MW) was estimated by gel to be about 93,000 Da. This value was used to calculate the concentration of protein used in the SPR analysis. The concentration of the constructs was calculated by the weight of each construct as determined using mass spectrometry. FL C1-INH hFc IgG1 LALA and FL C1-INH hFc IgG4m have MW of approximately 200,000 Da. The Tr C1-INH hFc IgG1 LALA and Tr C1-INH hFc IgG4m have MW of approximately 160,000 Da.

In multiple tests, the fusion proteins demonstrated a higher affinity to C1s over native C1-inhibitor. The truncated C1-INH fusion protein similarly demonstrated a higher affinity to C1s over native C1-inhibitor.

### Inhibition of the ability of C1s to cleave a colorimetric substrate

The ability of the effector dead constructs' ability to inhibit C1s cleavage of a colorimetric peptide by C1s +/- C1-INH was tested using an R&D systems activity assay for Cl-inhibitor. The assay protocol can be found at https://www.rndsystems.com/products/human-serpin-g1-c1-inhibitor-plasma-protein-cf_2488-pi.

Final Assay Conditions Per Well:
- rhC1s: 0.010 µg (1.33 nM)
- hSerpin G1 curve: 100, 50, 25, 5, 2.5, 1.25, 0.625, 0.2, 0.04 and 0.01 nM
- Substrate: 100 µM
- DTNB: 100 µM

Activity Assay setup
25 µL Cl-inhibitor + 25 µL C1s (2µg/mL)
30 minute incubation at RT
Dilute complexes 5 fold with assay buffer
50 µL of diluted C1-INH-C1s complexes + 50 µL of substrate(500uM)/reactant(200uM)
15 minute incubation at RT
Read at OD405

Titration curves for each of the effector dead constructs tested in the assay were calculated using mass spec or gel estimates for molecular weights as shown in FIGs. 9A and 9B. The full length fusion protein demonstrated a higher affinity to C1s over native C1-inhibitor. The truncated C1-INH fusion protein similarly demonstrated a higher affinity to C1s over native C1-inhibitor.

### Hemolysis assay

FIGs. 10A and 10B depict a schematic overview of hemolysis assays of the alternative and classical complement pathways. Seelen et al., J. of Immun. Methods, 296: 187-198 (2005). FIGs. 11A, 11B, and 11C depict the comparison of hemolysis activity, e.g, the degree of activation of the alternative pathway of complement, exhibited by plasma-derived Cl-INH, IgG1 LALA Fc truncated Cl-inhibitor, IgG1 LALA Full Length Cl-inhibitor, and two preparation of plasma-derived C1-INH . Fusion proteins behaved similarly to plasma-derived C1 inhibitor.

### Summary of C1-ihhibitor Fc fusion Protein Activity

All of the Fc-C1-inhibitor fusion proteins are able to inhibit C1s activity. The Fc fusion proteins, in particular, full length C1-inhibitor fusion proteins consistently inhibit the C1s with a higher affinity than the plasma-derived C1 -inhibitor. Both full length and truncated C1-inhibitor fusion proteins (e.g. made with Fc IgG1 LALA) are able to inhibit lysis of red blood cells in vitro.

### In vivo PK profiles of Fc Fusion Proteins

FIG. 12 shows exemplary results of a Rabbit PK study of the effector dead fusion constructs compared with plasma-derived C1-inhibitor and recombinant C1-INH. Plasma-derived C1-INH administered by IV exhibits a monophasic serum concentration-time profile. rhC1-INH-IgG constructs administered by IV exhibit bi-phasic serum concentration-time profiles. The initial rapid clearance phase (approximately 2 hrs) was indicative of receptor-mediated cellular uptake. The constructs exhibited a secondary slower clearance phase.

### Example 3. Albumin Fusion Proteins

### A. Albumin-C1-Inhibitor Fusion Expression Constructs

A number of albumin protein constructs were generated. Schematics of these constructs are depicted in FIGs. 13A-F. Fusion construct expression vectors comprising albumin or the D3 domain of albumin joined directly to the N-terminus of full length and truncated Cl-INH joined directly to the N-terminus of full length C1-INH were made. Fusion construct expression vectors comprising albumin or the D3 domain of albumin joined using linkers to the N-terminus of full length and truncated C1-INH joined directly to the N-terminus of full length C1-INH were also made. The amino acid sequences of the fusion proteins expressed by these constructs comprised the following sequences:

Albumin full length C1-INH Direct Fusion (the albumin domain is underlined):

MKWVTFISLLFLFSSAYSRGVFRRDAHKSEVAHRFKDLGEENFKALVLIAFAQYLQQCP
FEDHVKLVNEVTEFAKTCVADESAENCDKSLHTLFGDKLCTVATLRETYGEMADCCAK
QEPERNECFLQHKDDNPNLPRLVRPEVDVMCTAFHDNEETFLKKYLYEIARRHPYFYAP
ELLFFAKRYKAAFTECCQAADKAACLLPKLDELRDEGKASSAKQRLKCASLQKFGERA
FKAWAVARLSQRFPKAEFAEVSKLVTDLTKVHTECCHGDLLECADDRADLAKYICENQ
DSISSKLKECCEKPLLEKSHCIAEVENDEMPADLPSLAADFVESKDVCKNYAEAKDVFL
GMFLYEYARRHPDYSVVLLLRLAKTYKTTLEKCCAAADPHECYAKVFDEFKPLVEEPQ
NLIKQNCELFEQLGEYKFQNALLVRYTKKVPQVSTPTLVEVSRNLGKVGSKCCKHPEAK
RMPCAEDYLSVVLNQLCVLHEKTPVSDRVTKCCTESLVNRRPCFSALEVDETYVPKEFN
AETFTFHADICTLSEKERQIKKQTALVELVKHKPKATKEQLKAVMDDFAAFVEKCCKA
DDKETCFAEEGKKLVAASRAALGLNPNATSSSSQDPESLQDRGEGKVATTVISKMLFVE

PILEVSSLPTTNSTTNSATKITANTTDEPTTQPTTEPTTQPTIQPTQPTTQLPTDSPTQPTTG
SFCPGPVTLCSDLESHSTEAVLGDALVDFSLKLYHAFSAMKKVETNMAFSPFSIASLLTQ
VLLGAGENTKTNLESILSYPKDFTCVHQALKGFTTKGVTSVSQIFHSPDLAIRDTFVNAS
RTLYSSSPRVLSNNSDANLELINTWVAKNTNNKISRLLDSLPSDTRLVLLNAIYLSAKWK
TTFDPKKTRMEPFHFKNSVIKVPMMNSKKYPVAHFIDQTLKAKVGQLQLSHNLSLVILV
PQNLKHRLEDMEQALSPSVFKAIMEKLEMSKFQPTLLTLPRIKVTTSQDMLSIMEKLEFF
DFSYDLNLCGLTEDPDLQVSAMQHQTVLELTETGVEAAAASAISVARTLLVFEVQQPFL
FVLWDQQHKFPVFMGRVYDPRA (SEQ ID NO:18).

Albumin truncated C1-INH Direct Fusion (the albumin domain is underlined):

MKWVTFISLLFLFSSAYSRGVFRRDAHKSEVAHRFKDLGEENFKALVLIAFAQYLQQCP
FEDHVKLVNEVTEFAKTCVADESAENCDKSLHTLFGDKLCTVATLRETYGEMADCCAK
QEPERNECFLQHKDDNPNLPRLVRPEVDVMCTAFHDNEETFLKKYLYEIARRHPYFYAP
ELLFFAKRYKAAFTECCQAADKAACLLPKLDELRDEGKASSAKQRLKCASLQKFGERA
FKAWAVARLSQRFPKAEFAEVSKLVTDLTKVHTECCHGDLLECADDRADLAKYICENQ
DSISSKLKECCEKPLLEKSHCIAEVENDEMPADLPSLAADFVESKDVCKNYAEAKDVFL
GMFLYEYARRHPDYSVVLLLRLAKTYKTTLEKCCAAADPHECYAKVFDEFKPLVEEPQ
NLIKQNCELFEQLGEYKFQNALLVRYTKKVPQVSTPTLVEVSRNLGKVGSKCCKHPEAK
RMPCAEDYLSVVLNQLCVLHEKTPVSDRVTKCCTESLVNRRPCFSALEVDETYVPKEFN
AETFTFHADICTLSEKERQIKKQTALVELVKHKPKATKEQLKAVMDDFAAFVEKCCKA
DDKETCFAEEGKKLVAASRAALGLGSFCPGPVTLCSDLESHSTEAVLGDALVDFSLKLY
HAFSAMKKVETNMAFSPFSIASLLTQVLLGAGENTKTNLESILSYPKDFTCVHQALKGFT
TKGVTSVSQIFHSPDLAIRDTFVNASRTLYSSSPRVLSNNSDANLELINTWVAKNTNNKIS
RLLDSLPSDTRLVLLNAIYLSAKWKTTFDPKKTRMEPFHFKNSVIKVPMMNSKKYPVAH
FIDQTLKAKVGQLQLSHNLSLVILVPQNLKHRLEDMEQALSPSVFKAIMEKLEMSKFQP
TLLTLPRIKVTTSQDMLSIMEKLEFFDFSYDLNLCGLTEDPDLQVSAMQHQTVLELTETG
VEAAAASAISVARTLLVFEVQQPFLFVLWDQQHKFPVFMGRVYDPRA (SEQ ID NO:19).

D3 Albumin full length C1-INH Direct Fusion (the D3 Albumin domain is underlined):

METPAQLLFLLLLWLPDTTGVEEPQNLIKQNCELFEQLGEYKFQNALLVRYTKKVPQVS
TPTLVEVSRNLGKVGSKCCKHPEAKRMPCAEDYLSVVLNQLCVLHEKTPVSDRVTKCC
TESLVNRRPCFSALEVDETYVPKEFNAETFTFHADICTLSEKERQIKKQTALVELVKHKP
KATKEQLKAVMDDFAAFVEKCCKADDKETCFAEEGKKLVAASRAALGLNPNATSSSS
QDPESLQDRGEGKVATTVISKMLFVEPILEVSSLPTTNSTTNSATKITANTTDEPTTQPTT
EPTTQPTIQPTQPTTQLPTDSPTQPTTGSFCPGPVTLCSDLESHSTEAVLGDALVDFSLKL
YHAFSAMKKVETNMAFSPFSIASLLTQVLLGAGENTKTNLESILSYPKDFTCVHQALKG
FTTKGVTSVSQIFHSPDLAIRDTFVNASRTLYSSSPRVLSNNSDANLELINTWVAKNTNN
KISRLLDSLPSDTRLVLLNAIYLSAKWKTTFDPKKTRMEPFHFKNSVIKVPMMNSKKYP
VAHFIDQTLKAKVGQLQLSHNLSLVILVPQNLKHRLEDMEQALSPSVFKAIMEKLEMSK
FQPTLLTLPRIKVTTSQDMLSIMEKLEFFDFSYDLNLCGLTEDPDLQVSAMQHQTVLELT
ETGVEAAAASAISVARTLLVFEVQQPFLFVLWDQQHKFPVFMGRVYDPRA (SEQ ID
NO:21).

D3 Albumin truncated C1-INH Direct Fusion (the D3 Albumin domain is underlined):

METPAQLLFLLLLWLPDTTGVEEPQNLIKQNCELFEQLGEYKFQNALLVRYTKKVPQVS
TPTLVEVSRNLGKVGSKCCKHPEAKRMPCAEDYLSVVLNQLCVLHEKTPVSDRVTKCC
TESLVNRRPCFSALEVDETYVPKEFNAETFTFHADICTLSEKERQIKKQTALVELVKHKP
KATKEQLKAVMDDFAAFVEKCCKADDKETCFAEEGKKLVAASRAALGLGSFCPGPVT
LCSDLESHSTEAVLGDALVDFSLKLYHAFSAMKKVETNMAFSPFSIASLLTQVLLGAGE
NTKTNLESILSYPKDFTCVHQALKGFTTKGVTSVSQIFHSPDLAIRDTFVNASRTLYSSSP
RVLSNNSDANLELINTWVAKNTNNKISRLLDSLPSDTRLVLLNAIYLSAKWKTTFDPKK
TRMEPFHFKNSVIKVPMMNSKKYPVAHFIDQTLKAKVGQLQLSHNLSLVILVPQNLKHR
LEDMEQALSPSVFKAIMEKLEMSKFQPTLLTLPRIKVTTSQDMLSIMEKLEFFDFSYDLN
LCGLTEDPDLQVSAMQHQTVLELTETGVEAAAASAISVARTLLVFEVQQPFLFVLWDQ
QHKFPVFMGRVYDPRA (SEQ ID NO:22).

Albumin full length C1-INH GGG Linker Fusion (the Albumin domain is underlined, the linker is bolded):

MKWVTFISLLFLFSSAYSRGVFRRDAHKSEVAHRFKDLGEENFKALVLIAFAQYLQQCP
FEDHVKLVNEVTEFAKTCVADESAENCDKSLHTLFGDKLCTVATLRETYGEMADCCAK
QEPERNECFLQHKDDNPNLPRLVRPEVDVMCTAFHDNEETFLKKYLYEIARRHPYFYAP
ELLFFAKRYKAAFTECCQAADKAACLLPKLDELRDEGKASSAKQRLKCASLQKFGERA
FKAWAVARLSQRFPKAEFAEVSKLVTDLTKVHTECCHGDLLECADDRADLAKYICENQ
DSISSKLKECCEKPLLEKSHCIAEVENDEMPADLPSLAADFVESKDVCKNYAEAKDVFL
GMFLYEYARRHPDYSVVLLLRLAKTYKTTLEKCCAAADPHECYAKVFDEFKPLVEEPQ
NLIKQNCELFEQLGEYKFQNALLVRYTKKVPQVSTPTLVEVSRNLGKVGSKCCKHPEAK
RMPCAEDYLSVVLNQLCVLHEKTPVSDRVTKCCTESLVNRRPCFSALEVDETYVPKEFN
AETFTFHADICTLSEKERQIKKQTALVELVKHKPKATKEQLKAVMDDFAAFVEKCCKA
DDKETCFAEEGKKLVAASRAALGLGGGNPNATSSSSQDPESLQDRGEGKVATTVISKM
LFVEPILEVSSLPTTNSTTNSATKITANTTDEPTTQPTTEPTTQPTIQPTQPTTQLPTDSPTQ
PTTGSFCPGPVTLCSDLESHSTEAVLGDALVDFSLKLYHAFSAMKKVETNMAFSPFSIAS
LLTQVLLGAGENTKTNLESILSYPKDFTCVHQALKGFTTKGVTSVSQIFHSPDLAIRDTFV
NASRTLYSSSPRVLSNNSDANLELINTWVAKNTNNKISRLLDSLPSDTRLVLLNAIYLSA
KWKTTFDPKKTRMEPFHFKNSVIKVPMMNSKKYPVAHFIDQTLKAKVGQLQLSHNLSL
VILVPQNLKHRLEDMEQALSPSVFKAIMEKLEMSKFQPTLLTLPRIKVTTSQDMLSIMEK
LEFFDFSYDLNLCGLTEDPDLQVSAMQHQTVLELTETGVEAAAASAISVARTLLVFEVQ
QPFLFVLWDQQHKFPVFMGRVYDPRA (SEQ ID NO:23).

Albumin truncated C1-INH GGG Linker Fusion (the Albumin domain is underlined, the linker is bolded):

MKWVTFISLLFLFSSAYSRGVFRRDAHKSEVAHRFKDLGEENFKALVLIAFAQYLQQCP
FEDHVKLVNEVTEFAKTCVADESAENCDKSLHTLFGDKLCTVATLRETYGEMADCCAK
QEPERNECFLQHKDDNPNLPRLVRPEVDVMCTAFHDNEETFLKKYLYEIARRHPYFYAP
ELLFFAKRYKAAFTECCQAADKAACLLPKLDELRDEGKASSAKQRLKCASLQKFGERA

FKAWAVARLSQRFPKAEFAEVSKLVTDLTKVHTECCHGDLLECADDRADLAKYICENQ
DSISSKLKECCEKPLLEKSHCIAEVENDEMPADLPSLAADFVESKDVCKNYAEAKDVFL
GMFLYEYARRHPDYSVVLLLRLAKTYKTTLEKCCAAADPHECYAKVFDEFKPLVEEPQ
NLIKQNCELFEQLGEYKFQNALLVRYTKKVPQVSTPTLVEVSRNLGKVGSKCCKHPEAK
RMPCAEDYLSVVLNQLCVLHEKTPVSDRVTKCCTESLVNRRPCFSALEVDETYVPKEFN
AETFTFHADICTLSEKERQIKKQTALVELVKHKPKATKEQLKAVMDDFAAFVEKCCKA
DDKETCFAEEGKKLVAASRAALGLGGGGSFCPGPVTLCSDLESHSTEAVLGDALVDFSL
KLYHAFSAMKKVETNMAFSPFSIASLLTQVLLGAGENTKTNLESILSYPKDFTCVHQAL
KGFTTKGVTSVSQIFHSPDLAIRDTFVNASRTLYSSSPRVLSNNSDANLELINTWVAKNT
NNKISRLLDSLPSDTRLVLLNAIYLSAKWKTTFDPKKTRMEPFHFKNSVIKVPMMNSKK
YPVAHFIDQTLKAKVGQLQLSHNLSLVILVPQNLKHRLEDMEQALSPSVFKAIMEKLEM
SKFQPTLLTLPRIKVTTSQDMLSIMEKLEFFDFSYDLNLCGLTEDPDLQVSAMQHQTVLE
LTETGVEAAAASAISVARTLLVFEVQQPFLFVLWDQQHKFPVFMGRVYDPRA (SEQ ID
NO:24).

D3 Albumin full length C1-INH GGG Linker Fusion (the D3 Albumin domain is underlined, the linker is bolded):

METPAQLLFLLLLWLPDTTGVEEPQNLIKQNCELFEQLGEYKFQNALLVRYTKKVPQVS
TPTLVEVSRNLGKVGSKCCKHPEAKRMPCAEDYLSVVLNQLCVLHEKTPVSDRVTKCC
TESLVNRRPCFSALEVDETYVPKEFNAETFTFHADICTLSEKERQIKKQTALVELVKHKP
KATKEQLKAVMDDFAAFVEKCCKADDKETCFAEEGKKLVAASRAALGLGGGNPNATS
SSSQDPESLQDRGEGKVATTVISKMLFVEPILEVSSLPTTNSTTNSATKITANTTDEPTTQP
TTEPTTQPTIQPTQPTTQLPTDSPTQPTTGSFCPGPVTLCSDLESHSTEAVLGDALVDFSLK
LYHAFSAMKKVETNMAFSPFSIASLLTQVLLGAGENTKTNLESILSYPKDFTCVHQALK
GFTTKGVTSVSQIFHSPDLAIRDTFVNASRTLYSSSPRVLSNNSDANLELINTWVAKNTN
NKISRLLDSLPSDTRLVLLNAIYLSAKWKTTFDPKKTRMEPFHFKNSVIKVPMMNSKKY
PVAHFIDQTLKAKVGQLQLSHNLSLVILVPQNLKHRLEDMEQALSPSVFKAIMEKLEMS
KFQPTLLTLPRIKVTTSQDMLSIMEKLEFFDFSYDLNLCGLTEDPDLQVSAMQHQTVLEL
TETGVEAAAASAISVARTLLVFEVQQPFLFVLWDQQHKFPVFMGRVYDPRA (SEQ ID
NO:25).

D3 Albumin truncated C1-INH GGG Linker Fusion (the D3 Albumin domain is underlined, the linker is bolded):

METPAQLLFLLLLWLPDTTGVEEPQNLIKQNCELFEQLGEYKFQNALLVRYTKKVPQVS
TPTLVEVSRNLGKVGSKCCKHPEAKRMPCAEDYLSVVLNQLCVLHEKTPVSDRVTKCC
TESLVNRRPCFSALEVDETYVPKEFNAETFTFHADICTLSEKERQIKKQTALVELVKHKP
KATKEQLKAVMDDFAAFVEKCCKADDKETCFAEEGKKLVAASRAALGLGGGGSFCPG
PVTLCSDLESHSTEAVLGDALVDFSLKLYHAFSAMKKVETNMAFSPFSIASLLTQVLLG
AGENTKTNLESILSYPKDFTCVHQALKGFTTKGVTSVSQIFHSPDLAIRDTFVNASRTLYS
SSPRVLSNNSDANLELINTWVAKNTNNKISRLLDSLPSDTRLVLLNAIYLSAKWKTTFDP
KKTRMEPFHFKNSVIKVPMMNSKKYPVAHFIDQTLKAKVGQLQLSHNLSLVILVPQNLK
HRLEDMEQALSPSVFKAIMEKLEMSKFQPTLLTLPRIKVTTSQDMLSIMEKLEFFDFSYD
LNLCGLTEDPDLQVSAMQHQTVLELTETGVEAAAASAISVARTLLVFEVQQPFLFVLWD
QQHKFPVFMGRVYDPRA (SEQ ID NO:26).

Albumin full length C1-INH (GGGGS)2 Linker Fusion (the Albumin domain is underlined):

MKWVTFISLLFLFSSAYSRGVFRRDAHKSEVAHRFKDLGEENFKALVLIAFAQYLQQCP
FEDHVKLVNEVTEFAKTCVADESAENCDKSLHTLFGDKLCTVATLRETYGEMADCCAK
QEPERNECFLQHKDDNPNLPRLVRPEVDVMCTAFHDNEETFLKKYLYEIARRHPYFYAP
ELLFFAKRYKAAFTECCQAADKAACLLPKLDELRDEGKASSAKQRLKCASLQKFGERA
FKAWAVARLSQRFPKAEFAEVSKLVTDLTKVHTECCHGDLLECADDRADLAKYICENQ
DSISSKLKECCEKPLLEKSHCIAEVENDEMPADLPSLAADFVESKDVCKNYAEAKDVFL
GMFLYEYARRHPDYSVVLLLRLAKTYKTTLEKCCAAADPHECYAKVFDEFKPLVEEPQ
NLIKQNCELFEQLGEYKFQNALLVRYTKKVPQVSTPTLVEVSRNLGKVGSKCCKHPEAK
RMPCAEDYLSVVLNQLCVLHEKTPVSDRVTKCCTESLVNRRPCFSALEVDETYVPKEFN
AETFTFHADICTLSEKERQIKKQTALVELVKHKPKATKEQLKAVMDDFAAFVEKCCKA
DDKETCFAEEGKKLVAASRAALGLGGGGSGGGGSNPNATSSSSQDPESLQDRGEGKV
ATTVISKMLFVEPILEVSSLPTTNSTTNSATKITANTTDEPTTQPTTEPTTQPTIQPTQPTTQ
LPTDSPTQPTTGSFCPGPVTLCSDLESHSTEAVLGDALVDFSLKLYHAFSAMKKVETNM
AFSPFSIASLLTQVLLGAGENTKTNLESILSYPKDFTCVHQALKGFTTKGVTSVSQIFHSP
DLAIRDTFVNASRTLYSSSPRVLSNNSDANLELINTWVAKNTNNKISRLLDSLPSDTRLV
LLNAIYLSAKWKTTFDPKKTRMEPFHFKNSVIKVPMMNSKKYPVAHFIDQTLKAKVGQ
LQLSHNLSLVILVPQNLKHRLEDMEQALSPSVFKAIMEKLEMSKFQPTLLTLPRIKVTTS
QDMLSIMEKLEFFDFSYDLNLCGLTEDPDLQVSAMQHQTVLELTETGVEAAAASAISVA
RTLLVFEVQQPFLFVLWDQQHKFPVFMGRVYDPRA (SEQ ID NO:28).

Albumin truncated C1-INH (GGGGS)2 Linker Fusion (the Albumin domain is underlined, the linker is bolded):

MKWVTFISLLFLFSSAYSRGVFRRDAHKSEVAHRFKDLGEENFKALVLIAFAQYLQQCP
FEDHVKLVNEVTEFAKTCVADESAENCDKSLHTLFGDKLCTVATLRETYGEMADCCAK
QEPERNECFLQHKDDNPNLPRLVRPEVDVMCTAFHDNEETFLKKYLYEIARRHPYFYAP
ELLFFAKRYKAAFTECCQAADKAACLLPKLDELRDEGKASSAKQRLKCASLQKFGERA
FKAWAVARLSQRFPKAEFAEVSKLVTDLTKVHTECCHGDLLECADDRADLAKYICENQ
DSISSKLKECCEKPLLEKSHCIAEVENDEMPADLPSLAADFVESKDVCKNYAEAKDVFL
GMFLYEYARRHPDYSVVLLLRLAKTYKTTLEKCCAAADPHECYAKVFDEFKPLVEEPQ
NLIKQNCELFEQLGEYKFQNALLVRYTKKVPQVSTPTLVEVSRNLGKVGSKCCKHPEAK
RMPCAEDYLSVVLNQLCVLHEKTPVSDRVTKCCTESLVNRRPCFSALEVDETYVPKEFN
AETFTFHADICTLSEKERQIKKQTALVELVKHKPKATKEQLKAVMDDFAAFVEKCCKA
DDKETCFAEEGKKLVAASRAALGLGGGGSGGGGSGSFCPGPVTLCSDLESHSTEAVLG
DALVDFSLKLYHAFSAMKKVETNMAFSPFSIASLLTQVLLGAGENTKTNLESILSYPKDF
TCVHQALKGFTTKGVTSVSQIFHSPDLAIRDTFVNASRTLYSSSPRVLSNNSDANLELINT
WVAKNTNNKISRLLDSLPSDTRLVLLNAIYLSAKWKTTFDPKKTRMEPFHFKNSVIKVP
MMNSKKYPVAHFIDQTLKAKVGQLQLSHNLSLVILVPQNLKHRLEDMEQALSPSVFKAI
MEKLEMSKFQPTLLTLPRIKVTTSQDMLSIMEKLEFFDFSYDLNLCGLTEDPDLQVSAM
QHQTVLELTETGVEAAAASAISVARTLLVFEVQQPFLFVLWDQQHKFPVFMGRVYDPR
A (SEQ ID NO:29).

D3 Albumin full length C1-INH (GGGGS)2 Linker Fusion (the D3 Albumin domain is underlined, the linker is bolded):

METPAQLLFLLLLWLPDTTGVEEPQNLIKQNCELFEQLGEYKFQNALLVRYTKKVPQVS
TPTLVEVSRNLGKVGSKCCKHPEAKRMPCAEDYLSVVLNQLCVLHEKTPVSDRVTKCC
TESLVNRRPCFSALEVDETYVPKEFNAETFTFHADICTLSEKERQIKKQTALVELVKHKP
KATKEQLKAVMDDFAAFVEKCCKADDKETCFAEEGKKLVAASRAALGLGGGGSGGG
GSNPNATSSSSQDPESLQDRGEGKVATTVISKMLFVEPILEVSSLPTTNSTTNSATKITAN
TTDEPTTQPTTEPTTQPTIQPTQPTTQLPTDSPTQPTTGSFCPGPVTLCSDLESHSTEAVLG
DALVDFSLKLYHAFSAMKKVETNMAFSPFSIASLLTQVLLGAGENTKTNLESILSYPKDF
TCVHQALKGFTTKGVTSVSQIFHSPDLAIRDTFVNASRTLYSSSPRVLSNNSDANLELINT
WVAKNTNNKISRLLDSLPSDTRLVLLNAIYLSAKWKTTFDPKKTRMEPFHFKNSVIKVP
MMNSKKYPVAHFIDQTLKAKVGQLQLSHNLSLVILVPQNLKHRLEDMEQALSPSVFKAI
MEKLEMSKFQPTLLTLPRIKVTTSQDMLSIMEKLEFFDFSYDLNLCGLTEDPDLQVSAM
QHQTVLELTETGVEAAAASAISVARTLLVFEVQQPFLFVLWDQQHKFPVFMGRVYDPR
A (SEQ ID NO:30).

D3 Albumin truncated C1-INH (GGGGS)2 Linker Fusion (the D3 Albumin domain is underlined):

METPAQLLFLLLLWLPDTTGVEEPQNLIKQNCELFEQLGEYKFQNALLVRYTKKVPQVS
TPTLVEVSRNLGKVGSKCCKHPEAKRMPCAEDYLSVVLNQLCVLHEKTPVSDRVTKCC
TESLVNRRPCFSALEVDETYVPKEFNAETFTFHADICTLSEKERQIKKQTALVELVKHKP
KATKEQLKAVMDDFAAFVEKCCKADDKETCFAEEGKKLVAASRAALGLGGGGSGGG
GSGSFCPGPVTLCSDLESHSTEAVLGDALVDFSLKLYHAFSAMKKVETNMAFSPFSIASL
LTQVLLGAGENTKTNLESILSYPKDFTCVHQALKGFTTKGVTSVSQIFHSPDLAIRDTFV
NASRTLYSSSPRVLSNNSDANLELINTWVAKNTNNKISRLLDSLPSDTRLVLLNAIYLSA
KWKTTFDPKKTRMEPFHFKNSVIKVPMMNSKKYPVAHFIDQTLKAKVGQLQLSHNLSL
VILVPQNLKHRLEDMEQALSPSVFKAIMEKLEMSKFQPTLLTLPRIKVTTSQDMLSIMEK
LEFFDFSYDLNLCGLTEDPDLQVSAMQHQTVLELTETGVEAAAASAISVARTLLVFEVQ
QPFLFVLWDQQHKFPVFMGRVYDPRA (SEQ ID NO:31).

### B. Expression of Albumin Fusion Proteins

FreeStyle™ CHO-S Cells were transfected with these fusion construct expression vectors as described above CHO-GS stable pools were prepared and used to generate CM for collection and analysis. Cells were seeded at 2x10⁶ cells/mL and incubated for 4 days at 33°C. Glutamine was supplemented on day 3.

Conditioned Medium (CM) from the transfected cell cultures was harvested 4 days post transfection and run on Coomassie gel to evaluate expression. The 4 day batch harvests were concentrated using a 50 kDa Vivaspin®20 centrifugal concentrator (Vivaproducts, Inc., Cat. No. VS2031) and purified using Invitrogen's CaptureSelect Albumin Affinity Matrix (Invitrogen, Cat. No. 19129701L). The elution buffer was 20mM Tris pH 7.4 + 2M MgCl₂). Samples were run on Coomassie gel against BSA to evaluate expression.

The yield based on this purification for HSA-C1-INH was approximately 3.6 mg/L and for D3-C1-INH was approximately 1.6 mg/L. Proteins were concentrated to ∼2 mg/mL using Vivaspin® 50 kDa and stored at -20°C. Purified proteins were dialyzed overnight into C1-Inh formulation buffer (50 mM Tris pH7.2, 50 mM sorbitol, 150 mM glycine) and concentrated.

Proteins were checked for C1s activity. The data is shown in FIG. 14, which presents the results of an assay to measure the ability of some exemplary albumin C1-INH fusion constructs to inhibit C1s cleavage of a colorimetric peptide, including a plasma-derived C1-INH and HT1080 expressed recombinant C1-INH for comparison.

Proteins were run on SEC-MALS to check for aggregation and to determine size. Exemplary parameters of the assay were:
Column: Tosoh G3000swxl
Mobile Phase Buffer: 0.2M sodium phosphate pH 6.8
Flow Rate: 0.5 mL/min
Samples:
   HSA D3 C1-INH inject 30 µg
   HSA C1-INH inject 35 µg
   BSA control inject 40 µg
   C1-INH IgG1 LALA Fc injected at 35 µg

SEC-MALS determination was used for HSA-C1 -INH molecules using the SEC method developed for 293 C1-INH samples. No large molecular weight molecules in the HSA samples were detected using this method.

Dynamic light scattering (DLS) analysis of the C1-inhibitor albumin fusion proteins was then performed. HSA and C1-INH C46 were used as controls. All samples and controls were diluted into 0.5mg/mL in DPBS buffer. Exemplary DLS results of C1-inhibitor Albumin fusion proteins are shown in Table 4.

**Table 4: DLS Analysis of C1-inhibitor Albumin fusion proteins**

| **Sample** | **mg/mL** |
|---|---|
| HSA | 5 |
| C46 C1-INH | 2.5 |
| HSA-GGG-C1-INH CHO-GNE | 3.5 |
| D3-GGG-C1-INH CHO-GNE | 8 |
| D3-GGGGS-C1-INH CHO-GNE | 3.4 |

Purification of both HSA-C1-INH as well as HSA_D3-C1-INH using Invitrogen CaptureSelect Human Albumin Affinity Matrix from CHO-GS CM worked well. Purified proteins had C1s activity comparable to plasma-derived C1-INH. SEC-MALS showed only a single peak with no HMW species at the expected size.

Stable pools expressing Albumin-Cl-INH +/- linker constructs were generated. CHO-GS GNE cells were transfected via electroporation following manufacturer's protocol. Stable pools were selected and a 7 day batch harvest production was performed at 33°C. Batch harvests were evaluated by Coomassie Gel for expression. Coomassie results show Full-length HSA-C1-INH fusion proteins show less expression than HSA_D3 fusions. No significant differences were seen with the addition of a linker sequence.

### EQUIVALENTS AND SCOPE

Those skilled in the art will recognize, or be able to ascertain using no more than routine experimentation, many equivalents to the specific embodiments of the invention described herein. The scope of the present invention is not intended to be limited to the above Description, but rather is as set forth in the following claims:

## Claims

1. A fusion protein comprising:
a human C1-inhibitor polypeptide; and
an Fc domain,
wherein the fusion protein has an amino acid sequence selected from: SEQ ID NO:11, SEQ ID NO:13, SEQ ID NO:14, SEQ ID NO:15, or SEQ ID NO:16.

2. The fusion protein of claim 1, comprising the amino acid sequence of SEQ ID NO:13.

3. A nucleic acid encoding a fusion protein of any one of the preceding claims.

4. A cell comprising a nucleic acid of claim 3, optionally wherein the cell is a mammalian cell, such as a human cell or a Chinese Hamster Ovary (CHO) cell, optionally wherein the cell is engineered to modify glycosylation, such as engineered to improve sialylation.

5. A fusion protein of any one of claims 1-2, wherein the fusion protein is produced by a cell engineered to improve sialylation, optionally wherein producing the fusion protein comprises a step of cultivating a cell of claim 4.

6. A pharmaceutical composition comprising a fusion protein of any one of claims 1-2 and a pharmaceutically acceptable carrier, optionally wherein the pharmaceutical composition comprises a fusion protein comprising the amino acid sequence of SEQ ID NO: 13.

7. A composition comprising the pharmaceutical composition of claim 6, for use in a method of treating a complement-mediated disorder in a subject in need of treatment, optionally wherein the complement-mediated disorder is selected from hereditary angioedema, antibody mediated rejection, neuromyelitis optica spectrum disorders, traumatic brain injury, spinal cord injury, ischemic brain injury, burn injury, toxic epidermal necrolysis, multiple sclerosis, amyotrophic lateral sclerosis (ALS), Parkinson's disease, stroke, chronic inflammatory demyelinating polyneuropathy (CIDP), myasthenia gravis or multifocal motor neuropathy.

## Patentansprüche

1. Fusionsprotein, umfassend:
ein menschliches C1-Inhibitor-Polypeptid; und
eine Fc-Domäne,
wobei das Fusionsprotein eine Aminosäuresequenz ausgewählt aus SEQ ID NO:11, SEQ ID NO:13, SEQ ID NO:14, SEQ ID NO:15 oder SEQ ID NO:16 aufweist.

2. Fusionsprotein nach Anspruch 1, umfassend die Aminosäuresequenz unter SEQ ID NO:13.

3. Nukleinsäure, codierend ein Fusionsprotein nach einem der vorhergehenden Ansprüche.

4. Zelle, umfassend eine Nukleinsäure nach Anspruch 3, gegebenenfalls wobei es sich bei der Zelle um eine Säugerzelle, wie z. B. eine menschliche Zelle oder eine CHO(Chinese Hamster Ovary)-Zelle handelt, gegebenenfalls wobei die Zelle so konstruiert ist, dass die Glykosylierung modifiziert ist, z. B. so konstruiert ist, dass die Sialylierung verbessert ist.

5. Fusionsprotein nach einem der Ansprüche 1-2, wobei das Fusionprotein von einer Zelle produziert wird, die so konstruiert ist, dass die Sialylierung verbessert ist, gegebenenfalls wobei Produzieren des Fusionsproteins einen Schritt umfasst, bei dem eine Zelle nach Anspruch 4 kultiviert wird.

6. Pharmazeutische Zusammensetzung, umfassend ein Fusionsprotein nach einem der Ansprüche 1-2 und einen pharmazeutisch unbedenklichen Träger, gegebenenfalls wobei die pharmazeutische Zusammensetzung ein Fusionsprotein umfasst, das die Aminosäuresequenz unter SEQ ID NO:13 umfasst.

7. Zusammensetzung, umfassend die pharmazeutische Zusammensetzung nach Anspruch 6, zur Verwendung bei einem Verfahren zur Behandlung einer komplementvermittelten Erkrankung bei einem Individuum, bei dem eine Behandlung notwendig ist, gegebenenfalls wobei die komplementvermittelte Erkrankung ausgewählt ist aus hereditärem Angioödem, antikörpervermittelter Abstoßung, Neuromyelitisoptica-Spektrum-Erkrankungen, Hirntrauma, Rückenmarkschädigung, ischämischer Hirnschädigung, Verbrennungen, toxischer epidermaler Nekrolyse, multipler Sklerose, amyotropher Lateralsklerose (ALS), Morbus Parkinson, Schlaganfall, CIDP (Chronic Inflammatory Demyelinating Polyneuropathy), Myasthenia gravis oder multifokaler motorischer Neuropathie.

## Revendications

1. Protéine de fusion comprenant :
un polypeptide inhibiteur de C1 humain ; et
un domaine Fc,
la protéine de fusion ayant une séquence d'acides aminés choisie parmi : SEQ ID NO : 11, SEQ ID NO : 13, SEQ ID NO : 14, SEQ ID NO : 15,ou SEQ ID NO : 16.

2. Protéine de fusion selon la revendication 1, comprenant la séquence d'acides aminés de SEQ ID NO : 13.

3. Acide nucléique codant pour une protéine de fusion selon l'une quelconque des revendications précédentes.

4. Cellule comprenant un acide nucléique selon la revendication 3, la cellule étant facultativement une cellule de mammifère, telle qu'une cellule humaine ou une cellule d'ovaire de hamster chinois (CHO), la cellule étant facultativement modifiée de façon à modifier la glycosylation, par exemple modifiée pour améliorer la sialylation.

5. Protéine de fusion selon l'une quelconque des revendications 1 à 2, la protéine de fusion étant produite par une cellule modifiée de façon à améliorer la sialylation, la production de la protéine de fusion comprenant facultativement une étape de culture d'une cellule selon la revendication 4.

6. Composition pharmaceutique comprenant une protéine de fusion selon l'une quelconque des revendications 1 à 2 et un véhicule pharmaceutiquement acceptable, la composition pharmaceutique comprenant facultativement une protéine de fusion comprenant la séquence d'acides aminés de SEQ ID NO : 13.

7. Composition comprenant la composition pharmaceutique selon la revendication 6, pour utilisation dans un procédé de traitement d'un trouble médié par le complément chez un sujet ayant besoin d'un traitement, le trouble médié par le complément étant facultativement choisi parmi un angio-œdème héréditaire, un rejet médié par anticorps, des troubles du spectre de la neuromyélite optique, une lésion cérébrale traumatique, une lésion de la moelle épinière, une lésion cérébrale ischémique, une lésion de brûlure, la nécrolyse épidermique toxique, la sclérose en plaques, la sclérose latérale amyotrophique (SLA), la maladie de Parkinson, un accident vasculaire cérébral, la polyneuropathie inflammatoire démyélinisante chronique (PIDC), la myasthénie grave ou la neuropathie motrice multifocale.
